(19) Europäisches Patentamt
European Patent Office
Office européen des brevets

(11) Numéro de publication: **0 076 528**
**B1**

(12) # FASCICULE DE BREVET EUROPEEN

(45) Date de publication du fascicule du brevet: **23.05.90**

(51) Int. Cl.$^5$: **C 07 D 501/36**, C 07 D 501/34

(21) Numéro de dépôt: **82201058.3**

(22) Date de dépôt: **13.02.81**

(60) Numéro de publication de la demande initiale en application de l' article 76 CBE: **0 034 536**

(54) **Nouvelles oximes dérivées de l'acide 3-alkyloxy ou 3-alkylthiométhyl 7-amino thiazolylacétamido céphalosporanique et leur préparation.**

(30) Priorité: **18.02.80 FR 8003479**

(43) Date de publication de la demande:
**13.04.83 Bulletin 83/15**

(45) Mention de la délivrance du brevet:
**23.05.90 Bulletin 90/21**

(84) Etats contractants désignés:
**AT BE CH DE GB IT LI LU NL SE**

(56) Documents cités:
**EP-A- 29 557**
**FR-A-2 137 899**
**FR-A-2 348 218**
**FR-A-2 385 722**

(73) Titulaire: **ROUSSEL-UCLAF**
**35, boulevard des Invalides**
**F-75007 Paris (FR)**

(72) Inventeur: **Heymes, René**
**93, rue Saint Martin**
**F-70000 Vesoul (FR)**
Inventeur: **Pronine, Didier**
**65, rue de la Ferone**
**F-93110 Rosny-sous-Bois (FR)**

(74) Mandataire: **Fritel, Hubert et al**
**Département des Brevets ROUSSEL UCLAF B.P. no 9**
**F-93230 Romainville (FR)**

**EP 0 076 528 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne de nouvelles oximes dérivées de l'acide 3-alkyloxy ou 3-alkylthiométhyl 7-amino thiazolyl acétamido céphalosporanique et leur procédé de préparation.

La demande de brevet européen EP 0.029.557 qui bénéficie d'une priorité antérieure à celle de la présente demande mais n'a été publiée qu'après la priorité de la dite présente demande décrit des produits proches des produits définis ci-après. Cependant aucun de ces produits n'est décrit précisément dans le document cité.

La demande FR 2.385.722 décrit de façon générale des céphalosporines comportant un groupement amino thiazole et une fonction oxime sur la chaîne latérale. Cependant aucun produit du type de ceux définis ci-après n'est décrit précisément dans ce document.

Le brevet français 2.348.218 décrit des céphalosporines comportant un groupement amino thiazole et un radical méthoxyimino sur la chaîne latérale et en position 3 un groupement nucléophile attaché au noyau céphème par l'intermédiaire d'un groupement méthylène. En exemple figure le produit ayant un radical hydroxyméthyle en position 3.

Le brevet français 2.137.899 décrit de façon très générale des céphalosporines possédant sur la chaîne latérale un groupement hydroxyimino éthérifié. La Céfuroxime tombe dans cette formule générale.

L'invention a pour objet des produits de formule générale (A'):

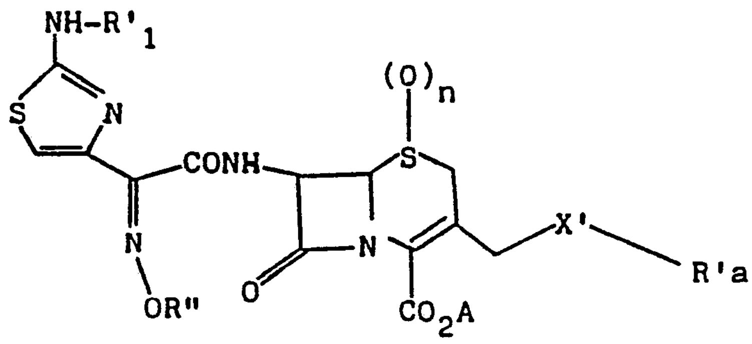

(A')

Isomère *syn* dans laquelle $R'_1$, $R''$, n, A, X' et $R'_a$ ont les significations suivantes:

*ou bien* A] A représente un atome d'hydrogène, un équivalent de métal alcalin, alcalino-terreux, de magnésium, d'ammonium ou d'une base organique aminée ou A représente le reste d'un groupement ester facilement clivable, $R'_a$ représente un radical alkyle éventuellement interrompu par un hétéroatome, alkényle ou alkynyl ayant au plus 6 atomes de carbone linéaire ou ramifié ou un radical benzyle ou phényléthyle éventuellement substitué par un radical carboxy, amino ou diméthylaminoéthyle, n est égal à 0, 1 ou 2, X' représente un atome de soufre éventuellement oxydé sous forme de sulfoxyde ou de sulfone ou un atome d'oxygène et $R''$ et $R'_1$ sont tels que:

*soit* $R'_1$représente un groupement protecteur du radical amino et $R''$ représente R', R' représente un atome d'hydrogène, un groupement protecteur du radical hydroxyle, un radical alkyle linéaire ou ramifié, alkényle, alkynyle ou cycloalkyle ayant au plus 6 atomes de carbone, ou bien R' représente un radical acétyle, propionyle, butyryle, valéryle, héxanoyle, acryloyle, crotonoyle, benzoyle, carbamoyle, chacun de ces radicaux étant éventuellement substitué par un ou plusieurs radicaux choisis parmi les radicaux carboxy éventuellement salifié ou estérifié méthoxycarbonyle, éthoxycarbonyle, carbamoyle, diméthylcarbamoyle, amino, méthylamino, diméthylamino, halogène, méthoxy, éthoxy, propyloxy, méthylthio, éthylthiophényle, tétrazolyle, phénylthio, tétrazolylthio ou thiadiazolylthio éventuellement substitué par méthyle ou bien R' représente un radical alkoxy carbonyle.

*soit* $R'_1$ représente un atome d'hydrogène et $R''$ représente un groupement protecteur du radical hydroxyle choisi parmi les radicaux phénoxyacétyle, benzoylformyle, p-nitro benzoyle, propoxycarbonyle, bêta-bêta-bêta-trichloroéthoxycarbonyle, benzyloxycarbonyle, tert-butoxycarbonyle, 1-cyclopropyléthoxycarbonyle, tétrahydropyrannyle, tétrahydrothiopyrannyle, méthoxytétrahydropyrannyle, 4-méthoxy benzyle, phtaloyle, isobutyryle, isovaléryle, oxalyle, succinyle, pivaloyle, mesyle, para-tert-butyl benzoyle, caprylyle, acryloyle, méthyl carbamoyle, phénylcarbamoyle ou naphtylcarbamoyle, étant entendu que $R''$ ne représente pas un radical alkényle ou alkyle ayant au plus 6 atomes de carbone ou un radical alkyle linéaire ou ramifié avant au plus 6 atomes de carbone éventuellement substitué par un ou plusieurs radicaux choisi parmi les radicaux amino, carboxy éventuellement estérifié ou salifié, alkoxycarbonyle, alkylamino, dialkylamino ou aryle hétérocyclique lorsque X'—$R'_a$ représente un radical alkoxy, alkylthio ou alkénylthio.

*ou bien* B] n est égal à O, A représente un atome d'hydrogène, $R'_1$ représente un groupement protecteur du radical amino et

*soit* $R''$ représente un radical $—CH_2CO_2H$ ou $—C(CH_3)_2CO_2H$, X' représente un atome d'oxygène ou de soufre et $R'_a$ représente un radical méthyle ou éthyle

*soit* R'' représente un radical

$$-\underset{\displaystyle CH_3}{\underset{|}{CH}}-CO_2H,$$

X' représente un atome d'oxygène ou de soufre et $R'_a$ représente un radical méthyle.

L'invention a notamment pour objet, parmi les produits de formule A', les produits répondant à la formule (IV'):

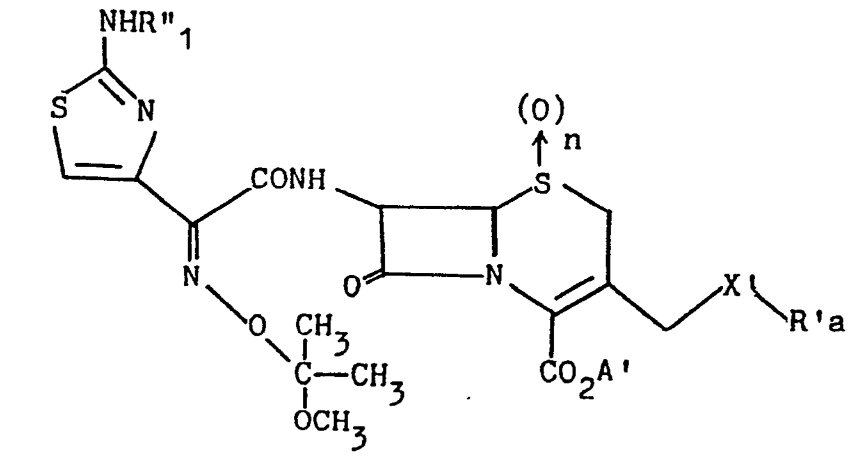

IV'

dans laquelle $R'_a$, X' et n ont la signification indiquée précédemment et A' représente un atome d'hydrogène ou le reste d'un groupement ester facilement éliminable et $R''_1$ représente un groupement protecteur du radical amino ainsi que les produits répondant à la formule générale VI.

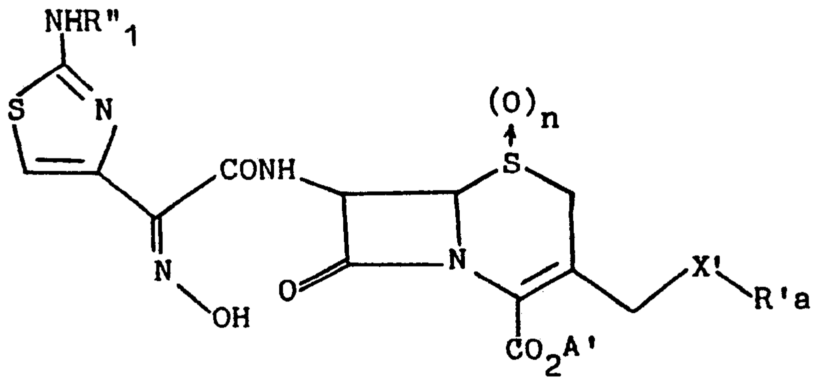

VI

dans laquelle $R'_a$, X', $R''_1$, A' et n ont la signification indiquée précédemment.

Les produits de formules A', IV' et VI constituent des intermédiaires pour la préparation de produits pharmacologiquement actifs et décrits dans les demandes européennes EP 0104671 et 0034536.

Parmi les valeurs de R'' on peut citer:

a) les radicaux méthyle, éthyle, propyle, isopropyle, butyle, sec-butyle, tert-butyle, pentyle, iso-pentyle, sec-pentyle, tert-pentyle, néo-pentyle, hexyle, iso-hexyle, sec-hexyle, tert-hexyle;

b) les radicaux vinyle, allyle, 1-propényle, butényle, pentényle, hexényle;

c) les radicaux éthynyle, propargyle, butynyle;

d) les radicaux cyclopropyle, cyclobutyle, cyclopentyle, cyclohexyle;

e) les radicaux acétyle, propionyle, butyryle, valéryle, hexanoyle, acryloyle, crotonoyle, benzoyle, carbamoyle, alkoxy carbonyle tel que méthoxy carbonyle ou éthoxycarbonyle.

Les radicaux indiqués ci-dessus aux paragraphes a) à d) peuvent eux-mêmes être substitués par un ou plusieurs radicaux tels que les radicaux: carboxy éventuellement salifié ou esterifié, alkoxycarbonyle, tel que méthoxycarbonyle, ethoxycarbonyle; carbamoyle, di-méthylcarbamoyle; amino; di-alkylamino tel que diméthylamino, diéthylamino; alkylamino tel que méthylamino; halogène tel que chlore, brome, iode; alkoxy tel que methoxy, éthoxy, propyloxy; alkylthio tel que méthylthio, éthylthio; aryle tel que phényle; aryle hétérocyclique tel que tétrazolyle; arylthio tel que phénylthio éventuellement substitué, aryle hétérocyclique thio tel que tétrazolylthio, thiadiazolylthio éventuellement substitué par alkyle tel que méthyle.

Les radicaux cités au paragraphe d) peuvent de surcroit être substitués par un radical alkyle tel que défini au paragraphe a).

Parmi les valeurs de A ou A' on peut citer un équivalent de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

On peut citer entre autres restes de groupements ester facilement clivables que peut représenter A, les groupements méthoxyméthyle, éthoxyméthyle, isopropyloxyméthyle, α-méthoxyéthyle, α-éthoxyéthyle, méthylthiométhyle, èthylthiométhyle, isopropylthiométhyle, pivaloyloxyméthyle, acétoxyméthyle, propionyloxyméthyle, butyryloxyméthyle, isobutyryloxyméthyle, valéryloxyméthyle, isovaléryloxyméthyle, tert-butyl carbonyloxyméthyle, hexadécanoyloxyméthyle, propionyloxyéthyle, isovaléryloxyéthyle, 1-acétyloxyéthyle, 1-propionyloxyéthyle, 1-butyryloxyéthyle, 1-tert-butylcarbonyloxyéthyle, 1-acétyloxypropyle, 1-hexadécanoyloxyéthyle, 1-propionyloxypropyle, 1-méthoxycarbonyloxyéthyle, méthoxycarbonyloxyméthyle, 1-acétyloxybutyle, 1-acétyloxyhexyle, 1-acétyloxyheptyle, phtalidyle, 5,6-diméthoxyphtalidyle, tert-butylcarbonylméthyle, allyle, 2-chloroallyle, méthoxycarbonylméthyle, benzyle ou tert-butyle.

On peut encore citer entre autres restes de groupements esters qui peut représenter A ou A', les groupements méthoxyéthoxyméthyle, diméthylaminoéthyle, cyanométhyle, tert-butyloxycarbonylméthyle, 2,2-éthylènedioxyéthyle, cyanoéthyle, 2,2-diméthoxyéthyle, 2-chloroéthoxyméthyle, 2-hydroxyéthoxyéthyle, 2,3-époxypropyle, 3-diméthylamino, 2-hydroxypropyle, 2-hydroxyéthyle, 2-méthylaminoethoxyméthyle, 2-aminoéthoxyméthyle, 3-méthoxy 2,4-thiadiazol-5-yle, 2-tétrahydropyranyle, 2-méthoxyprop-2-yle, 1-hydroxyprop-2-yle, isopropyle, carbamoylméthyle, chlorométhyle, 2-chloroéthyle, acétyl méthyle, 2-méthylthioéthyle ou thiocyanatométhyle.

On peut encore citer entre autres restes de groupements esters qui peut représenter A ou A', les groupements 2-chloro 1-acétyloxyéthyle, 2-bromo 1-acétyloxyéthyle, 2-fluoro 1-acétyloxyéthyle, 2-méthoxy 1-acétyloxyéthyle, 2-méthyl 1-acétyloxypropyle, 2-acétyloxyprop-2-yle, 1-méthoxyacétyloxyéthyle, 1-acétylcarbonyloxyéthyle, 1-hydroxyacétyloxyéthyle, 1-formylcarbonyloxyéthyle, 1-(2-thiényl)-carbonyloxyéthyle, 2-(2-furyl)carbonyloxyéthyle, 1-(5-nitro 2-furyl)carbonyloxyéthyle, 1-(2-pyrrolyl)-carbonyloxyéthyle, 1-(propionyloxycarbonyloxyéthyle, 1-(propyloxycarbonyloxy)éthyle, 1-(isopropyloxycarbonyloxy)éthyle, 1-(méthoxyèthoxycarbonyloxy)éthyle, 1-(allyloxycarbonyloxy)éthyle, 1-(2,3-époxy)-propyloxycarbonyloxy éthyle, 1-(2-furyl)méthyloxycarbonyloxy éthyle, 1-(2-fluoro)éthyloxycarbonyloxy éthyle, 1-(méthoxycarbonyloxy)propyle, (2-méthoxycarbonyloxy)prop-2-yle, (méthoxycarbonyloxy)chlorométhyle, 1-(méthoxycarbonyloxy)-2-chloroéthyle, 1-(méthoxycarbonyloxy)-2-méthoxyéthyle, 1-(méthoxycarbonyloxy)-1-allyle.

$R'_a$ peut représenter l'un des radicaux cités ci-dessus pour le substituant R'' aux paragraphes a) à c), en particulier méthyle, éthyle, propyle, isopropyle , allyle. $R'_a$ peut également représenter un radical méthoxy, méthyle, éthoxyméthyle.

En plus des groupements cités ci-dessus, le groupement ester facilement éliminable que peut représenter A ou A' peut être par exemple l'ester formé avec les radicaux butyle, isobutyle, tert-butyle, pentyle, hexyle, acétoxyméthyle, propionyloxyméthyle, butyryloxyméthyle, valéryloxyméthyle, pivaloyloxyméthyle, 2-acétoxyéthyle, 2-propionyloxyéthyle, 2-butyryloxyéthyle.

On peut également citer les radicaux 2-iodoéthyle, βββ-trichloroéthyle, vinyle, allyle, éthynyle, propynyle, benzyle, 4-méthoxybenzyle, 4-nitrobenzyle, phényléthyle, trityle, diphénylméthyle, 3,4-diméthoxyphényle.

On peut également citer les radicaux phényle, 4-chlorophényle, tolyle, tert-butylphényle.

Le groupement protecteur du radical amino que peut représenter $R'_1$ ou $R''_1$ peut être par exemple un radical alkyle de 1 à 6 atomes de carbone tel que, préférentiellement, tert-butyle ou tert-emyle. $R'_1$ ou $R''_1$ peut également représenter un groupement acyle, aliphatique, aromatique ou hétérocyclique ou un groupe carbamoyle.

On peut citer les groupements alcanoyles inférieurs tel que par exemple formyle, acétyle, propionyle, butyryle, isobutyryle, valéryle, isovaléryle, oxalyle, succinyle, pivaloyle. $R'_1$ ou $R''_1$ peut également représenter un groupe alkoxy, ou cycloalkoxycarbonyle inférieur tel que par exemple, méthoxycarbonyle, éthoxycarbonyle, propoxycarbonyle, 1-cyclopropyléthoxycarbonyle, isopropyloxycarbonyle, butyloxycarbonyle, tert-butyloxycarbonyle, pentyloxycarbonyle, hexyloxycarbonyle, un groupe benzoyle, toluolyle, naphtoyle, phtaloyle, mésyle, phénylacétyle, phénylpropionyle, un groupe aralcoxycarbonyle, tel que benzyloxycarbonyle.

Les groupements acyle peuvent être substitués par exemple par un atome de chlore, de brome, d'iode ou de fluor. On peut citer les radicaux chloroacétyle, dichloroacétyle, trichloroacétyle, bromoacétyle ou trifluoroacétyle.

$R'_1$ ou $R''_1$ peut également représenter un groupement aralkyle inférieur tel que benzyle, 4-méthoxybenzyle ou phényléthyle, trityle, 3,4-di-méthoxybenzyle ou benzhydryle.

$R'_1$ ou $R''_1$ peut également représenter un groupe haloalkyle tel que trichloroéthyle.

$R'_1$ ou $R''_1$ peut également représenter un groupement chlorobenzoyle, para-nitrobenzoyle, para-tert-butylbenzoyle, phénoxyacétyle, caprylyle, n-décanoyle, acryloyle, trichloroéthoxycarbonyle.

R'$_1$ ou R''$_1$ peut également représenter un groupement méthylcarbamoyle, phénylcarbamoyle, naphtylcarbamoyle, ainsi que les thiocarbamoyles correspondants.

La liste ci-dessus n'est pas limitative, il est évident que d'autres groupements protecteurs des amines, groupements connus en particulier dans la chimie des peptides, peuvent également être utilisés.

Le groupement de protection du radical hydroxyle que peut représenter R', peut être choisi dans la liste ci-dessous: R' peut représenter un groupe acyle tel que par exemple formyle, acétyle, chloroacétyle, bromoacétyle, dichloroacétyle, trichloroacétyle, trifluoroacétyle, méthoxyacétyle, phénoxyacétyle, benzoyle, benzoylformyle, p-nitrobenzoyle. On peut citer également les groupements éthoxycarbonyle, méthoxycarbonyle, propoxycarbonyle, βββ-trichloroéthoxycarbonyle, benzyloxycarbonyle, tert-butoxycarbonyle, 1-cyclo propyléthoxycarbonyle, tétrahydropyrannyle, tétrahydrothiopyrannyle, méthoxytétrahydropyrannyle, trityle, benzyle, 4-mèthoxybenzyle, benzhydryle, trichloroéthyle 1-méthyl 1-méthoxyéthyle, phtaloyle.

On peut également citer d'autres acyles tels que propionyle, butyryle, isobutyryle, valéryle, isovaléryle, oxalyle, succinyle et pivaloyle.

On peut également citer les radicaux phénylacétyle, phénylpropionyle, mésyle, chlorobenzoyle, para-nitrobenzoyle, para-tert-butylbenzoyle, caprylyle, acryloyle, méthylcarbamoyle, phénylcarbamoyle, naphtylcarbamoyle.

L'invention a plus particulierement pour objet les produits de formule générale (A):

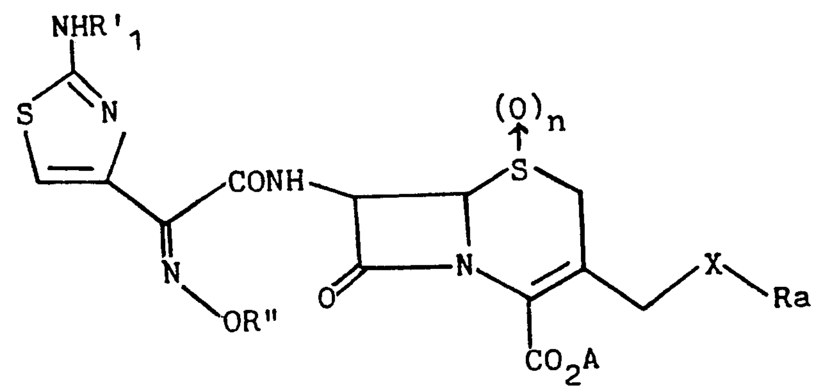

A

dans laquelle R'$_1$, A, R'' n ont la signification indiquée ci-dessus, X représente un atome de soufre ou un atome d'oxygène et Ra représente un radical alkyle, alkényle ou alkynyle ayant au plus 6 atomes de carbone linéaire ou ramifié ou un radical benzyle ou phényléthyle et X représente un atome de soufre ou un atome d'oxygène.

L'invention a également en particulier pour objet les produits de formule générale A' telle que définie ci-dessus, dans laquelle R'$_a$ représente un radical méthyle et n représente le nombre O.

L'invention a spécialement pour objet les produits de formule générale A' telle que définie ci-dessus et répondant aux formules suivantes:

l'acide 7-[[2-(2-tritylamino triazol-4-yl)2-(hydroxyimino) acétyl]amino]3-(méthylthiométhyl)ceph-3-ème 4-carboxylique isomère *syn.*

l'acide 3-méthylthiométhyl 7-[2-(2-tritylamino thiazol-4-yl2-[(1-méthyl 1-méthoxy)éthoxy imino]acétamido]ceph-3-ème 4-carboxylique isomère syn.

L'invention concerne également un procédé de préparation des produits de formule générale A' telle que définie ci-dessus caractérisé en ce que l'on traite un produit de formule (II').

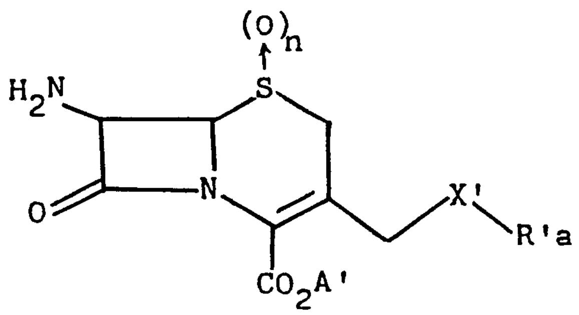

(II')

dans laquelle n, X' et R'$_a$ ont la signification indiquée ci-dessus et A' représente un atome d'hydrogène ou le reste d'un groupement ester facilement éliminable, par un produit de formule (III$_A$)

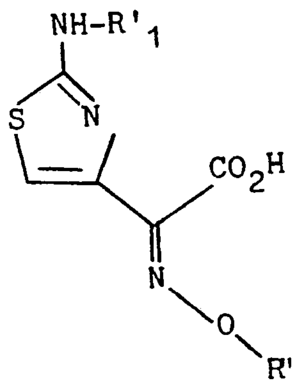

$(III_A)$

ou un dérivé fonctionnel de cet acide, formule $(III_A)$ dans laquelle $R'_1$ et R'' sont tels que définis ci-dessus et que l'on estérifie ou salifie éventuellement le produit obtenu.

L'invention a également pour objet un procédé de préparation des produits de formules IV' et VI' telles que décrites précédemment, caractérisé en ce que l'on traite un produit de formule (II').

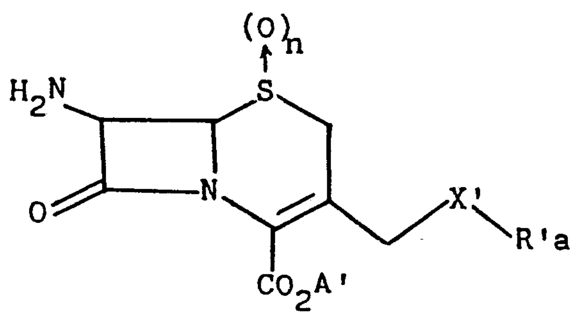

(II')

dans laquelle $R'_a$, n, A' et X' ont la signification indiquée ci-dessus, par un produit de formule (III'):

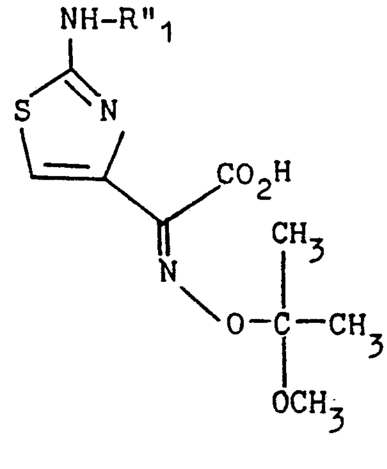

(III')

ou un dérivé fonctionnel de cet acide, formule (III') dans laquelle $R''_1$ représente un groupement protecteur du radical amino, pour obtenir un produit de formule (IV'):

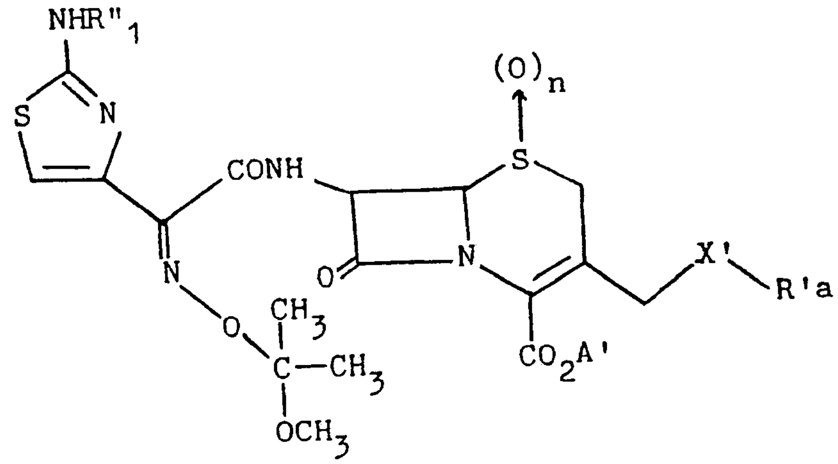

(IV')

dans laquelle A', R''$_1$, R'$_a$, X' et n ont la signification précédente, produit de formule (IV') que l'on triate si désiré par un acide dans des conditions modérées, pour obtenir un produit de formule VI.

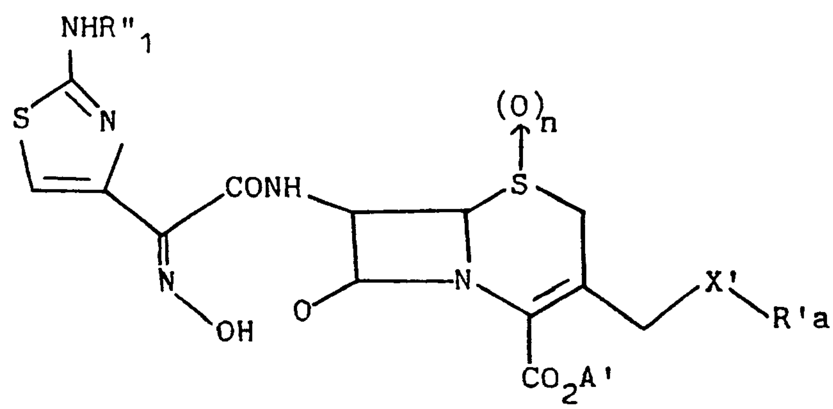

(VI)

dans laquelle R''$_1$, X', R'$_a$, A' et n ont la signification précédente.

Dans un mode préférentiel d'exécution du procédé, on traite le produit de formule (II') par un dérivé fonctionnel d'un produit de formule (III$_A$) ou (III'). Ce dérivé fonctionnel peut être par exemple un halogènure, un anhydride symétrique ou mixte, l'amide, l'azide, ou un ester activé.

Comme exemple d'anhydride mixte on peut citer par exemple celui formé avec le chloroformiate d'isobutyle et celui formé avec le chlorure de pivaloyle et les anhydrides mixtes carboxylique-sulfonique formé par exemple avec le chlorure de para-toluène sulfonyle.

Comme exemple d'ester active, on peut mentionner l'ester formé avec le 2,4-dinitrophénol et celui formé avec l'hydroxybenzothiazole.

Comme exemple d'halogénure, on peut citer le chlorure ou le bromure.

L'anhydride peut être formé in situ par action de carbodiimide NN'di substitué, par exemple N,N-dicyclohexylcarbondiimide.

La réaction d'acylation est conduite de préférence dans un solvant organique tel que le chlorure de méthylène. On peut cependant utiliser d'autres solvants tels que le tétrahydrofuranne, le chloroforme ou le diméthylformamide.

Lorsqu'on utilise un halogénure d'acide et de manière générale lorsqu'une molécule d'acide halohydrique est libérée au cours de la réaction, on réalise la réaction de préférence en présence d'une base telle que la soude, la potasse, les carbonates et carbonates acides de sodium ou de potassium, l'acétate de sodium, la triéthylamine, la pyridine, la morpholine ou la N-méthylmorpholine.

La température de réaction est en générale inférieure ou égale à la température ambiante.

Lorsque R'$_1$ représente un atome d'hydrogène on utilise de préférence un anhydride mixte carboxylique sulfonique.

L'acide que l'on utilise pour obtenir le produit de formule (VI) à partir du produit de formule (IV') est, de préférence, l'acide chlorhydrique aqueux.

La salification des produits peut être effectuée selon les méthodes usuelles.

La salification peut, par exemple, être obtenue par action sur un produit sous forme acide ou sur un

solvat, par exemple le solvat éthanolique ou un hydrate de cet acide, d'une base minérale telle que l'hydroxyde de sodium ou de potassium, le carbonate ou le carbonate acide de sodium ou de potassium. On peut également utiliser les sels d'acides minéraux tels que le phosphate tri-sodique. On peut également faire appel à des sels d'acides organiques.

Comme sels d'acides organiques, on peut mentionner, par exemple, les sels de sodium d'acides carboxyliques aliphatiques linéaires ou ramifiés, saturés ou insaturés de 1 à 18 et de préférence de 2 à 10 atomes de carbone. Les chaînes aliphatiques de ces acides peuvent être interrompues par un ou plusieurs hétéroatomes tels que l'oxygène ou le soufre ou substituées par des radicaux aryle, comme par exemple: phényle, thiényle, furyle, par un ou plusieurs radicaux hydroxyle ou par un ou plusieurs atomes d'halogène tels que fluor, chlore ou brome, préférentiellement chlore, par un ou plusieurs radicaux carboxyliques ou alkoxycarbonyle inférieurs, de préférence méthoxycarbonyle, éthoxycarbonyle ou propyloxycarbonyle, par un ou plusieurs radicaux aryloxy, de préférence phénoxy.

De plus, on peut utiliser comme acides organiques, des acides aromatiques suffisamment solubles comme par exemple des acides benzoïques substitués, de préférence par des radicaux alkyles inférieurs.

Come exemples de tels acides organiques on peut mentionner: les acides formique, acétique, acrylique, butyrique, adipique, isobutyrique, n-caproïque, isocaproïque, chloropropioniques, crotonique, phénylacétique, 2-thiénylacétique, 3-thiénylacétique, 4-éthylphénylacétique, glutarique, l'ester monoéthylique de l'acide adipique, les acides hexanoïque, heptanoïque, décanoïque, oléïque, stéarique, palmitique, 3-hydroxypropionique, 3-méthoxypropionique, 3-méthylthiòbutyrique, 4-chlorobutyrique, 4-phénylbutyrique, 3-phénoxybutyrique, 4-éthylbenzoïque, 1-propylbenzoïque.

On utilise cependant de préférence comme sels de sodium l'acétate de sodium, le 2-éthyl héxanoate de sodium ou le diéthyl acétate de sodium.

La salification peut également être obtenue par action d'une base organique comme la triéthylamine, la diéthylamine, la triméthylamine, la propylamine, la N,N-diméthyléthanolamine, la tris (hydroxyméthyl) amino méthane, la méthylamine, l'éthanolamine, la pyridine, la picoline, la dicyclohexylamine, la morpholine et la benzylamine.

Elle peut également être obtenue par acton de l'arginine, de la lysine, de la procaïne, de l'histidine, de la N-méthyl glucamine.

Cette salification est réalisée de préférence dans un solvant ou un mélange de solvants tels que l'eau, l'éther éthylique, le méthanol, l'éthanol ou l'acétone.

Les sels sont obtenus sous forme amorphe ou cristallisée selon les conditions réactionnelles employées.

Les sels cristallisés sont préparés de préférence en faisant réagir les acides libres avec l'un des sels des acides carboxyliques aliphatiques mentionnés ci-dessus, de préférence, avec l'acétate de sodium.

La salification des produits par les acides minéraux ou organiques est effectuée dans les conditions usuelles.

L'estérification éventuelle des produits est effectuée dans les conditions classiques. On opére en général en faisant réagir l'acide de formule (I') ou un dérivé fonctionnel, avec un dérivé de formule:

Z—Re

dans laquelle Z représente un radical hydroxyle ou un atome d'halogène tel que le chlore, le brome, l'iode et Re désigne le groupement ester à introduire, groupement dont une liste non exhaustive figure ci-dessus.

Les produits de formule (II') sont connus ou peuvent être préparés selon les procédés indiqués dans les brevets français 2 379 540 et 2 119 074. Les produits de formules (III') et (IIIA) sont décrits par exemple dans les brevets français 2 346 014 et 2 385 722.

Les exemples suivants illustrent l'invention sans toutefois la limiter.

## Exemple 1

Acide 3-méthoxyméthyl 7-[2-(2-amino thiazol-4-yl) 2-hydroxyimino acétamido] ceph-3-ème 4-carboxylique isomère syn.

Stade A: Anhydride para-toluène sulfonique 2-(2-tritylamino thiazol-4-yl)2-[(1-méthyl-1-méthoxy)éthoxyimino]acétique isomère syn.

On met en suspension 3,01 g de sel de triéthylamine de l'acide 2-(2-tritylamino thiazol-4-yl) 2-(1-methyl 1-méthoxy éthoxy imino) acétique isomère syn dans 15 $cm^3$ d'acétone. On ajoute 1,05 g de chlorure de tosyle et agite pendant une heure trente minutes. On introduit 20 $cm^3$ d'éther éthylique dans le mélange, refroidit à $-10$°C, essore, lave à l'éther et obtient 2,90 g de produit composé de l'anhydride recherché et de chlorhydrate de triéthylamine.

Stade B: Acide 3-méthoxy méthyl 7-[2-(2-tritylamino trizaol-4-yl) 2-[(1-méthyl 1-méthoxy) éthoxy imino]-acétamido]ceph-3-ème 4-carboxylique isomère syn.

On dissout 0,732 g d'acide 7-amino 3-méthoxy méthyl-cèph-3-ème 4-carboxylique dans 10 $cm^3$ de chlorure de méthylène et 0,84 $cm^3$ de triéthylamine, refroidit à $-20$°C et ajoute 2,4 g du mélange obtenu au Stade A laisse revenir à température ambiante, ajoute 0,5 $cm^3$ d'acide acétique, lave à l'eau, sèche,

concentre à sec, triture avec de l'éther éthylique, essore et obtient 3,07 g de produit brut que l'on recristallise dans le méthanol pour obtenir 1,21 g de produit attendu.

*RMN* ($CDCL_3$) ppm

1,52: $CH_3$ géinés
3,22—3,25: —O—$CH_3$
3,45: —$CH_2$S
4,25: —$CH_2$OMe
4,99: (d; J=5): H6
5,73: (dd; J=5; J=8): H7
6,70: $H_5$ du thiazole (syn)
7,28: —C$\phi_3$

Stade C: Acide 3-méthoxyméthyl 7-[2-(2-amino thiazol-4-yl) 2-hydroxyimino acétamido] ceph-3-ème-4-carboxylique isomère syn.

On agite pendant 10 minutes 1,1 g de produit obtenu ci-dessus avec 5 $cm^3$ d'acide formique aqueux, à 45—50°C, ajoute 2 $cm^3$ d'eau essore, concentre à sec le filtrat sous pression réduite à 30°C, en entrainant l'eau à l'éthanol. Le résidu cristallise dans l'eau. On essore et obtient 0,54 g de produit brut que l'on dissout, à l'aide de triéthylamine, dans 5 $cm^3$ d'éthanol aqueux à 50%. On acidifie à pH 2—3 avec de l'acide formique, essore le produit cristallisé, lave à l'éthanol puis à l'éther et obtient 0,44 g de produit solvaté.

*Analyse*: $C_{14}H_{15}O_6N_5A_2$ 1/2 $H_2O$ PM: 422,43

| | | | | |
|---|---|---|---|---|
| Calculé: | C% 39,9 | H% 3,82 | N% 16,58 | S% 15,18 |
| Trouvé: | 40,0 | 4,0 | 16,1 | 14,8 |

*Spectre IR* (Nujol)

C = O β lactame 1757 $^{cm^{-1}}$
C = C, C = N: 1637—1638 $^{cm^{-1}}$
aromatiques 1605—1572—1488 $^{cm^{-1}}$

*Spectre UV* (ETOH, HCl N/10)

infl: 220 nm $E^{1\%}_{1cm}$ = 288
λmax: 262 nm $E^{1\%}_{1cm}$ = 421 ε: 17400

*Spectre RMN* (DMSO) ppm

3,20:O$CH_3$
3,50: S—$CH_2$
4,17: $CH_2$O
5,14: (d; J=5): H6
5,77: (dd; J=5; J=8): H7
6,65: $H_5$ du thiazole
7,1: $NH_2$
9,43: (d; J=8) N*H*CO

## Exemple 2

3-méthoxyméthyl 7-[2-(2-amino thiazol-4-yl)2-hydroxyimino acétamido] ceph-3-ème-4-carboxylate de 1-oxopropoxy méthyle isomère syn.

Stade A: 3-méthoxyméthyl 7-[2-(2-tritylamino thiazol-4-yl) 2-[(1-méthyl 1-méthoxy)éthoxy imino] acétamido] ceph-3-ème 4-carboxylate de 1-oxopropoxy méthyle isomère syn.

On dissout à température ambiante 4,15 g d'acide 3-méthoxy méthyl 7-[2-(2-tritylamino thiazol-4-yl) 2-[(1-mèthoxy 1-méthyl) éthoxy imino] acétamido ceph-3-ème 4-carboxylique, isomère syn et 0,456 g de carbonate de potassium sec dans 14 $cm^3$ de diméthylformamide anhydre. On refroidit à 0°C, introduit, en 10 minutes, une suspension de propionete d'iodométhyle préparé comme ci-après et agite 30 minutes à 0°C puis 30 minutes à 20°C. On verse le milieu réactionnel dans un mélange constitué par 340 $cm^3$ d'eau, 17 $cm^3$ de solution aqueuse normale de bicarbonate de sodium et 50 $cm^3$ d'acétate d'éthyle. On agite, décante, extrait à l'acétate d'éthyle, lave à l'eau, sèche, concentre à sec sous pression réduite à moins de 35°C. On reprend le résidu par 25 cm $^3$ d'éther isopropylique et essore 4,42 g de produit attendu.

*RMN* ($CDCL_3$) ppm

1,15 (t, j=7); 2,40 (q, J=7) $C_2H_5$
3,34: O$CH_3$
3,55: S$CH_2$
4,33: $CH_2OCH_3$
5,05: (d, J=5): H6
6,71: $H_5$ du thiazole syn
7,33: trityle

*Préparation du propionate d'iodométhyle.*

On porte au reflux pendant 10 minutes 1,4 g de propionate de chlorométhyle, 1,71 g d'iodure de sodium et 23 $cm^3$ d'acétone anhydre. On obtient une suspension que l'on utilise immédiatement.

Stade B: 3-méthoxyméthyl 7-[2-(2-amino thiazol-4-yl) 2-hydroxyimino acétamido] ceph-3-ème-4-carboxylate de 1-oxopropoxy méthyle isomère syn.

On agite, à 45—50°C, pendant 15 minutes, 4,37 g du produit obtenu précédemment dans 22 $cm^3$ d'acide formique aqueux à 65%. On dilue avec 90 $cm^3$ d'eau à chaud, essore et distille le filtrat sous pression réduite, à moins de 30°C. On reprend le résidu par 100 $cm^3$ de chlorure de méthylène, lave avec une solution saturée de chlorure de sodium diluée au $\frac{1}{10}$ ème, et 7 $cm^3$ d'une solution normale de bicarbonate de sodium puis avec une solution saturée de chlorure de sodium diluée au $\frac{1}{10}$ ème. On sèche la phase organique, distille à sec sous pression réduite et reprend le résidu par 100 $cm^3$ d'éther éthylique, essore et obtient 2,10 g de produit brut. On le reprend dans 15 $cm^3$ d'acétate d'éthyle, agite 30 minutes, essore, rince à l'acétate d'éthyle puis à l'éther éthylique et obtient 1,69 g de produit. On en dissout 1,57 g dans 15 $cm^3$ de chlorure de méthylène, filtre, distille à sec sous pression réduite, reprend le résidu par 10 $cm^3$ d'acétate d'éthyle, agite 30 minutes, essore, rince à l'acétate d'éthyle puis à l'éther et obtient 1,29 g de produit attendu.

$[\alpha]_D = +52° \pm 1$ C = 1,5% DMSO

*RMN* ($CDCL_3$) ppm

1,13 (t; J=7); 2,41 (q; J=7): $C_2H_5$
3,30: $OC\mathit{H}_3$
3,53: $SC\mathit{H}_2$
4,3: $C\mathit{H}_2$—$OCH_3$
5,02: (d; J=5): H6
6,92: $H_5$ du thiazole (syn)

Exemple 3

Acide 3-méthylthiométhyl 7-[2-(2-amino thiazol-4-yl) 2-hydroxyimino acétamido] ceph-3-ème 4-carboxylique isomère syn.

Stade A: Acide 3-méthylthiométhyl 7-[2-(2-tritylamino thiazol-4-yl) 2-[(1-méthyl 1-méthoxy) éthoxy imino] acétamido] ceph-3-ème 4-carboxylique isomere syn.

On mélange 2,60 g d'acide 3-méthylthiométhyl 7-amino ceph-3-ème 4-carboxylique, 37,5 $cm^3$ de chlorure de méthylène et 2,8 $cm^3$ de trièthylamine, refroidit à −20°C et introduit 9,5 g d'anhydride para-toluène sulfonique 2-(2-tritylamino thiazol-4-yl) 2-[(1-méthyl 1-méthoxy) éthoxy imino] acétique préparé comme au stade A de l'exemple I. On agite à 0°C pendant deux heures, acidifie par 1,75 $cm^3$ d'acide acétique, lave à l'eau la phase organique, sèche et concentre à sec sous pression réduite. On reprend le résidu par 50 $cm^3$ d'éther éthylique, essore, rince à l'éther et obtient 8,45 g de produit brut. On le reprend par 45 $cm^3$ de méthanol et agite pendant 30 minutes. On amorce la cristallisation, essore, rince au méthanol puis à l'éther éthylique et obtient 5,2 g du produit attendu.

*RMN* ($CDCL_3$) ppm

1,85: = $CH_3S$
5,05: (d; J=5): H6
5,70: (d,d; J=5, J=8): H7
6,71: $H_5$ du thiazole (syn)
7,28: = trityle

Stade B: Acide 3-méthylthiométhyl 7-[2-(2-amino thiazol-4-yl) 2-(hydroxyamino) acétamido]cephe-3-ème 4-carboxylique isomère syn.

On agite à 50°C pendant 10 minutes 3,72 g du produit obtenu précédemment et 18,6 $cm^3$ d'acide formique aqueux à 66%. On ajoute 7,4 $cm^3$ d'eau et essore aussitôt. On distille le filtrat sous pression réduit à 30°C au maximum, effectue 2 entrainements avec un mélange éthanol eau (2/1), reprend le résidu par 10 $cm^3$ d'eau, essore, rince à l'eau, puis à l'ether éthylique et obtient 1,945 g de produit brut. On le reprend par 136 $cm^3$ d'éthanol aqueux à 50% et ajoute lentement 0,63 $cm^3$ de triéthylamine. On essore l'insoluble, acidifie le filtrat à pH ≃ 3—4 par 0,45 $cm^3$ d'acide formique aqueux à 50%. On essore, rince à l'éthanol aqueux à 50%, à l'éthanol anhydre puis à l'éther éthylique et obtient 1,392 g de produit purifié.

*Spectre IR* (Nujol)

C = O β lactame 1772 $^{cm^{-1}}$
amide 1693 $^{cm^{-1}}$
$NH_2$ (de formation) 1619 $^{cm^{-1}}$
aromatique 1595 $^{cm^{-1}}$ 1535 $^{cm^{-1}}$

*Spectre UV* (EtOH —HCl N/10)

infl: 220 nm $E_1^1 = 320$
λmax: 262 nm $E_1^1 = 444$ ε = 19100

*Spectre RMN* (DMSO) ppm

1,98: $C\mathit{H}_3S$
3,58: $C\mathit{H}_2S$
5,17: (d; J=5) H6
5,72: (d, d; J=5, J=8): H7
6,66: $H_5$ du thiazole (syn)
9,43: (d; J=8): NHCO

Exemple 4

3-méthylthiométhyl 7-[2-(2-amino thiazol-4-yl) 2-hydroxyimino acétamido) ceph-3-ème 4-carboxylate de 1-oxopropoxyméthyle isomère syn.

Stade A: 3-méthylthiométhyl 7-[2-(2-tritylamino thiazol-4-yl) 2-[(1-méthyl 1-méthoxy) éthoxy imino] acétamido] ceph-3-ème 4-carboxylate de 1-oxopropoxy méthyle isomère syn.

On agite à 20°C 5,2 d'acide 3-méthylthiométhyl 7-[2-(2-tritylamino thiazol-4-yl) 2-[(1-méthyl 1-méthoxy) éthoxyimino] acétamido] ceph-3-ème 4-carboxylique isomère syn et 0,56 g de carbonate de potassium dans 20 $cm^3$ de diméthyl formamide anhydre, refroidit à +5°C et ajoute à +5 +10°C 28 $cm^3$ d'une suspension acétonique de propionate d'iodométhyle préparé extemporaranément à partir de 1,715 g de propionate de chlorométhyle et comme décrit à l'exemple II. On agite 30 minutes à 5°C puis 30 minutes à 20°C. On verse le mélange réactionnel dans une solution constituée par 260 $cm^3$ d'eau à +10 +15°C, 20 $cm^3$ d'une solution aqueuse N de bicarbonate de sodium et 50 $cm^3$ d'acétate d'éthyle, on agite, décante, extrait à l'acétate d'éthyle, lave à l'eau sèche et distille à sec sous pression réduite. On reprend le résidu par 50 $cm^3$ d'éther isopropylique, essore et obtient 5,53 g de produit attendu.

Stade B: 3-méthylthiométhyl 7-[2-(2-amino thiazol-4-yl) 2-hydroxyimino acétamido) ceph-3-ème 4-carboxylate de 1-oxopropoxyméthyle isomère syn.

Pendant 10 minutes on agite à 50°C, 3,75 g du produit obtenu précédemment dans 18,7 $cm^3$ d'acide formique aqueux à 66%. On ajoute 7,5 $cm^3$ d'eau, essore, distille le filtrat sous pression réduite à 30°C maximum et effectue 2 entrainements par le mélange éthanol-eau 2/1. On reprend le résidu par 10 $cm^3$ d'eau, essore, rince à l'eau, puis à l'éther éthylique et obtient 2,17 g de produit brut. On le chromatographie sur silice, élue par un mélange acétate d'éthyle-acétone (3/1) reprend le résidu sec par 10 $cm^3$ d'éther isopropylique, essore 1,55 g de produit que l'on reprend par 4,5 $cm^3$ d'acétate d'éthyle, ajoute 7,5 $cm^3$ d'acétate d'éthyle, essore, rince à l'acétate d'éthyle puis à l'éther isopropylique et obtient 0,536 g de produit attendu.

*Spectre UV*

(éthanol)
λmax: 222 nm $E_1^1$ = 384 ε = 19.800
λmax: 262 nm $E_1^1$ = 271 ε = 14.000
(éthanol, HCl N/10)
infl: 218 nm $E_1^1$ = 293
λmax: 263 nm $E_1^1$ = 382 ε = 19.700

*Spectre RMN* ($CDCl_3$) ppm

1,17: (t; J=7) } $C_2H_5$
2,42: (q; J=7) }
2,1: $CH_3S$
5,11: (d; J=5) H6
5,9: COO—$CH_2$O
7,1: $H_5$ du thiazole

*Spectre IR* (Nujol)

C = O β lactame 1779 $^{cm-1}$
ester + propionate 1738 $^{cm-1}$ 1759 $^{cm-1}$
amide 1664 $^{cm-1}$
$NH_2$ (de formation) 1613 $^{cm-1}$
thiazole + amide II 1528 $^{cm-1}$

Exemple 5

Acide 3-éthylthiométhyl 7-[2-(2-amino thiazol-4-yl) 2-hydroxyimino acétamido]ceph-3-ème 4-carboxylique isomère syn.

Stade A: Acide 3-éthylthiométhyl 7-[2-(2-tritylamino thiazol-4-yl) 2-hydroxyimino acétamido] ceph-3-ème 4-carboxylique isomère syn.

A température ambiante et sous atmosphère inerte, on dissout 1,37 g d'acide 3-èthylthiométhyl 7-amino ceph-3-ème 4-carboxylique dans 15 $cm^3$ de chlorure de méthylene et 1,75 $cm^3$ de triéthylamine. On refroidit à −30°C pour introduire, en une fois, 3,97 g d'anhydride mixte tosylique de l'acide 2-(2-tritylamino thiazol 4-yl) 2-[(1-méthyl 1-méthoxy) éthoxy imino] acétique isomère syn. On laisse revenir à 0°C, agite pendant 2 heures à cette température, puis neutralise par 0,75 $cm^3$ d'acide acétique. On extrait au chlorure de méthylène, lave à l'eau la phase organique, sèche et concentre à sec sous pression réduite à ⩽30°C. On triture le résidu dans 20 $cm^3$ d'éther, essore 3,55 g de produit brut. Après recristallisation dans le méthanol, on obtient 2,17 g de produit attendu.

Stade B: Acide 3-éthylthiométhyl 7-[2-(2-amino thiazol-4-yl) 2-hydroxyimino acétamido]ceph-3-ème 4-carboxylique isomère syn.

On dissout 2,16 g de produit obtenu ci-dessus dans 10,8 $cm^3$ d'acide formique aqueux (à 66%), agite 10 minutes à 50°C et ajoute 4,3 $cm^3$ d'eau. On essore l'insoluble, concentre à sec le filtrat sous pression réduite à moins de 35°C et reprend le résidu, par 6 $cm^3$ d'eau pour concréter le produit. On esore, sèche et obtient

1,168 g de produit brut. On le purifie par dissolution dans 6 $cm^3$ d'eau et 0,4 $cm^3$ de triéthylamine. Le sel de triéthylamine cristallise. On ajoute 10 $cm^3$ d'eau. On filtre un insoluble, le lave avec 5 $cm^3$ de méthanol ajoute au filtrat 11 $cm^3$ d'éthanol et acidifie à pH: 3—4 par 1 $cm^3$ d'acide formique à 50% d'eau. Après un repos de 30 minutes à 20°C, on essore le produit cristallisé, le rince à l'éthanol anhydre, enfin à l'éther éthylique. On obtient 0,885 g de produit attendu.

*Spectre UV* (EtOH, HCl N/10)
infl: 218 nm $E_1^1$ = 320
max: 263 nm $E_1^1$ = 448 ε = 19900

*Spectre RMN* (DMSO) ppm
1,15: (t: J=7) }
2,62: (1: J=7) } —S—Et
3,61: $CH_2S$—Et
11,4: N—OH
6,75: $H_5$ du thiazole
5,12—5,2: $H_6$
5,61—5,7: }
5,75—5,83: } $H_7$
9,62—9,48: NH—C=O

L'acide 3-éthylthiométhyl 7-amino ceph 3-ème 4-carboxylique utilisé au début de l'exemple a été préparé comme suit:

On mélange 54,4 g d'acide 7-amino céphalosporanique et 544 $cm^3$ d'acide acétique. On ajoute 170 $cm^3$ d'éthérate de trifluerure de bore, puis 45 $cm^3$ d'éthanediol et on agite pendant 2 heures à 45°—50°C. On refroidit à 20—30°C, ajoute 170 $cm^3$ de triéthylamine et essore le précipité formé. On le rince à l'acide acétique, à l'acétone et à l'ether éthylique et obtient 32,45 g de produit attendu.

*Spectre UV* (EtOH, HCl N/10)
max: 262 nm $E_1^1$ = 217 ε = 5950

*Spectre RMN* (DMSO) ppm
1,13: (t: J=7) }
2,46: (q: J=7) } —S—Et
3,6: —$CH_2S$
4,72—4,79 et 4,98—5,05: H du β lactame

## Exemple 6

Acide 3-éthoxyméthyl 7-[2-(2-amino thiazol 4-yl) 2-hydroxyimino acétamido]ceph 3-ème 4-carboxylique isomère syn.

Stade A: acide 3-éthoxyméthyl 7-[2-(2-tritylamino thiazol 4-yl) 2-(1-méthoxy 1-méthyl éthoxy) imino acetamido]ceph 3-ème 4-carboxylique isomère syn.

On opère comme au stade A de l'exemple 5, avec 1,29 g d'acide 3-éthoxyméthyl 7-amino ceph 3-ème 4-carboxylique pour obtenir 3,74 g de produit brut que l'on recristallise dans de l'acétate d'éthyle pour isoler 1,791 g de produit attendu.

*Spectre UV*
1] (EtOH)
infl: 230 nm $E_1^1$ = 370
infl: 260 nm $E_1^1$ = 233 ε: 17.300
infl: 300 nm $E_1^1$ = 80 ε: 5.900
2] (EtOH, HCl N/10)
max: 271 nm $E_1^1$ = 256 ε: 19.000

*Spectre RMN* ($CDCl_3$) ppm
1,05—1,13—1,21 et 3,23—3,31—3,38—3,47: $OCH_2$—$CH_3$
1,54: $CH_3$ géminé
3,24: $OCH_3$
3,51: —S—$CH_2$
4,15: —$CH_2$—O
4,98—5,05: $H_6$
de 5,68 à 5,87: $H_7$
6,75 = $H_5$ du thiazole

Stade B: Acide 3-éthoxyméthyl 7-[2-(2-amino thiazol 4-yl) 2-hydroxyimino acétamido]ceph 3-ème 4-carboxylique isomère syn.

On dissout, en agitant, 1,735 g d'acide 3-éthoxy méthyl 7-[2-(2-tritylamino thiazol 4-yl) 2-(1-méthoxy 1-méthyl) éthoxy imino acétamido]ceph 3-ème 4-carboxylique isomère syn et 8,6 $cm^3$ d'acide formique aqueux (à 66%). On chauffe à 50°C pendant 10 minutes et ajoute à chaud 3,5 $cm^3$ d'eau. On essore le

triphénylcarbinol formé, concentre presqu'à sec le filtrat sous pression réduite, ajoute 5 $cm^3$ d'éthanol à 50% d'eau et concentre à sec à moins de 35°C. On reprend le résidu par 6 $cm^3$ d'eau, triture pour concréter le produit, essore, rince à l'eau puis à l'éther et obtient 0,915 g de produit brut. On dissout 0,813 g de ce dernier dans 10 $cm^3$ d'éthanol à 50% d'eau et 0,45 $cm^3$ de triéthylamine. On filtre, rince avec 6 $cm^3$ d'éthanol aqueux acidifie le filtrat à pH: 3.4 en ajoutant 0,7 $cm^3$ d'acide formique à 50%. On amorce la cristallisation, essore, rince à l'éthanol aqueux et à l'éther pour obtenir 0,688 g de produit attendu.

*Spectre UV* (EtOH, HCl N/10)
infl: 219 nm E: 288 ε = 12.300
max: 261 nm E: 409 ε = 17.500
*Spectre RMN* (DMSO) ppm
1,11: (t: J=7) } —OEt
3,40: (q: J=7) }
4,22: $—CH_2—O—$
5,11: −5,19 $H_6$
5,67: −5,75 −5,8 −5,87 $H_7$
6,75: $H_5$ du thiazole
7,17: $NH_2$
9,47—9,6: NHCO

L'acide 3-éthoxyméthyl 7-amino ceph 3-ème 4-carboxylique utilisé au débit de l'exemple peut être préparé de la façon suivante:

On agite 27,2 g d'acide 7-amino céphalosporanique et 272 $cm^3$ d'acétonitrile anyhydre, ajoute 128 $cm^3$ d'éthérate de trifluorure de bore puis 71 $cm^3$ d'éthanol et chauffe à 45°—50°C pendant 16 heures sous atmosphère inerte. On refroidit le mélange réactionnel à 15—20°C, et ajoute 98 $cm^3$ de triéthylamine en 15 minutes. On essore le précipité, rince à l'acétonitrile, à l'acétone puis à l'éther éthylique pour obtenir 19,25 g de produit brut. On dissout 18 g de celui-ci dans 54 $cm^3$ d'acide chlorhydrique 2N, chauffe à 45°C, traite au charbon actif, filtre et neutralise le filtrat par addition de 11 $cm^3$ d'ammoniaque à chaud. On essore à 20°C le précipité obtenu, rince à l'eau, à l'acétone et à l'éther et obtient 8,6 g de produit. On reprend 5,86 g de ce dernier par 24 $cm^3$ d'acide chlorhydrique 2N, agite pendant 1 heure à 45°C et ajoute 4 $cm^3$ d'ammoniaque à chaud. On essore à 20°C, rince à l'eau, à l'acétone puis à l'éther éthylique et obtient 4,75 g de produit attendu.

*Spectre UV* (EtOH, HClN/10)
max: 259 nm E: 239 ε: 6.200
*Spectre RMN* (DMSO) ppm
1,08: (t- J=7) } —OEt
3,39: (q: J=7) }
4,18: $—O—CH_2$
4,72—4,8 et 4,93—5,03: $H_6$ et $H_7$

Exemple 7

Acide 3-(2-propényloxy méthyl) 7-[2-(2-amino thiazol 4-yl) 2-méthoxyimino acétamido]ceph 3-ème 4-carboxylique isomère syn.

On mélange 0,96 g d'acide 2-(2-aminothiazol 4 yl) 2-méthoxyimino acétique isomère syn, 0,912 g de chlorure de tosyle et 4 $cm^3$ de diméthylacétamide anhydre. On refroidit à −20°C et ajoute 0,67 $cm^3$ de triéthylamine en maintenant l'agitation et la température pendant 1 heure. On ajoute en 5 minutes cette suspension à −20°C, à une solution, refroidie à −65—70°C obtenue en mélangeant 0,992 g d'acide 3-allyloxyméthyl 7-amino ceph 3-ème 4-carboxylique, 10 $cm^3$ de chlorure de méthylène et 1,5 $cm^3$ de triéthylamine à 15—20°C. On agite pendant 30 minutes à −65°C ± 2°, ajoute 2 $cm^3$ d'un mélange acide acétique-chlorure de méthylène 1—1, agite encore 15 minutes, laisse revenir à −50°C pour introduire 2 $cm^3$ d'eau. A 0°C en ajoute 4 $cm^3$ d'acide formique aqueux à 50%. On élimine le chlorure de méthylène par distillation à moins de 30°C sous pression réduite, tout en ajoutant 11 $cm^3$ d'eau. On ajoute 50 $cm^3$ d'une solution aqueuse saturée de chlorure de sodium. On décante la phase aqueuse et empâte le précipité gommeux avec 5 $cm^3$ d'eau pour concréter le produit. On essore, rince à l'eau puis à l'éther pour obtenir 0,728 g de produit brut. A la phase aqueuse décantée et aux eaux d'empâtage et de rinçage on ajoute 20 $cm^3$ d'une solution aqueuse saturée en chlorure de sodium et extrait à l'acétate de méthyle. On sèche la phase organique, concentre à sec et reprend le résidu par 20 $cm^3$ d'une solution aqueuse saturée en chlorure de sodium. On recueille le précipité, l'empâte avec 5 $cm^3$ d'eau pour obtenir encore 0,338 g de produit brut. On chromatographie sur silice 1,060 g de produit brut, en éluant par une solution 2M de chlorure de sodium renfermant 4‰ d'une solution 1M de bicarbonate de sodium. On acidifie à pH 3—4 les fractions aqueuses recueillies avec de l'acide formique aqueux à 50%, essore et obtient 0,180 g de produit attendu. Du filtrat on récupère à nouveau 0,368 g de produit attendu, par extraction à l'acétate de méthyle, en opérant comme ci-dessus.

*Spectre UV* (EtOH, HCl N/10)
max: 265 nm $E_1^1$: 423 ε: 19.200
*Spectre RMN* (DMSO) ppm
3,84: N—O—$CH_3$
3,86—3,93: O—$CH_2$ —CH=
4,24: —$CH_2O$—
5,13 à 5,33: =$CH_2$ et $H_6$
5,7 à 6,1: —*CH* = $CH_2$ et H7
6,72: $H_5$ du thiazole
9,5—9,6: —NHCO—

L'acide 3-allyloxyméthyl 7-amino cèph 3-ème 4-carboxylique utilisé au début de l'exemple a été préparé de la même manière que l'acide 3-éthoxy méthyl 7-amino cèph 3-ème 4-carboxylique, à l'example 8. On emploie 27,2 g d'acide 7-amino céphalosporanique, 170 $cm^3$ d'éthérate de trifluorure de bore, 136 $cm^3$ d'alcool allylique et 115 $cm^3$ de triéthylamine. De même, on purifie le produit brut (9 g) en 2 fois successives, avec de l'acide chlorhydrique chaud et de l'ammoniaque. On obtient 4,72 g de produit attendu.

*Spectre UV* (EtOH, HCl N/10)
max: 259—260 nm $E_1^1$ = 244 ε: 6.600
*Spectre* RMN (DMSO) ppm
4.23: —$CH_2$—O—
de 5,62 à 6,25: O—*CH* = $CH_2$
4,72—4,8 et 4,95—5,03: les H du β lactame

Exemple 8

Acide 3-(phénylméthoxy) méthyl 7-[2-(2-amino thiazol 4-yl) 2-méthoxyimino acétamido]ceph 3-ème 4-carboxylique isomère syn.

On opère comme à l'exemple 7 avec 1,175 g d'acide 3-(phényléthoxy) méthyl 7-amino cèph 3-ème 4-carboxylique jusqu'au stade de l'élimination du chlorure de méthylène et l'addition d'eau.

On ajoute une solution aqueuse saturée en chlorure de sodium, essore le précipité formé, rince àl'eau puis à l'éther éthylique, et obtient 0,905 g puis 0,590 g de produit. On dissout 0,707 g du produit dans 7 $cm^3$ d'eau et 0,2 $cm^3$ de triéthylamine. Après traitement au charbon actif, on ajoute 0,15 $cm^3$ d'acide formique à 50%. On essore 0,484 g de précipité, ajoute les 0,590 g de produit et triture l'ensemble dans 10 $cm^3$ d'éthanol à 50% d'eau contenant 0,6 $cm^3$ d'acide formique, pendant 15 minutes à 20°C. On essore l'insoluble et ajoute au filtrat 1,6 $cm^3$ d'ammoniaque, et 20 $cm^3$ d'eau, et extrait à l'acétate de méthyle, lave à l'eau la phase organique, sèche et concentre à sec sous pression réduite. On triture le résidu avec 10 $cm^3$ d'éthanol, essore, rince à l'éthanol puis à l'éther et obtient 0,283 g de produit ettendu.

*Spectre UV dans EtOH*
max: 237 nm $E_1^1$ ε: 17.700
infl: 255 nm $E_1^1$: 308
infl: 290 nm $E_1^1$: 154
*dans EtOH—HCl N/10*
max: 262 nm $E_1^1$ 367 ε: 18.500
*Spectre RMN* (DMSO) ppm
3,85: —N—O—$CH_3$
4,31: —$CH_2$—O—
4,45: —O—$CH_2$—Ø
5,12—5,2: $H_6$
5,68—5,77 et 5,82—5,9: $H_7$
6,75: $H_5$ du thiazole
7,33: phényle
~9,52—9,65: —NH—CO—

L'acide 3-(phénylméthoxy)méthyl 7-amino cèph 3-ème 4-carboxylique utilisé dans l'exemple a été préparé de la même manière que l'acide 3-éthoxyméthyl 7-amino cèph 3-ème 4-carboxylique à l'exemple 6. On emploie 27,2 g d'acide 7-amino céphalosporanique, 128 $cm^3$ d'éthérate de trifluorure de bore, 126 $cm^3$ d'alcool benzylique et 90 $cm^3$ de triéthylamine, et obtient 27,1 g de produit brut.

On agite 25 g de produit brut et 250 $cm^3$ d'acide acétique ajoute 25 $cm^3$ d'éthérate de trifluorure de bore, précipite la solution par addition de 25 $cm^3$ de triéthylamine. On essore le précipitaté, rince à l'acide acétique, à l'acétone puis à l'éther et obtient 4,6 g. On dissout à 45°C, 4,8 g de produit traité comme ci-dessus, dans 20 $cm^3$ d'acide chlorhydrique 2N et 8 $cm^3$ d'acide chlorhydrique concentré; traite au charbon actif, ajoute 6 $cm^3$ d'ammoniaque. On essore le précipité formé rince à l'eau, à l'acétone et à l'éther et obtient 2 g de produit attendu.

*Spectre UV* (EtOH, HCl N/10) + DMSO
max: 259 nm $E_1^1$: 216 ε: 6.900
*Spectre RMN* (DMSO)
4,28 et 4,45: —$CH_2$—O—$CH_2$
4,73—4,82 et 4,95—5,03: $H_6$ et $H_7$
7,33: aromatiques

Exemple 9

Acide 3-(2-méthoxy éthoxy) méthyl 7-[2-(2-amino thiazol 4-yl) 2-méthoxyimino acétamido]cèph 3-ème 4-carboxylique isomère syn.

On opère comme à l'exemple 7 avec 1, 160 g d'acide 3-(2-méthoxy éthoxy) méthyl 7-amino cèph 3-ème 4-carboxylique jusqu'au stade de l'élimination du chlorure de méthylène et l'addition d'eau. On ajoute 50 $cm^3$ d'une solution aqueuse saturée de chlorure de sodium, essore l'insoluble formé et extrait le filtrat à l'acétate de méthyle. On lave la phase organique à l'eau, sèche et concentre à sec sous pression réduite. On reprend le résidu par 50 $cm^3$ d'une solution aqueuse saturée de chlorure de sodium. On élimine le précipité formé par décantation et extrait le surnageant à l'acétate de méthyle. On lave à l'eau la phase organique sèche et concentre à sec sous pression réduite à moins de 30°C. On triture le résidu dans 20 $cm^3$ d'éther éthylique, essore et obtient 0,725 g de produit brut. On reprend 0,720 g de ce dernier dans 15 $cm^3$ d'eau, agite pendant 5 minutes à 20°C, essore un insoluble et extrait le filtrat à l'acétate de méthyle. On lave à l'eau la phase organique, sèche et concentre à sec sous pression réduite. On triture le résidu dans 20 $cm^3$ d'éther, essore et obtient 0,500 g de produit.

*Spectre UV*
*EtOH*
max: 238 nm $E_1^1$: 342 ε: 16.100
infl: 250 nm $E_1^1$: 313
infl: 290 nm $E_1^1$: 141
*EtOH, HCl N/10*
max 265 nm $E_1^1$: 369 ε: 17.400
*Spectre RMN* (DMSO) ppm
3,23: O—$CH_3$
3,45: $O(CH_2)_2$—O
3,83: N—O—$CH_3$
4,28: —$CH_2$—O—
5,11—5,17: $H_6$
5,69—5,74 et 5,77—5,83: $H_7$
6,74: $H_5$ du thiazole
7,22: $NH_2$
9,5—9,6: NHCO

L'acide 3-(2-méthoxy éthoxy) méthyl 7-amino cèph 3-ème 4-carboxylique utilisé au début de l'exemple a été préparé de la même façon que l'acide 3-éthoxy méthyl 7-amino cèph 3-éme 4-carboxylique à l'exemple 6. On emploie 27,2 g d'acide 7-amino céphalòsporanique, 170 $cm^3$ d'éthérate de trifluorure de bore 136 $cm^3$ de méthoxy-éthanol, 125 $cm^3$ de triéthylamine et obtient 19,25 g de produit brut. On mélange 17 g de celui-ci, 170 $cm^3$ de chlorure de méthylène, et 9,3 $cm^3$ de triéthylamine, agite pendant 20 minutes, essore l'insoluble et ajoute 8,3 $cm^3$ d'acide acétique au filtrat. On essore le précipité formé, on reprend après séchage, 9,28 g dans 130 $cm^3$ d'acétone à 2% d'eau et 4,5 $cm^3$ de triéthylamine et agite pendant 15 minutes. On filtre l'insoluble et précipite le filtrat avec 4,5 $cm^3$ d'acide formique. On essore le précipité, rince à l'acétone puis à l'éther et obtient 6,35 g de produit que l'on traite à nouveau de la même façon pour obtenir 5,77 g de produit attendu.

*Spectre UV* (EtOH, HCl N/10)
max: 260—261 nm $E_1^1$: 226 6.500
infl: 330 nm $E_1^1$: 9
*Spectre RMN* (DMSO) ppm
3,23: O—$CH_3$
3,45: $CH_2$—S— et —$O(CH_2)_2O$—
4,23: —$CH_2$—O
4,7—4,77 et 4,92—5: $H_6$ et $H_7$

Exemple 10

Acide 3-méthoxy méthyl S-oxyde 7[2-(2-amino thiazol 4-yl) 2-hydroxyimino acetamido]ceph 3-ème 4-carboxylique isomère syn.

Stade A: acide 3-méthoxy méthyl S-oxyde 7[2-(2-tritylamino thiazol 4-yl) 2[(1-méthyl 1-méthoxy)éthoxy imino]acetamido]ceph 3-ème 4-carboxylique isomère syn.

On dissout à température ambiante 1,45 g d'acide 3-méthoxyméthyl 7[2-(2-tritylamino thiazol-4-yl) 2[1-méthyl 1-méthoxy) éthoxyimino]acétamido]ceph-3-eme 4-carboxylique isomère syn dans 14 $cm^3$ de

chlorure de méthylène, refroidit à −20°C, ajoute 440 mg d'acide métachloroperbenzoïque, agite 15 mn et laisse remonter à la température ambiante. On ajoute 10 $cm^3$ d'éther isopropylique chasse le chlorure de méthylène, ajoute de nouveau 10 $cm^3$ d'éther isopropylique, essore 1,47 g de produit brut.

Stade B: Acide 3-méthoxy méthyl S-oxyde 7[2-(2-amino thiazol 4-yl) 2-hydroxyimino acetamido]ceph 3-ème 4-carboxylique isomère syn.

On mélange 1,47 g de produit obtenu au stade A et 5 $cm^3$ d'une solution aqueuse d'acide formique à 66%. On ajoute 50 $cm^3$ d'éther éthylique, essore le précipité, obtient 0,785 g de produit brut que l'on reprend par 1 $cm^3$ d'eau et 2 gouttes de pyridine, ajoute 10 $cm^3$ d'éthanol, essore, lave à l'éthanol puis à l'éther et obtient 0,51 g de produit attendu.

*Spectre UV* (EtOH, HCl N/10)
inf: 218 nm $E_1^1 = 303$
max: 261 nm $E_1^1 = 423$ ε : 18.200
inf: 375 nm $E_1^1 = 2$
*Spectre RMN* (DMSO) ppm
3,25: $OCH_3$
4,3—4,5 et 4—4,21: $CH_2$—O
4,98—5,06: $H_6$
6,83: $H_5$ du thiazole
5,95—5,98 et 6,05—6,13: $H_7$

Exemple 11

Acide 3-méthyl sulfinylméthyl S-oxyde 7[2-(2-amino thiazol 4-yl) 2-hydroxyimino acetamido[ceph-3-ème 4-carboxylique isomère syn.

On opère comme à l'exemple 10 à partir de 0,744 g d'acide 3-méthylthio méthyl 7-[2-(2-tritylamino thiazol 4-yl) [2(1-méthyl 1-méthoxy) éthoxyimino]acetamido]ceph 3-ème 4-carboxylique isomère syn et 0,404 g d'acide métachloroperbenzoïque. On obtient 0,250 g de produit attendu.

*Spectre UV* (EtOH, HCl N/10)
max: 269 nm $E_1^1 = 396$ ε = 18.300
*Spectre RMN* (DMSO) ppm
2,6: $CH_3$—S—O
5—5,08: $H_6$
5,88—5,96 } $H_7$
6,03—6,12 } $H_7$
6,78: $H_5$ du thiazole

Exemple 12

Acide 3-méthylthiométhyl 7-[2-(2-amino thiazol-4-yl) 2-[(phénylméthyl) oxy imino] acétamido] ceph-3-ème 4-carboxylique isomère syn.

Stade A: Anhydride paratoluène sulfonique 2-(2-tritylamino thiazol-4-yl) 2-[phénylméthyl) oxy imino] acétique isomère syn.

On mélange à 0°C 2,6 g d'acide 2-(2-tritylamino thiazol-4-yl) 2-[(phénylméthyl) oxy imino] acétique isomère syn dans 26 $cm^3$ d'acétone, ajoute 0,95 g de chlorure de tosyle et 0,7 $cm^3$ de triéthylamine, laisse revenir à température ambiante, agite 1 heure et obtient une suspension qui sera utilisée aussitôt pour le stade suivant.

Stade B: Acide 3-méthylthiométhyl 7-[2(2-tritylamino thiazol-4-yl) 2-[(phénylméthyl) oxy imino] acétamido] ceph-3-ème 4-carboxylique isomère syn.

On verse la suspension obtenue au stade A dans la solution préalablement refroidie, de 1,3 g d'acide 7-amino 3-méthylthiométhyl ceph-3-ème 4-carboxylique dans 13 $cm^3$ de chlorure de méthylène et 1,4 $cm^3$ de triéthylamine. On laisse revenir à température ambiante, agite pendant 45 minutes, ajoute 0,7 $cm^3$ d'acide acétique puis chasse les solvants sous pression réduite. On reprend le résidu avec 40 $cm^3$ de chlorure de méthylène et 40 $cm^3$ d'acide chlorhydrique 0,1N, décante, lave la phase organique à l'eau, sèche, chasse les solvants sous pression réduite et recueille 4,44 g de résidu.

Stade C: Acide 3-méthylthiométhyl 7-[2-(2-amino thiazol-4-yl) 2-[(phénylméthyl) oxy imino] acétamido] ceph-3-ème 4-carboxylique isomère syn.

On reprend le résidu par 22 $cm^3$ d'acide formique à 67%, agite 15 minutes à 45°C, ajoute 10 $cm^3$ d'eau, élimine l'insoluble, chasse les solvants sous pression réduite à 35°C, reprend le résidu par une solution aqueuse d'éthanol à 50%, puis dans l'eau, essore, sèche sous pression réduite et obtient 2,28 g de produit brut que l'on chromatographie sur silice (éluant: solution aqueuse de chlorure de sodium 3M contenant 0,4% de carbonate acide de sodium). On acidifie la phase organique par de l'acide chlorhydrique N, essore, empâte à l'eau, sèche et obtient 0,911 g de produit que l'on reprend par 8 $cm^3$ de chlorure de méthylène à 10% de méthanol, ajoute un peu de sulfate de magnésium, essore. On concentre à sec le filtrat sous

pression réduite, reprend le résidu à l'éther éthylique, essore, sèche et recueille 0,686 g de produit attendu pur. F = 168°C (décomposition).

*Spectre UV* (EtOH)
max: 236 nm ε = 18100
inf: 257 nm
inf: 301 nm ε = 6700
(EtOH, HCl N/10)
max: 266 nm ε = 18800

*Spectre RMN* (DMSO) ppm
1,92: S—$CH_3$
3 à 4,16: $CH_2$—S
6,8: $H_5$ du thiazole
7,41: H du phényle
5,2: $CH_2$ Ø

L'acide 2-(2-tritylamino thiazol-4-yl) 2-[(phénylméthyl) oxy imino] acétique isomère syn utilisé au départ de l'exemple 12 peut être préparé commit suit:

a) *2-(2-tritylamino thiazol-4-yl) 2-[(phénylméthyl) oxy imino] carboxylate d'éthyle*

ON mélange 9,88 g de chlorhydrate du 2-(2-tritylamino thiazol-4-yl) 2-hydroxyimino carboxylate d'éthyle décrit dans le brevet français 2 383 188, 50 $cm^3$ de diméthylformamide et 13,8 g de carbonate de potassium. On ajoute en 5 minutes à 0°C, 23 $cm^3$ de chlorure de benzyle et laisse 20 heures sous agitation à température ambiante. On ajoute 500 $cm^3$ d'eau et 100 $cm^3$ d'acétate d'éthyle, décante, lave à l'eau la phase organique, sèche, et concentre sous pression réduite. Après chromatographie sur silice (éluant: cyclohexane-acétate d'éthyle 9—1), on obtient 5,51 g de produit attendu.

b) *Acide 2-(2-tritylamino thiazol-4-yl) 2-[(phénylméthyl) oxy imino] acétique isomère syn.*

On mélange 5,3 g du produit obtenu au stade précédent dans 30 $cm^3$ d'éthanol, 8 $cm^3$ de dioxanne et 4,8 $cm^3$ de soude 2N. On agite 20 heures à température ambiante, essore, rince par le mélange éthanol-dioxanne 4—1, puis par l'éther éthylique et recueille 4,452 g de sel de sodium. On ajoute 50 $cm^3$ d'eau, 50 $cm^3$ de chlorure de méthylène et 6 $cm^3$ d'acide chlorhydrique 2N. On décante, lave la phase organique à l'eau, sèche, chasse les solvants sous pression réduite, reprend le résidu à l'éther isopropylique et obtient après séchage 3,708 g d'acide attendu.

F~ = 153°C.

*Spectre RMN* ($CDCl_3$) ppm
7,3: les Ø
6,5: $H_5$ du thiazole
5,25: $CH_2$—Ø

## Exemple 13

Acide 3-méthylthiométhyl 7-[2-(2-amino thiazol-4-yl) 2-[(2-bromoéthyl) oxy imino] acétamido] ceph-3-ème 4-carboxylique isomère syn.

On opère comme à l'exemple 12 en partant de 1,909 g d'acide 2-(2-tritylamino thiazol-4-yl) 2-[(2-bromoéthyl) oxy imino] acétique isomère syn décrit dans la demande française n° 2 438 050 et 0,68 g de chlorure de tosyle, 0,927 g d'acide 7-amino 3-méthylthiométhyl ceph-3-ème 4-carboxylique et 15 $cm^3$ d'acide formique à 67%. On obtient 0,615 g de produit attendu pur.

F = 185°C (décomposition).

*Spectre UV* (EtOH)
max: 232 nm ε = 18000
inf: 255 nm
inf: 296 nm ε = 7600
(EtOH, HCl N/10)
max: 265 nm ε = 18800
inf: 280 nm

*Spectre RMN* (DMSO) ppm
2: S—$CH_3$
3,44 à 5,2: $CH_2$—$CH_2$—Br et $CH_2$—S—$CH_3$
4,32 à 4,45: $CH_2$—$CH_2$—Br
6,9: $H_5$ du thiazole

## Exemple 14

3-méthylthiométhyl 7-[2-(2-amino thiazol-4-yl 2-hydroxyimino acétamido] ceph-3-ème 4-carboxylate de 1-[(1-oxopropyl) oxy] propyle isomère syn.

Stade A: 3-méthylthiométhyl 7-[2-(2-tritylamino thiazol-4-yl) 2-[(1-méthyl 1-méthoxy) éthoxy imino] acétamido] ceph-3-ème 4-carboxylate de 1[-(1-oxopropyl)oxy] propyle isomère syn.

On mélange sous atmosphère inerte 5 g d'acide 3-méthyl thio-méthyl 7-[2-(2-tritylamino thiazol-4-yl) 2-[(1-mèthyl 1-méthoxy) éthoxy imino] acétamido] ceph-3-ème 4-carboxylique isomère syn dans 16,5 $cm^3$ de diméthylformamide et 0,49 g de carbonate de potassium. On ajoute goutte à goutte 3 $cm^3$ de propionate de bromopropyle préparé comme ci-après et agite pendant 15 minutes. On verse le mélange réactionnel dans 200 $cm^3$ d'eau puis extrait à l'acétate d'éthyle. On lave la phase organique à l'aide d'une solution aqueuse saturée en chlorure de sodium, sèche, concentre à sec sous pression réduite à une température inférieure à 40°C et obtient 6,3 g de produit brut.

Stade B: 3-méthylthiométhyl 7-[2-(2-amino thiazol-4-yl) 2-hydroxyimino acétamido] ceph-3-ème 4-carboxylate de 1-[(1-oxopropyl) oxy] propyle isomère syn.

On agite pendant 1 heure 45 6,3 g de produit obtenu au stade précédent dans 53 $cm^3$ d'une solution d'acide formique à 90%, filtre, ajoute au filtrat 500 $cm^3$ d'eau, agite 5 minutes et extrait à l'acétate d'éthyle. On lave la phase organique à l'aide d'une solution aqueuse saturée en chlorure de sodium, sèche, concentre à sec sous pression réduite et obtient 2,66 g de produit attendu que l'on purifir par chromatographie sur silice (éluant: chlorure de méthylène-méthanol 92—8). On évapore à sec, dissout le résidu dans le méthanol et le recristallise dans l'éther isopropylique. On obtient 1,11 g de produit attendu pur. F = 140°C (décomposition).

*Spectre UV* (EtOH, HCl N/10)
max: 264 nm $E_1^1 = 347$ ε = 18800
*Spectre RMN* ($CDCl_3$) ppm
2,06: $SCH_3$
1,03 à 1,26 et 2,21 à 2,56: C—Et
||
O
6,83—7,08: $CO_2$—CH—
0,85 à 1,83: Et.

Le propionate de bromopropyle utilisé au stade A de l'exemple 19 a été préparé comme suit:

On mélange sous agitation 180 mg de chlorure de zinc et 20 $cm^3$ de bromure de propionyle, refroidit à +5°C et ajoute en 25 minutes 26,4 $cm^3$ de propionaldéhyde. On laisse 15 heures sous agitation à température ambiante puis distille sous pressio réduite et recueille 14,27 g de produit attendu utilisé tel quel pour le stade suivant.

## Exemple 15

3-méthylthiométhyl 7-[2-(2-amino thiazol-4-yl) 2-hydroxyimino acétamido] ceph-3-ème 4-carboxylate de cyanométhyle isomère syn.

On opère comme à l'exemple 14 à partir de 5 g d'acide 3-méthylthiométhyl 7-[2-(2-tritylamino thiazol-4-yl) 2-[(1-méthyl-1-méthoxy) éthoxy imino] acétamido] ceph-3-ème 4-carboxylique isomère syn et 2,2 $cm^3$ de bromure d'acétonitrile. On obtient 0,767 g de produit cristallisé que l'on purifie par addition d'éther isopropylique. F~ = 145°C.

*Spectre UV* (EtOH, HCl N/10)
max: 265 nm $E_1^1 = 367$ ε = 17200
*Spectre RMN* (DMSO) ppm
2,01: $SCH_3$
5,23: $COOCH_2$
6,75: $H_5$ du thiazole

## Exemple 16

3-méthylthiométhyl 7-[2-(2-amino thiazol-4-yl) 2-hydroxyimino acétamido] ceph-3-ème 4-carboxylate de 1-[(1-oxoéthyl oxy] propyle isomère syn.

On opère comme à l'exemple 14 à partir de 5 g d'acide 3-méthylthiométhyl 7-[2-(2-tritylamino thiazol-4-yl) 2-[(1-méthyl 1-méthoxy) éthoxy imino] acétamido] ceph-3-ème 4-carboxylique isomère syn et 7 $cm^3$ d'acétate de 3-bromopropyle en solution chloroformique. On obtient 0,965 g de produite pur. F = 130°C (décomposition).

*Spectre UV* (EtOH, HCl N/10)
max: 264 nm $E_1^1 = 347$ ε = 18400
*Spectre RMN* ($CDCl_3$) ppm
0,85 à 1,08: $CH_2$—$CH_3$
1,88 et 1,93: $SCH_3$ et O—C—$CH_3$
||
O
7,01: $H_5$ du thiazole
6,83 à 7,16: COOCH—O

L'acétate de 3-bromopropyle utilisé à l'exemple 16 a été préparé comme suit:

On mélange 37 $cm^3$ de bromure d'acétyle et 0,242 g de chlorure de zinc puis ajoute en 12 minutes à 15°C, 40 $cm^3$ de propionaldéhyde. On laisse 15 heures sous agitation à température ambiante puis distille à 53°—58°C (80/90 mm Hg). On mélange sous agitation et à température ambiante 5 $cm^3$ du produit recueilli et 5 $cm^3$ de chloroforme, ajoute par petites fractions 840 mg d'hexaméthylène tétramine, agite 10 minutes et obtient une solution chloroformique d'acétate de 3-bromopropyle utilisé immédiatement.

Exemple 17

3-méthylthiométhyl 7-[2-(2-amino thiazol-4-yl) 2-hydroxyimino acétamido] ceph-3-ème 4-carboxylate de 1-acétyloxy éthyl isomère syn.

On opère comme à l'exemple 14 à partir de 5 g d'acide 3-mèthylthiométhyl 7-[2-(2-tritylamino thiazol-4-yl) 2-[(1-méthyl 1-méthoxy) éthoxy imino] acétamido] ceph-3-ème 4-carboxylique isomère syn et 1,55 $cm^3$ d'acétate de bromoéthyl et obtient 0,867 g de produit attendu. F ~ 150°C (dècomposition).

*Spectre UV* (EtOH, HCl N/10)

max: 265 nm $E_1^1 = 347$ $\varepsilon = 17800$

*Spectre RMN* ($CDCl_3$) ppm

2,26—2,35: $CH_3$—CH

3,06—3,12: S—$CH_3$ et OAc

5,38: les $CH_2S$

10,4: $H_5$ du thiazole et 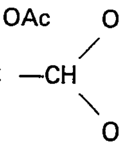

Exemple 18

3-méthylthiométhyl 7-[2-(2-amino thiazol-4-yl) 2-hydroxyimino acétamido] ceph-3-ème 4-carboxylae de 2-chloro 2-propényle isomère syn.

Stade A: 3-méthylthiométhyl 7-[2-(2-tritylamino thiazol-4-yl) 2-(1-méthyl 1-méthoxy) éthoxy imino] acétamido ceph-3-ème 4-carboxylate de 2-chloro 2-propényle isomère syn.

On dissout sous atmosphère inerte 5 g d'acide 3-méthylthiométhyl 7-[2-(2-tritylamino thiazol-4-yl) 2-[(1-méthyl 1-méthoxy) éthoxy imino] acétamido] ceph-3-ème 4-carboxylique isomère syn dans 16 $cm^3$ de diméthylformamide et 0,477 g de carbonate de potassium. On ajoute goutte à goutte la solution acétonique de 3-iodo 2-chloro 1-propène préparée ci-dessus. Après 30 minutes, on distille l'acétone sous pression réduite à une température inférieure à 40°C. On mélange la solution de diméthlformamide avec 250 ml d'éther isopropylique, décante, sèche le résidu sous pression réduite et recueille 7,8 g de produit brut.

Stade B: 3-méthylthiométhyl 7-[2-(2-amino thiazol-4-yl) 2-hydroxyimino acétamido] ceph-3-ème 4-carboxylate de 2-chloro 2-propényle isomère syn.

On opère comme au stade B de l'exemple 14 à partir de 7,8 g de produit obtenu au stade précédent, et recueille 1,18 g de produit attendu. F ~ 146°C (décomposition).

*Spectre UV* (EtOH, HCl N/10)

max: 265 nm $E_1^1 = 377$ $\varepsilon = 19000$

*Spectre RMN* ($CDCl_3$) ppm

2,03: S—$CH_3$

3,63: S—$CH_2$

4,83 à 5,6: $CO_2CH_2$ et $CH_2$=

5,1—5,18: $H_6$

La solution acétonique de 3-iodo 2-chloro 1-propène utilisée au stade A de l'exemple a été préparée comme suit:

On mélange sous atmosphère inerte 6,1 $cm^3$ de 2,3-dichloro 1-propène et 10 g d'iodure de sodium dans 43 ml d'acétone. On porte au reflux pendant 40 minutes, ramène à température ambiante et essore le chlorure de sodium formé. La solution acétonique de 3-iodo 2-chloro 1-propène est utilisée immédiatement.

Exemple 19

3-méthylthiométhyl 7-[2-(2-amino thiazol-4-yl) 2-hydroxyimino acétamido] ceph-3-ème 4-carboxylate de méthylthiométhyle isomère syn.

Stade A: 3-méthylthiométhyl 7-[2-(2-tritylamino thiazol-4-yl) 2-[(1-méthyl 1-méthoxy) éthoxy imino] acétamido] ceph-3-ème 4-carboxylate de méthylthiométhyle isomère syn.

On dissout sous atmosphère inerte 5 g d'acide 3-méthylthiométhyl 7-[2-(2-tritylamino thiazol-4-yl) 2-[(1-méthyl 1-méthoxy) éthoxy imino] acétamido] ceph-3-ème 4-carboxylique isomère syn dans 16 $cm^3$ de diméthylformamide et 480 mg de carbonate de potassium. On ajoute goutte à goutte 3,4 $cm^3$ de chlorométhyl méthyl sulfure et agite pendant 25 minutes. On verse le mélange dans 200 $cm^3$ d'une solution aqueuse saturée en chlorure de sodium essore le précipité formé, le dissout dans le chloroforme, lave avec une solution saturée en chlorure de sodium, sèche et concentre à sec sous pression réduite à une température inférieure à 40°C. On reprend le résidu à l'éther de pétrole (Eb: 60°—80°C) jusqu'à concrétion du produit attendu. On obtient 5,2 g de protuit utilisé tel quel pour le stade suivant.

Stade B: 3-méthylthiométhyl 7-[2-(2-amino thiazol-4-yl) 2-hydroxyimino acétamido] ceph-3-ème 4-carboxylate de méthylthiométhyle isomère syn.

On dissout 5,05 g du produit obtenu au stade A dans 50 $cm^3$ de méthanol et laisse 3 jours à température ambiante. On concentre sous pression réduite à une température inférieure à 40°C, reprend le résidu par 15 $cm^3$ d'éther isopropylique et agite 20 minutes à température ambiante. On essore le précipité formé, le rince à l'éther isopropylique et obtient 4,5 g de produit que l'on purifie par chromatographie sur silica (éluant: chlorure de méthylène-méthanol 92—8). On concentre à sec, reprend le résidu dans le chloroforme, précipite par addition d'éther isopropylique, essore, sèche et obtient 0,969 g de produit attendu. F = 140°C (décomposition).

*Spectre UV* (ETOH, HCl N/10)
max: 265 nm $E_1^1$ = 379 ε = 18600
*Spectre RMN* ($CDCl_3$) ppm
2,08: $S—CH_3$ en 3
2,3: $S—CH_3$ en 4
5,51: $CO_2—CH_2—S$

Exemple 20

3-méthylthiométhyl 7[2-(2-amino thiazol-4-yl) 2-hydroxyimino acétamido] ceph-3-ème 4-carboxylate de 1-[(2,2-diméthyl 1-oxopropyl) oxy] éthyle isomère syn.

Stade A: 3-mèthylthiométhyl 7-[2-(2-tritylamino thiazol-4-yl) 2-[(1-méthyl 1-méthoxy) éthoxyimino] acétamido] ceph-3-ème 4-carboxylate de 1-[(2,2-diméthyl 1-oxopropyl) oxy] éthyle isomère syn.

On introduit lentement sous atmosphère inerte, 3,72 g d'acide 3-méthylthiométhyl 7-[2-(2-tritylamino thiazol-4-yl) 2-[(1-méthyl méthoxy) éthoxyimino] ceph-3-ème 4-carboxylique isomère syn dans 7,5 $cm^3$ de tétraméthyl urée. On ajoute 0,375 g de carbonate de potassium puis le tert-butyl carboxylate de 1-iodo éthyle, préparé ci-dessous et de nouveau en 1 heure 10, 1,125 g de carbonate de potassium. On agite 10 minutes puis verse le mélange réactionnel sur un mélange composé de 500 $cm^3$ d'eau, 250 g de glace et 25 $cm^3$ d'acide chlorhydrique N/10. On agite 15 minutes, extrait à l'acétate d'éthyle. On lave la phase organique à l'eau puis à l'aide d'une solution aqueuse saturée en chlorure de sodium, sèche et concentre à sec à une température inférieure à 30°C. On obtient 8,69 g de produit brut que l'on reprend dans l'éther de pétrole (60°—80°C) jusqu'à concrétion. On recueille 5,42 g de produit attendu.

Stade B: 3-méthylthiométhyl 7-[2-(2-amino thiazol-4-yl) 2-hydroxyimino acétamido] ceph-3-ème 4-carboxylate de 1-[(2,2-diméthyl 1-oxopropyl) oxy] éthyle isomère syn.

On reprend le produit obtenu au stade précédent dans 55 $cm^3$ d'acide formique à 88%, agite pendant 1 heure 30, filtre, ajoute au filtrat 650 $cm^3$ d'eau glacée et extrait au chloroforme. On lave la phase organique à l'eau puis avec une solution aqueuse saturée en chloure de sodium, sèche et concentre à sec sous pression réduite à une température inférieure à 35°C. On recueille 3,04 g de produit que l'on chromatographie sur silice (éluant: chlorure de mèthylène-méthanol 98—2). On concentre à sec et reprend le résidu dans le chloroforme et précipite par addition d'éther isopropylique, sèche et obtient 0,733 g de produit attendu. F ~ 140°C.

*Spectre UV* (EtOH, HCl N/10)
infl: 219 nm $E_1^1$ = 265
max: 265 nm $E_1^1$ = 337 ε = 18800
*Spectre RMN* ($CDCl_3$) ppm
2,06 $CH_3—S$
1,19—1,23: t-Bu
1,5—1,6: $\mathit{CH_3}—CH$
3,33 et 3,9: $CH_2—S$

Le tert-butyl carboxylate de 1-iodo éthyle utilisé au stade A de l'exemple a été préparé comme suit:

Stade a) Pivalate de 2-chloroéthyle

On mélange 61,4 $cm^3$ de chlorure de pivaloyle et 250 mg de chlorure de zinc, agite 5 minutes, refroidit à +15°C et ajoute en 1 heure 40 $cm^3$ d'acétaldehyde. Après 15 heures à température ambiante, on distille sous pression réduite et recueille 29,01 g de produit distillant entre 56 et 58°C (27 mmHg).

Stade b) tert-butyl carboxylate de 1-iodo éthyle

On introduit lentement dans 20 $cm^3$ de tétraméthylurée 7,45 g d'iodure de sodium, agite 10 minutes et ajoute 5 $cm^3$ de pivalate de 2-chloroéthyle. On agite 30 minutes à 18°C et utilise ce réactif immédiatement.

Exemple 21

3-méthylthiométhyl 7-[2-(2-amino thiaozl-4-yl) 2-hydroxyimino acétamido] ceph-3-ème 4-carboxylate de phénylméthyle isomère syn.

Stade A: Sel de sodium de l'acide 3-méthylthiométhyl 7-[2-(2-amino thiazol-4-yl) 2-hydroxyimino acétamido] ceph-3-ème 4-carboxylique isomère syn

On dissout 1 g d'acide 3-méthylthiométhyl 7-[2-(aminothiazol-4-yl) 2-hydroxyimino acétamido]ceph-3-ème 4-carboxylique isomère syn dans 2 $cm^3$ de diméthylformamide puis ajoute 3,5 $cm^3$ d'une solution méthanolique d'acétate de sodium et 30 $cm^3$ d'éthanol. On essore le précipité, rince à l'éthanol puis à l'éther éthylique et recueille 0,804 g de produit attendu.

Stade B: 3-méthylthiométhyl 7-[2-(2-amino thiazol-4-yl) 2-hydroxyimino acétamido]ceph-3-ème 4-carboxylate de phénylméthyle isomère syn.

On mélange 0,84 g de produit préparé aus stade A et 4 $cm^3$ de diméthylformamide, ajoute lentement 0,21 $cm^3$ de bromure de benzyle et laisse 1 heure à température ambiante. On ajoute 40 $cm^3$ d'eau, essore le précipité, rince à l'eau puis à l'éther isopropylique, sèche et obtient 0,605 g de produit brut que l'on chromatographie sur silice (éluant: chlorure de méthylènemethanol 95—5). Aprés cristallisation dans l'éther isopropylique, on obtient 0,225 g de produit pur. F = 114°C.

*Spectre UV* (EtOH, HCl N/10)
Max. 263—264 nm ε = 19400
*Spectre RMN:* ($CDCl_3$) ppm.
2,01: S—$CH_3$
7,08: $H_5$ du thiazole
7.43: H du phényle
5,3: $CO_2$ $CH_2$ Φ

Exemple 22

3-méthylthiométhyl 7-[2-(2-amino thiazol-4-yl) 2-hydroxyimino acétamido]ceph-3-éme 4-carboxylate de 1-(oxo hexadecanoxy) méthyle isomère syn

On opère comme à l'exemple 14 à partir de 5 g d'acide 3-méthylthiométhyl 7-[2-(2-tritylamino thiazol-4-yl) 2-[(1-méthyl-1-méthoxy) éthoxy imino] acétamido]ceph-3-ème 4-carboxylique isomère syn et 20 $cm^3$ de solution acétonique de palmitate d'isodométhyle péparé ci-dessous. On obtient 2.02 g de produit après recristallisation dans l'éther de pétrole (60°—80°C). F = 125°C.

*Spectre UV* (EtOH, HCl N/10)
Max. 264 nm $E_1^1$ = 268 ε = 18700
*Spectre RMN* ($CDCL_3$)
0,89: $CH_2$ de l'ester
2,37: $CH_1$ de l'ester
2,04: S—$CH_3$

Le palmitate d'iodométhyle utilisé dans 'exemple a été préparé comme suit:

Stade a: Palmitate de chlorométhyle

On chauffe à 80°C sous atmosphère inerte pendant 20 minutes 33 $cm^3$ de chlorure de palmitoyle, 3 g de paraformaldéhyde et 50 mg de clorure de zinc. On ramène à température ambiante, essore le précipité et le dissout dans 10 $cm^3$ de chloroforme, ajoute 100 $cm^3$ d'éthanol, essore, obtient 5 g de produit cristallisé.

Stade b: Palmitate d'iodométhyle

On porte au reflux pendant 20 minutes sous atmosphère inerte, le mélange de 4,116 g de produit obtenu au stade a et de 2,025 g d'iodure de sodium dans 20 $cm^3$ d'acétone. On liasse revenir à température ambiante, filtre l'insoluble et recueille la solution acétonique que l'on utilise immédiatement.

Exemple 23

3-méthylthiométhyl 7-[2-(2-amino thiazol-4-yl) 2-hydroxyimino acétamido]ceph-3-éme 4-carboxylate de (2,2-diméthyl 1-oxopropoxy) méthyle isomère syn

On opère comme au stade A de l'exemple 14 à partir de 5 g d'acide 3-méthylthiométhyl 7-[2-(2-tritylamino thiazol-4-yl) 2-](1-méthyl 1-méthoxy) éthoxy imino] acétamido]ceph-3-éme 4-carboxylique isomère syn et la solution de pivalate d'iodométhyle préparée à partir de pivalate de chlorométhyle et d'iodure de sodium dans l'acetone. On obtient aprés concrétion dans l'éther isopropylique 4,1 g de produit brut utilisé tel quel pour la suite de la synthèse. On opère comme au stade B de l'exemple 19 et obtient 1,98 g de produit attendu. F = 140°C.

*Spectre UV* (EtOH, HCl N/10)
Max. 264 nm $E_1^1$ = 325 ε = 17700
*Spectre RMN* ($CDCl_3$) ppm
2,02: $CH_2$—S
3,61: $CH_2$—S
6,93: $H_5$ du thiazolea
1,24: t.Bu

Exemple 24

3-méthylthiométhyl 7-[2-(2-amino thiazol-4-yl) 2-hydroxyimino acétamido]ceph-3-éme 4-carboxylate de 1-oxopentoxy méthyle isomère syn

On opère comme au stade A de l'exemple 14 à partir de 3,72 g d'acide 3-méthylthiométhyl 7-[2-(2-tritylamino thiazol-4-yl) 2-[(1-méthyl 1-méthoxy) éthoxy imino] acétamido]ceph-3-éme 4-carboxylique isomère syn et la solution de n-valérate d'iodométhyle préparée ci-dessous. Le mélange réactionnel est extrait au chloroforme. La phase organique est lavée avec une solution aqueuse saturée en chlorure de sodium et concentrée à sec. On reprend à l'éther de pétrole (Eb = 60°—80°C), concentre à sec, reprend l'éther de pétrole jusqu'à concrétion et recueille 4.8 g de produit attendu.

On opère comme au stade B de l'exemple 14 à partir de 4.7 g du produit obtenu ci-dessus et 28,2 $cm^3$ d'une solution d'acide formique à 90%. Après extraction au chloroforme et cristallisation dans l'éther isopropylique, on obtient 0,6 g de produit attendu F~ 158°C (décomposition).

*Spectre UV* (EtOH, HCl N/10)
Max.: 265 nm $E_1^1$ = 360 ε = 19600
*Spectre RMN* ($CDCl_3$) ppm.
2,03: S—$CH_3$
0,82 à 0,97: *$CH_3$*—$CH_2$
2,3 à 2,46: C—$CH_2$
‖
O
6,96: $H_5$ du thiazole.

L n-valérate d'iodométhyle utilisé dans l'exemple a été préparé comme suit:

On agitate pendant 4 heures à 90°C le mélange de 12 g de paraformaldéhyde et 48,2 g de chlorure de l'acide n-valérique auquel on a ajouté 0,3 g de chlorure de zinc. On distille sous pression réduite à 71,5°—73°C (20 mmHg) Onn introduit 2,94 $cm^3$ du produit recueillie et 2.25 g d'iodure de sodium dans 15 $cm^3$ d'acetone, porte au reflex 20 minutes, laisse revenir à température ambiante, essore l'insoluble et reccueille la solution acétonique de n-valérate d'iodométhyle que l'on utilise immédiatement.

Exemple 25

3-méthylthiométhyl 7-[2-(2-amino thiazol-4-yl) 2-hydroxyimino acétamido]ceph-3-éme 4-carboxylate de 1-oxobutoxy méthyle isomère syn

On opère comme au stade A de l'exemple 20 à partir de 3,72 g d'acide 3-méthylthiométhyl 7-[2-(2-tritylamino thiazol-4-yl) 2-[(1-méthyl 1-méthoxy) éthoxy imino] acétamido ceph-3-éme 4-carboxylique isomère syn et la solution de n-butyrate d'iodométhyle préparée ci-dessous. On obtient 4,52 g de produit que l'on mélange à $cm^3$ d'acide formique à 90%. On poursuit la synthèse comme au stade B de l'exemple 25 et recueille 0, 627 g de produit attendu. F = 160°C (décomposition).

*Spectre UV* (HCl N/10)
inf.: 261 nm $E_1^1$ = 290 ε = 15300
Max.: 264 nm $E_1^1$ = 355 ε = 18800
*Spectre RMN (ppm)*
0,86 à 1,02: $CH_3$ du propyle
2,27 à 2,43: CO—$CH_2$
5,88: C—O—$CH_2$—O—
‖
O
6,94: $H_5$ du thiazole.

L n-butyrate d'iodométhyle a été préparé commme suit:

On mélange 12 g de paraformaldéhyde et 42,6 g de chlorure de n-butyryle et ajoute 1 g de chlorure de zinc, agite 3 heures à température ambiante puis 4 heures à 90°C. On distille (sous pression de 180—190 mm Hg) et recueille la fraction dont le point d'ébullition est compris entre 104—107°C. On introduit lentement 2,8 $cm^3$ du produit recueille dans le mélange de 6 $cm^3$ de tétraméthylurée et de 3.4 g d'iodure de sodium, agite pendant 2 heures, essore et utilise le filtrat immémiatement.

Exemple 26

3-méthylthiométhyl 7-[2-(2-amino thiazol-4-yl) 2-hydroxyimino acétamido acétamido]ceph-3-ème 4-carboxylate d'acétyloxyméthyle isomère syn

On opère comme à l'exemple 14 à partir de 5 g d'acide 3-méthylthiométhyl 7-[2-(2-tritylamino thiazol-4-yl) 2-[(1-méthyl 1-méthoxy) éthoxy imino] Acétamido]ceph-3-éme 4-carboxylique isomère syn et 20 $cm^3$ de solution acétonique d'acétate d'iodométhyle contentant 1,92 g d'acétate de chlorométhyle et 2,67 g d'iodure de sodium, et préparée extemporément. Avant la purification par chromatographie, on élimine l'acide formique résiduel par entraînement au nitrométhane. On recueille 1,677 g de produit attendu pur. F ~ 120°C (decomposition).

*Spectre UV* (EtOH, HCl N/10)
Max.: 263 nm $E_1^1$ = 354 ε = 17800
*Spectre RMN* (DMSO) ppm.
2: $CH_3S$
2,04: OAC
5,86: $COOCH_2O$
6,72: $H_5$ du thiazole.

Exemple 27

3-méthylthiométhyl 7-[2-(2-amino thiazol-4-yl) 2-hydroxyimino acétamido]ceph-3-ème 4-carboxylate de 3,3-diméthyl 2-oxobutyle isomère syn

Stade A: 3-(méthylthio) méthyl 7-[2-(2-tritylamino thiazol-4-yl) 2-[(1-méthyl 1-méthoxy) éthoxy imino]acétamido]ceph-3-ème 4-carboxylate de 3,3-diméthyl 2-oxobutyle isomère syn

On opère comme au stade A de l'exemple 20 à partir de 5 g d'acide 3-méthylthiométhyl 7-[2-(2-tritylamino thiazol-4-yl) 2-[(1-méthyl 1-méthoxy) éthoxy imino] acétamido ceph-3-ème 4-carboxylique isomère syn, 0.47 g carbonate de potassium, 27 $cm^3$ de tétraméthylurée et 0,95 $cm^3$ de bromopinacolone. On obtient 7.5 g de produit utilisé tel quel au stade suivant.

Stade B: 3-méthylthiométhyl 7-[2-(2-amino thiazol-4-yl) 2-hydroxyimino acétamido]ceph-3-ème 4-carboxylate de 3,3-diméthyl 2-oxobutyle isomère syn

On opère comme au stade B de l'exemple 20 à partir du produit obtenu stade A et de 53 $cm^3$ d'acide formique à 90%. Avant la purification per chromatographie, on effectue un entraînement au nitrométhane pour éliminer l'acide formique résiduel. On obtient 1,392 g de produit attendu. F ~ 160°C (décomposition).

*Spectre UV* (HCl N/10)
max.: 264 nm $E_1^1$ = 388 ε = 15300
*Spectre RMN* ($CDCl_3$) ppm
1,23: t.Bu
4,82—5,1 et 5,13—5,41: COO—$CH_1$CO

Exemple 28

3-méthylthiométhyl 7-[2-(2-amino thiazol-4-yl) 2-hydroxyimino acétamido]ceph-3-ème 4-carboxylate del-(1-oxopropyloxy) éthyle isomère syn

On opère comme à l'exemple 14 à partir de 10 g d'acide 3-méthylthiométhyl 7-[2-(2-tritylamino thiazol-4-yl) 2-[(1-méthyl 1-méthoxy) éthoxy imino] acétamido] ceph-3-ème 4-carboxylique isomère syn et 5,4 $cm^3$ de propionate de bromoéthyle préparé extemporément. Après traitement à l'acide formique, on effectue un entraînement au nitrométhane pour éliminer l'acide formique résiduel. On obtient 1,5 g de produit attendu, qui sont de nouveau purifiés par chromatographie sur silice (éluant: chlorure de méthyléne-méthanol 92,5—7,5). On recueille 0,566 g produit pur. F ~ 130°C.

*Spectre UV* (EtOH, HCl N/10)
max.: 264 nm $E_1^1$ = 364 ε = 19300
*Spectre RMN* ($CDCl_3$) ppm
1,5—1,6: $CH_3$—CH
7,05: $H_5$ du thiazole

Ce propionate de bromoéthyle a été préparé comme suit:

On mélange sous pression réduite 9 $cm^3$ de bromure de propionyle et 50 mg de chlorure de zinc, refroidit la solution à −15°C, ajoute goutte à goutte 5.4 $cm^3$ d'acétaldéhyde puis laisse revenir la température ambiante.

Exemple 29

3-méthylthiométhyl 7-[2-(2-amino thiazol-4-yl) 2-hydroxyimino acétamido]ceph-3-ème 4-carboxylate de-1-[1-(oxobutyl) oxy] éthyle isomère syn

On opère comme à l'exemple 14 à partir de 5 g d'acide 3-méthylthiométhyl 7-[2-(2-tritylamino thiazol-4-yl) 2-[(1-méthyl 1-méthoxy) éthoxy imino] acétamido] ceph-3-ème 4-carboxylique isomère syn dans le diméthylacétamide à la place du diméthylformamide et de la solution de butyrate d'iodo éthyle préparée comme ci-dessous. Avant la purification par chromatographie, on élimine l'acide formique résiduel par entraînement au nitrométhane. On obtient 0,530 g de produit attendu pur. F = 127°C.

*Spectre UV* (EtOH, HCl N/10)
max.: 264 nm $E_1^1$ = 353 ε = 19200
*Spectre RMN* ($CDCl_3$) ppm
0,87 à 1,03: $CH_3$—$(CH_2)_2$
1,54—1,6: $CH_3$—CH=
2,09: $CH_3$—S
7,08: $H_5$ du thiazole

Le butyrate d'iodo éthyle utilisé dans l'exemple a été préparé comme suit:

Stade a: Butyrate de chloroéthyle

On mélange à 16°C sous atmosphère inerte, 52 $cm^3$ de chlorure de butyryl et 31 $cm^3$ d'acétaldéhyde, verse dans un flacon ajoute 0,11 g de chlorure ferrique, ferme hermétiquement. Après 18 heures de repos à température ambiante, on verse le mélange réactionnel dans 200 $cm^3$ e'éther de pétrole (Eb: 60°—80°C), traite au charbon actif, filtre et distille le filtrat sous pression réduite à une température inférieure à 30°C. On recueille la fraction distillant entre 64 et 68°C (34 mm Hg).

Stade b: Butyrate d'iodoéthyle

On mélange 4 g de butyrate de chloroéthyle dans 16 $cm^3$ de diméthylacétamide et ajoute peu à peu 4 g d'iodure de sodium. On agite 15 minutes et utilise le mélange immédiatement.

Exemple 30

3-méthylthiométhyl 7-[2-(2-amino thiazol-4-yl) 2-hydroxyimino acétamido]ceph-3-ème 4-carboxylate de-1-[1-(oxohexadécyl) oxy] éthyle isomère syn

On opère comme à l'exemple 20 à partir de 744 mg d'acide 3-méthylthiométhyl 7-[2-(2-tritylamino thiazol-4-yl) 2-[(1-méthyl 1-méthoxy) éthoxy imino] acétamido] ceph-3-ème 4-carboxylique isomère syn 1,5 $cm^3$ de tétraméthylurée, 78 mg de carbonate de potassium, le palmitate d'iodoéthyl dans la tétraméthylurée préparé extemporément, et de nouveau 210 mg de carbonate de potassium. Après extraction au chloroforme, le produit recueilli est repris par 45 $cm^3$ d'acide formique à 98%. Après extraction au chloroforme et concrétion dans l'éther de pétrole (Eb: 60—80°C), on obtient 0,353 g de produit attendu. F = 108°C.

*Spectre UV* (EtOH, HCl N/10)
inf.: 218 nm $E_1^1$ = 215
max.: 265 nm $E_1^1$ = 266 ε = 18900
*Spectre RMN* ($CDCl_3$) ppm
0,88: $CH_3—(CH_2)_n$
1,5—1,6: $CH_3—CH=$
2,06: $CH_3—S$
3,63: les $CH_2S$

Exemple 31

3-méthylthiométhyl 7-[2-(2-amino thiazol-4-yl) 2-hydroxyimino acétamido]ceph-3-ème 4-carboxylate de 2-propényle isomère syn

On opère comme à l'examplе 14 à partir de 3,72 g d'acide 3-méthylthiométhyl 7-[2-(2-tritylamino thiazol-4-yl) 2-[(1-méthyl 1-méthoxy) éthoxy imino] acétamido]ceph-3-ème 4-carboxylique isomère syn dans le diméthylacétamide à la place du diméthylformamide et 1.85 $cm^3$ d'iodure d'allyle. On obtient 1.128 g de produit pur. F~ 138°C (décomposition).

*Spectre UV* (EtOH, HCl N/10)
max.: 262 nm $E_1^1$ = 266 ε = 18500
*Spectre RMN* ($CDCl_3$) ppm
2,08: $CH_3S$
4,73—4,83: COO—$CH_2$
5,08 à 6,5: $CH=CH_2$ et H du β lactame
7,08: $H_5$ du thiazole

Exemple 32

3-méthylthiométhyl 7-[2-(2-amino thiazol-4-yl) 2-hydroxyimino acétamido]ceph-3-ème 4-carboxylate de 2,2-diméthyle isomère syn

Stade A: 3-(méthylthiométhyl 7-[2-(2-tritylamino thiazol-4-yl) 2-[(1-méthyl 1-méthoxyéthoxyimino]acétamido]ceph-3-ème 4-carboxylate de 2,2-diméthyléthyle isomère syn

On porte au reflux une solution de 1.49 g d'acide 3-méthylthiométhyl 7-[2-(2-tritylamino thiazol-4-yl) 2-[(1-méthyl 1-méthoxy) éthoxy imino] acétamido]ceph-3-ème 4-carboxylique dans 30 $cm^3$ d'acétate d'éthyle et 0,8 g de O(terbutyl) N-N'-diisopropylurée. On glace, essore et concentre à sec le filtrat sous pression réduite.

Stade B: 3-méthylthiométhyl 7-[2-(2-amino thiazol-4-yl) 2-hydroxyimino acétamido]ceph-3-ème 4-carboxylate de 2,2-diméthyl isomère syn

On reprend le résidu dans 7,5 $cm^3$ d'acide formique à 67%, agite 15 minutes à 45°C, ajoute 3 $cm^3$ d'eau, essore, élimine l'insoluble. On chasse le solvant du filtrat sous pression réduite à 35°C, reprend le résidu à l'aide d'une solution aqueuse d'éthanol (1—1) puis à l'eau, sèche et obtient 1.237 g de produit brut que l'on purifie par chromatographie sur silice (éluant: chlorure de méthylène-méthanol 9—1). On obtient 0.47 g de produit pur. F = 107°C (décomposition).

*Spectre UV* (EtOH)
max.: 223 nm ε = 18100
max.: 260 nm ε = 13500
(EtOH, HCl N/10)
inf.: 220 nm
max.: 264 nm ε = 18600
*Spectre RMN* ($CDCl_3$) ppm
1,54: t.Bu
2,11: $CH_3$—S
7,08: $H_5$ du thiazole

Exemple 33

3-méthylthiométhyl 7-[2-(2-amino thiazol-4-yl) 2-hydroxyimino acétamido]ceph-3-ème 4-carboxylate de (2-méthoxy 2-oxoéthyle isomère syn

On opère comme à l'exemple 14 à partir de 3,44 g d'acide 3-méthylthiométhyl 7-[2-(2-tritylamino thiazol-4-yl) 2-[(1-méthyl 1-méthoxy) éthoxy imino] acétamido]ceph-3-ème 4-carboxylique isomère syn, et 10 $cm^3$ de solution acétonique d'iodométhyl acétate preparée ci-dessous. On obtient 2.8 g de produit que l'on triture 10 minutes à 60°C avec 5.6 $cm^3$ d'acide formique pur et 16,8 $cm^3$ d'acide formique à 50%, élimine le précipité, effectue un entraînement au nitrométhane sur le filtrat et concentre à sec. On reprend le résidu par du chloroforme et précipite à l'éther isopropylique, sèche et recueille 1.65 g de produit brut. On reprend le résidu dans 90 $cm^3$ d'acétate d'éthyle et 90 $cm^3$ d'acide chlorhydrique 0,1N, extrait à l'acétate d'éthyle, concentre à sec les phases organiques réunies, reprend le résidu au chloroforme et précipite par addition d'éther isopropylique. On purifie de nouveau par chromatographie sur silice (éluant: chlorure de méthylène-méthanol 92,5—7,5) et obtient 0,4 g de produit attendu pur. F = 156°C (décomposition).

*Spectre UV* (EtOH, HCl N/10)
max.: 264 nm $E_1^1$ = 266 ε = 19200
*Spectre RMN* ($CDCl_3$) ppm
2,07: S—$CH_3$
3,68: les S—$CH_2$
3,8: COO—$CH_3$
7: $H_5$ du thiazole

L'iodométhyle acétate a été préparé comme suit:

On mélange sous atmosphère inerte 1.6 $cm^3$ de monochloroacétate de méthyle, 2,72 g d'iodure de sodium dans 20 $cm^3$ d'acétone et chauffe 25 minutes, au reflux, essore et recueille le filtrat que l'on emploie immédiatement.

Exemple 34

3-méthoxyméthyl 7-[2-(2-amino thiazol-4-yl) 2-hydroxyimino acétamido]ceph-3-ème 4-carboxylate de 1-(acétyloxy) éthyle isomère syn

Stade A: 3-(méthoxyométhyl 7-[2-(2-tritylamino thiazol-4-yl) 2-[(1-méthyl 1-méthoxy éthoxy) imino] acétamido]ceph-3-ème 4-carboxylate de 1-(acétyloxyéthyle) isomère syn

On agite 15 minutes à 20°C mélange de 10,92 g d'acide 3-méthoxyméthyl 7-[2-(2-tritylamino thiazol-4-yl) 2-[(1-méthyl 1-méthoxyéthoxy) imino] acétamido]ceph-3-ème 4-carboxylique isomère syn, 55 $cm^3$ de diméthylformamide et 1.28 g de carbonate de potassium. On refroidit la solution à +5°C et ajoute 3,4 $cm^3$ d'acétate de 1-bromoéthyle, agite 20 minutes, ajoute 0,1 g de carbonate de potassium et 0,3 $cm^3$ d'acétate de 1-bromoéthyle, agite 25 minutes, ajoute 550 $cm^3$ d'eau, 150 $cm^3$ d'acétate d'éthyle et 25 $cm^3$ de solution aqueuse de bicarbonate de sodium (M), décante, extrait à l'acétate d'éthyle, sèche, concentre à sec sous pression réduite à une température inférieure à 30°C. On recueille 14,3 g de produit.

Stade B: 3-méthoxyméthyl 7-[2-(2-amino thiazol-4-yl) 2-hydroxyimino acétamido]ceph-3-ème 4-carboxylate de 1-(acétyloxy) éthyle isomère syn

On dissout 14,3 g du produit du staid A dans 61 $cm^3$ d'acide formique à66%, agite 10 minutes à 50°C, ajoute 24 $cm^3$ d'eau, élinine le précipité, concentre le filtrat sous pression réduite reprend par 50 $cm^3$ de chlorure de méthylène et 350 $cm^3$ d'eau et 10 $cm^3$ d'une solution aqueuse de bicarbonate de sodium, décante, extrait au chlorure de méthylène, lave à l'eau, sèche, distille à sec sous pression réduite, reprend à l'acétate d'éthyle, essore et, sèche 4,87 g de produit attendu.

*Spectre UV* (EtOH)
max.: 223 nm $E_1^1$ = 387 ε = 19300
max.: 259 nm $E_1^1$ = 285 ε = 19300
*Spectre UV* (EtOH, HCl N/10)
inf.: 219 nm $E_1^1$ = 286
max.: 261—262 nm $E_1^1$ = 390 ε = 19500
*Spectre RMN* (DMSO) ppm
1,43—1,51: $CH_3$—CH—
2,08: oAc
3,58: $CH_2$—S
3,25: O—$CH_3$
6,75: $H_5$ du thiazole

Exemple 35

3-(éthoxy) méthyl 7-[2-(2-amino thiazol-4-yl) 2-hydroxyimino acétamido]ceph-3-ème 4-carboxylate de 1-[(1-oxoéthyl) oxy] éthyle isomère syn

On opère comme à l'exemple 34 à partir de 6 g d'acide 3-(éthoxy) méthyl 7-[2-(2-tritylamino thiazol-4-yl) 2-[(1-méthyl-1-méthoxy) éthoxy imino]acétamido]ceph-3-ème 4-carboxylique préparé à l'exemple 8 et 2,1 $cm^3$ d'acétate de 1-bromoéthyle. On obtient 1,76 g de produit attendu.

*Spectre UV* (EtOH)
inf.: 220 nm $E_1^1$ = 268
max.: 264 nm $E_1^1$ = 369 ε = 18900
(EtOH, HCl N/10)
inf.: 219 nm $E_1^1$ = 268
max.: 261 nm $E_1^1$ = 390 ε = 19500

*Spectre RMN* (DMSO) ppm
1—1,12—1,23: $CH_3$ du O—Et
1,45—1,53: $CH_3$—CH
2,06: OAc
6,73: $H_5$ du thiazole

Exemple 36

3-(méthylthiométhyl 7-[2-(2-amino thiazol-4-yl) 2-hydroxyimino acétamido ceph-3-ème 4-carboxylate de 1-(méthoxycarbonyl) oxy] éthyle isomère syn

On opère comme à l'exemple 14 à partir de 3,72 g d'acide 3-méthylthiométhyl 7-[2-(2-tritylamino thiazol-4-yl 2-[(1-méthyl 1-méthoxy éthoxy imino]acétamido]ceph-3-ème 4-carboxylique isomère syn et 3.2 g de carbonate d'iodoéthyle et de méthyle préparé ci-dessous et obtient 1,12 g de produit attendu pur.

*Spectre UV* (EtOH, HCL N/10)
max.: 265 nm $E_1^1$ = 365 ε = 19400

*Spectre RMN* ($CDCl_3$) ppm
1,53—1,63: $CH_3$—CH
2,06: S—$CH_3$
3,83: $COOCH_3$
7,05: $H_5$ du thiazole
6,85 à 7,16: CH—$CH_3$

Le carbonate d'iodoéthyle et de méthyle utilisé dans l'exemple a été préparé comme suit:

a) chloroformiate de 1-chloroéthyle

On fait barboter du chlore dans 200 $cm^3$ de chloroformiate d'éthyle pendant 8 heures, bouche hermétiquement et laisse 5 jours au repos. On concentre sous pression réduite (50 mm Hg) et recueille la fraction distillant entre 42° et 46°C.

b) carbonate chloroéthyle et de méthyle

On mélange sous atmosphère inerte 5 $cm^3$ de chloroformiate déthyle et 16 $cm^3$ de méthanol et agite 1 heure à température ambiante.

c) carbonate d'iodoéthyle et de méthyle

On reprend le mélange par 38 $cm^3$ d'acétone, ajoute 10,9 g d'iodure de sodium, porte 5 minutes à reflux puis laisse revenir à température ambiante. On distille sous pression réduite à une température inférieure à 40°C, reprend le résidu par 100 $cm^3$ d'éther et 75 $cm^3$ d'eau. On décante, extrait la phase aqueuse à l'éther, lave la phase organique à l'eau puis avec une solution aqueuse de métabisulfate de sodium (0,25M) puis à l'eau et enfin avec une solution aqueuse saturée en chlorure de sodium. On sèche, concentre à sec sous pression réduite (température max. 40°C) et utilise le mélange immédiatement.

Exemple 37

3-(méthylthiométhyl 7-[2-(2-amino thiazol-4-yl) 2-hydroxyimino acétamido] ceph-3-ème 4-carboxylate de 1-[(éthoxycarbonyl) oxy] éthyle isomère syn

On opère comme à l'exemple 14 à partir de d'acide 3-méthylthiométhyl 7-[2-(2-tritylamino thiazol-4-yl 2-[(1-méthyl 1-méthoxy éthoxy imino]acétamido]ceph-3-ème 4-carboxylique isomère syn et de carbonate d'iodoéthyle et de d'éthyle.

Le carbonate d'iodoéthyle et d'éthyle a été préparé suivant un mode opératoire identique à celui donné à l'exemple 41 pour la préparation du carbonate d'iodoéthyle et de méthyle.

*Spectre RMN* ($CDCl_3$) ppm
1,2 à 1,45: $CH_3$ du COO—Et.
1,55—1.64: $CH_3$—CH—
6,9: $H_5$ du thiazole
2.08: S—$CH_3$

Exemple 38

3-(méthylthiométhyl 7-[2-(2-amino thiazol-4-yl) 2-hydroxyimino acétamido] ceph-3-ème 4-carboxylate de [(méthoxycarbonyl) oxy] méthyle isomère syn

On opère comme à l'exemple 14 à partir de 5 g d'acide 3-méthylthiométhyl 7-[2-(2-tritylamino thiazol-4-yl) 2-(1-méthyl 1-méthoxy éthoxy imino]acétamido]ceph-3-ème 4-carboxylique isomère syn et de 7,93 g de carbonate monoiodé préparé di-dessous. On obtient 0,537 g de produit pur.

*Spectre UV* (EtOH, HCl N/10)

max.: 265 nm $E_1^1$ = 371 ε = 19200

*Spectre RMN* ($CDCl_3$) ppm.

2,03: S—$CH_3$

3,85: $CO_2CH_3$

6,96: $H_5$ du thiazole

5,91: $CO_2CH_2$

Le carbonate monoiodé a été préparé comme suit:

a) carbonate monochloré

Sous atmosphère inerte on chauffe à 65°C 30 cm$^3$ de diméthyl carbonate et fait barboter du chlore pendant 5 heures, puis abandonne 56 heures au repos. On concentre sous pression réduite et recueille 7,8 cm$^3$ de produit distillant entre 59° et 61°C (50 mm Hg).

b) carbonate monoiodé

On chauffe 1 heure 30 à 30°C 5 cm$^3$ du produit obtenu précédement, 7,8 g d'iodure de sodium et 30 cm$^3$ d'acétone. On concentre à sec, reprend le résidu per 100 cm$^3$ d'éther et 100 cm$^3$ d'eau agite 5 minutes, décante et extrait la phase aqueuse à l'éther. On lave la phase organique avec une solution aqueuse de métabisulfite de sodium (0,25M) puis à l'eau et enfin avec une solution aqueuse saturée en chlorure de sodium. On sèche et évapore à sec sous pression réduite (température maximum 40°C). On recueille 7,93 g de réactif que l'on utilise immédiatement.

Exemple 39

Acide 7-[[2-(2-amino thiazol-4-yl) 2[(carboxyméthoxy) imino] acétyl] amino] 3-(méthylthiométhyl) ceph-3-ème 4-carboxylique isomère syn

Stade A: Sel de triéthylamine de l'acide 7[[2-(2-tritylaminothiazol-4-yl) 2-(hydroxyimino) acétyl] amino] 3-(méthylthiométhyl) ceph-3-ème 4-carboxylique isomère syn

On met en solution 14,88 g de 7-[[2-(2-tritylamino thiazol-4-yl) 2-[(1-méthyl 1-méthoxy) éthoxy imino]acétyl]amino 3-(méthylthiométhyl) ceph-3-ème 4-carboxylique isomère syn dans 60 cm$^3$ d'acétone à l'aide de 20 cm$^3$ d'acide chlorhydrique 2N. On agite 1 heure 10 minutes à température ambiante, chasse l'acétone, dilue avec 100 cm$^3$ d'eau, essore et lave à l'eau. On reprend le produit brut par 60 cm$^3$ d'acétone et 6 cm$^3$ d'eau. On ajoute 2,8 cm$^3$ triéthylamine, amorce la cristallisation et délite par 20 cm$^3$ d'acétone, essore, empâte à l'acétone puis à l'éther. On obtient un premier jet de 11.4 g puis après reprise du résidu à l'acétone 0,85 g d'un deuxième jet, on obtient donc en tout 12,25 g de produit.

*Spectre RMN* (DMSO) ppm

1,96: $CH_3$—S—

6,66: proton en 5 du thiazole

7,36: protons du trityle

Stade B: Sel de triéthylamine de l'acide 7[[2-(2-tritylaminothiazol-4-yl) 2-[(1,1-diméthyléthoxy) carbonylméthoxy imino] acétyl]amino] 3-(méthylthiométhyl) ceph-3-ème 4-carboxylique isomère syn

On mélange 3,09 g de sel de triéthylamine obtenus au stade A avec 30 cm$^3$ d'eau déminéralisée et 60 cm$^3$ de chlorure de méthylène, puis ajoute à 20°C 5 cm$^3$ de triéthylamine puis 4,8 g de bromacétate de tert-butyl. On agite 2 heures 30 à 20—25°C. On acidifie par 10 cm$^3$ d'acide chlorhydrique 2N, décante, lave au chlorure de méthylène puis par deux fois 100 cm$^3$ d'eau. On réextrait les eaux de lavage au chlorure de méthylène (2 fois 20 cm$^3$). On sèche les phases organiques, essore, rince et distille à sec sous vide. On empâte la résine formée à l'éther, essore, rince à l'éther et sèche sous vide à 20°C. On obtient 3 g d'acide. Le produit est repris dans un mélange benzène 7.5 cm$^3$ et triéthylamine 0,7 cm$^3$. Après dissolution on dilue par 75 cm$^3$ d'éther, essore le précipité et rince par 3 foils 2 cm$^3$ d'éther. On sèche le produit obtenu soit 2.4 g. Le produit est utilisé tel quel pour le stade suivant.

*Spectre RMN* ($CDCl_3$) ppm

2,04: $CH_3$—S—

4,76: =N—O—$CH_2$—

6,9: proton en 5 du thiazole

7,36: protons du trityle

Stade C: Acide 7[[2-(2-amino thiazole-4-yl) 2-[(carboxyméthoxy) imino. acétyl]amino] 3-(méthylthiométhyl) ceph-3-ème 4-carboxylique isomère syn

On agite à 20°C pendant 25 minutes un mélange de 2,3 g de sel obtenu stade B dans 11,5 cm$^3$ d'acide trifluoroacétique. On distille au bain d'eau la majorité de l'acide trifluoroacétique. On reprend par 100 cm$^3$ d'éther isopropylique en refroidissant. On agite 30 minutes à 20°C, essore, rince à l'éther isopropylique et

sèche le produit sous vide à 20°C. On obtient 1,957 g de produit brut. On purifie le produit comme suit:

On dissout le produit brut dans um mélange de 5 $cm^3$ de carbonate acide de sodium molaire et 0,3 $cm^3$ de triéthylamine et on dilue la solution par 5 $cm^3$ d'eau salée saturée. On chromatographie sur silice en éluant par de l'eau salée (2M) à 4% de carbonate acide de sodium molaire. On précipcite les élutions à pH4 par de l'acide formique à 50% d'eau. Après lavage et séchage on obtient 0,376 g de produit attendu.

Analyse: $C_{16}H_{17}O_7N_5S_3$ = 487,534

| | | | | |
|---|---|---|---|---|
| Calculé: | C% 39,42 | H% 3,51 | N% 14,36 | S% 19,73 |
| Trouvé: | 39,6 | 3,7 | 14,2 | 18,7 |

*Spectre RMN* (DMSO) ppm

2: $CH_3$—S—

4,63: N—O—$CH_2$—

6,85: proton en 5 du thiazole

Exemple 40

Acide 7-[[2-(2-amino thiazol-4-yl) 2[(éthoxycarbonyloxy) imino]acétyl]amino] 3-(méthylthiomethyl) ceph-3-ème 4-carboxylique isomère syn

Stade A: Sel de triéthylamine de l'acide 7[[2-(2-tritylaminothiazol-4-yl) 2-[(éthoxycarbonyloxy) imino]acétyl] amino] 3-(méthylthiométhyl) ceph-3-ème 4-carboxylique isomère syn

On mélange 2.32 g de sel de triéthylamine obtenu au stade A de l'exemple 44 dans un mélange de 23 $cm^3$ de chlorure de méthylène sec et 0,5 $cm^3$ de pyridine. On ajoute en 3 minutes, à température ambiente 0,6 $cm^3$ de chloroformiate d'éthyle. On abandonne 15 minutes à température ambiante. On ajoute 25 $cm^3$ d'eau contenant 6 $cm^3$ d'acide chlorhydrique normal. On décante et rince la phase aqueuse par 5 $cm^3$ de chlorure de méthylène. On réunit les phases organiques et les sèche. On essore, rince au chlorure de méthylène puis chasse le solvant. On reprend le résidu par 20 $cm^3$ d'acétate d'éthyle et agite jusqu'à dissolution totale. On ajoute 0,5 $cm^3$ de triéthylamine. Le sel précipite. On essore, rince à l'acétate d'éthyle puis à l'éther. On sèche et obtient 1,49 g de produit.

Stade B: Acide 7[[2-(2-amino thiazol-4-yl) 2-[(éthoxycarbonyloxy) imino]acétyl]amino] 3-(méthylthiométhyl) ceph-3-ème 4-carboxylique isomère syn

On reprend la totalité du sel obtenu au stade précédent dans 7,6 $cm^3$ d'acide formique à 67%. On agite 10 minutes à 45°C. On essore le triphénylcarbinol et rince à l'acide formique à 67%. Après séchage on obtient 0,434 g de produit. Le solvant est chassé sous pression réduite à 35°C environ. Le résidu est repris à l'eau. On délite, essore puis rince à l'eau. Au produit brut obtenu, on ajoute 0,319 g de produit brut de façon identique lors d'une autre manipulation. Le produit est mis en solution dans une solution 0,5M de carbonate acide de sodium puis fixé sur 30 g de silice. On élue par de l'eau salée (3M) contenant 4% de carbonate acide de sodium. Les fractions contenant le produit attendu sont acidifiées par de l'acide acétique (pH3). Le produit purifié précipite. On abandonne 30 minutes à température ambiante puis essore et empâte quatre fois à l'eau. On sèche sous pression réduite et obtient 0,798 g de produit attendu.

Analyse: $C_{17}H_{19}O_7N_5S_3$ = 501,56

| | | | | |
|---|---|---|---|---|
| Calculé: | C% 40,7 | H% 3,8 | N% 14,0 | S% 19,2 |
| Trouvé: | 40,7 | 3,7 | 13,9 | 19,3 |

*Spectre RMN* (DMSO) ppm

1,26 (t): $CO_2$ $CH_2$—$CH_3$ J = 7Hz

2: $CH_3$—S—

4,25 (q): $CO_2$—$CH_2$—$CH_3$ J = 7HZ

7,11: proton en 5 du thiazole

**Revendications pour les Etats contractants: BE CH DE GB IT LI LU NL SE**

1. Les produits de formule générale (A'):

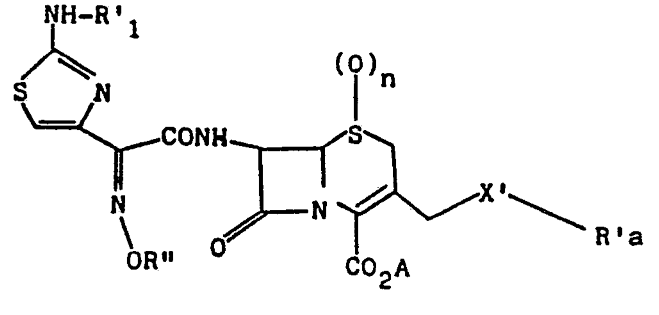

(A')

isomére *syn* dans laquelle R'$_1$, R'', n, A, X' et R'$_a$ ont les significations suivantes

*ou bien* A] A représente un atome d'hydrogène, un équivalent de métal alcalin, alcalino-terreux, de

magnésium, d'ammonium ou d'une base organique aminée ou A représente le reste d'un groupement ester facilement clivable,

$R'_a$ représente un radical alkyle éventuellement interrompu par un hétéroatome, alkényle ou alkynyle ayant au plus 6 atomes de carbone linéaire ou ramifié ou un radical benzyle ou phényléthyle éventuellement substitué par un radical carboxy, amino, ou diméthylaminoéthyle, n est égal à 0, 1 ou 2,

X' représente un atome de soufre éventuellement oxydé sous forme de sulfoxyde ou de sulfone ou un atome d'oxygène et R'' et $R'_1$ sont tels que

*soit* $R'_1$ représente un groupement protecteur du radical amino et R'' représente R', R' représente un atome hydrogène, un groupement protecteur du radical hydroxyle, un radical alkyle linéaire ou ramifié alkényle, alkynyle ou cycloalkyle ayant au plus 6 atomes de carbone, ou bien R' représente un radical acétyle, propionyle, butyryle, valéryle, hexanoyle, acryloyle, crotonoyle, benzoyle, carbamoyle, chacun de ces radicaux étant éventuellement substitué par un ou plusieurs radicaux choisis parmi les radicaux carboxy éventuellement salifié ou estérifié, méthoxycarbonyle, éthoxycarbonoyle, carbamoyle, deméthylcarbamoyle, amino, méthylamino, diméthylamino, halogène, méthoxy, éthoxy, propyloxy, méthylthio, éthylthiophényle, tetrazolyle, phénylthio, tetrazolylthio ou thiadiazolythio éventuellement substitué par méthyle ou bien R' représente un radical alkoxy carbonyle.

*soit* $R'_1$ représente un atome d'hydrogène et R'' représente un groupement protecteur du radical hydroxyle choisi parmi les radicaux phénoxyacétyle, benzoylformoyle, p-nitro benzoyle, propoxycarbonyle, bêta-bêta-bêta-trichloroethoxycarbonyle, benzyloxycarbonyle, tert-butoxycarbonyle, 1-cyclopropyléthoxycarbonyle, tétrahydropyrannyle, tetrahydrothiopyrannyle, méthoxytétrahydropyrannyle, 4-méthoxy benzyle, phtaloyle, isobutyryle, isovaléryle, oxalyle, succinyle, pivaloyle, mesyle, para-tert-butyl benzoyle, caprylyle, acryloyle, méthyl carbamoyle, phénylcarbamoyle ou naphtylcarbamoyle, étant entendu qui R'' ne représente pas un radical alkényle ou alkynyle ayant au plus 6 atomes de carbone ou un radical alkyle linéaire ou ramifié ayant au plus 6 atomes de carbone éventuellement substitué par un ou plusieurs radicaux choisi parmi les radicaux amino, carboxy éventuellement estérifié ou salifié, alkoxycarbonyle, alkylamino, dialkylamino ou aryle hétérocyclique lorsque X'—$R'_a$ représente un radical alkoxy, alkylthio ou alkénylthio.

*ou bien* B] n est égal à O, A représente un atome hydrogène, $R'_1$ représente un groupement protecteur de radical amino et

*soit* R'' représente un radical $—CH_2CO_2H$ ou $—C(CH_3)_2CO_2H$, X' représente un atome d'oxygène ou de soufre er $R'_a$ représente un radical méthyle ou éthyle

*soit* R'' représente un radical

$$—\underset{\underset{CH_3}{|}}{CH}—CO_2H,$$

X' représente un atome d'oxygène ou de soufre et $R'_a$ représente un radical méthyle.

2. Les produits selon la revendication 1, répondant à la formule IV'

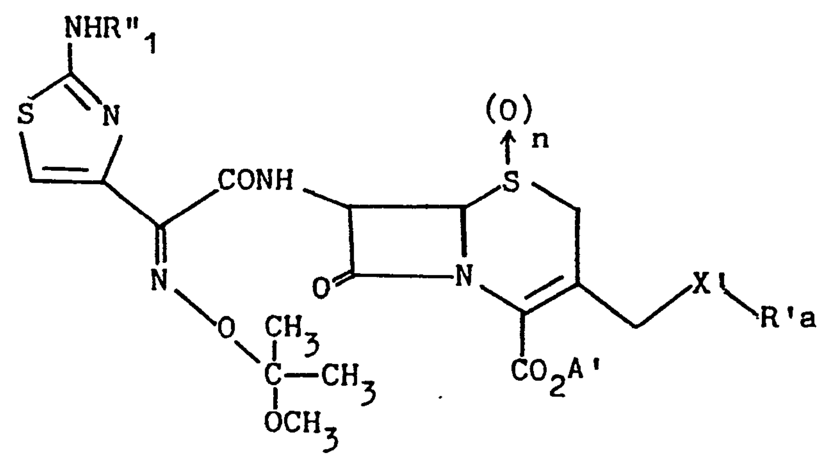

IV'

dans laquelle $R'_a$, X' et n ont a signification indiquée à la revendication 1 et A' représente un atome d'hydrogéne ou le reste d'un groupement ester facilement éliminable et $R''_1$ représente un groupement protecteur du radical amino.

3. Les produits selon la revendication 1, répondant à la formule VI

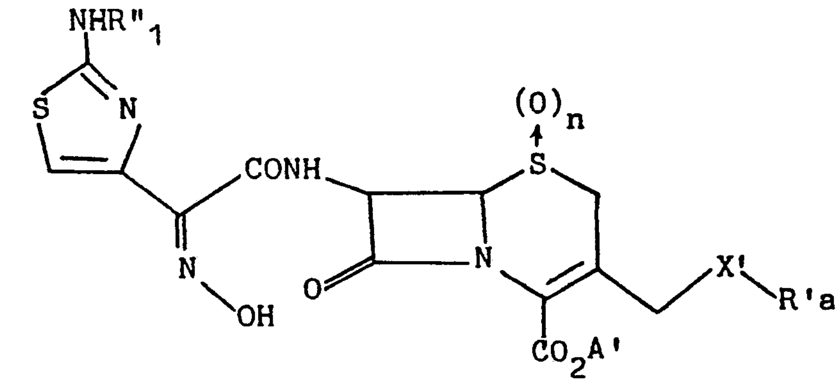

VI

dans laquelle $R'_a$, X' et n ont a signification indiquée à la revendication 1, $R''_1$ et A' ont la signification indiquée à la revendication 2.

4. Les produits selon la revendication 1 répondant à la formule générale A

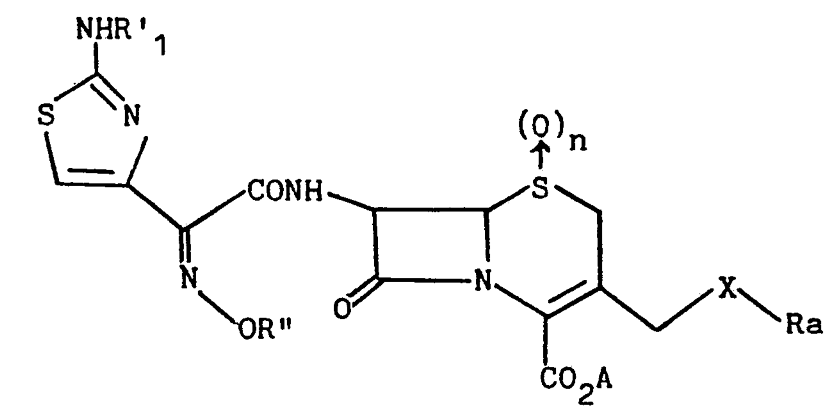

dans laquelle $R'_1$, A, R'' et n ont a signification indiquée à la revendication 1, X représente un atome de soufre ou un atome d'oxygène et Ra représente un radical alkyle, alkényle ou alkynyle ayant au plus t atomes de carbone linéaire ou ramifié ou un radical benzyle ou phényléthyle et X représente un atome de soufre ou un atome d'oxygène.

5. Les produits de formule A' telle que définie à l'une des revendications 1 à 3 dans laquelle $R'_a$ représente un radical méthyle et n représente le nombre 0.

6. Les produits de formule A' telle que définie à la revendication 1 répondant aux formules suivantes:

L'acide 7-[[2-(2-tritylamino thiazol-4-yl)2-(hydroxyimino) acétyl]amino]3-(méthylthiométhyl)ceph-3-ème 4-carboxylique isomère *syn.*

L'acide 3-méthylthiométhyl 7-[2-(2-tritylamino thiazol-4-yl)2-[(méthyl 1-méthoxy)éthoxy imino]acétamido])ceph-3-ème 4-carboxylique isomère syn.

7. Procédé de préparation des produits de formule générale A' telle que définie à la revendication 1, caractérisé en ce que l'on traite un produit de formule (II').

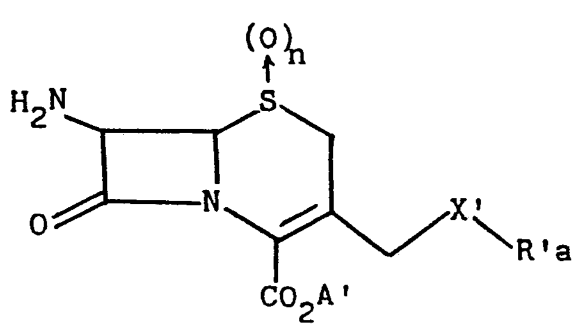

(II')

dans laquelle n, X' et $R'_a$ ont a signification indiquée à la revendication 1 et A' représente un atome d'hydrogène ou le reste d'un groupement ester facilement éliminable, par un produit de formule ($III_A$)

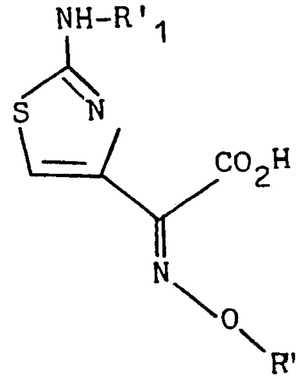

$(III_A)$

ou un dérivé fonctionel de cet acide, formule ($III_A$) dans laquelle $R'_1$ et R'' sont tels que définis à la revendication 1 et que l'on estérifie ou salifie éventuellement le produit obtenu.

8. Procédé de préparation des produits de formule IV' et VI telles que définies aux revendications 2 ou 3 caractérisé en ce que l'on traite un produit de formule (II')

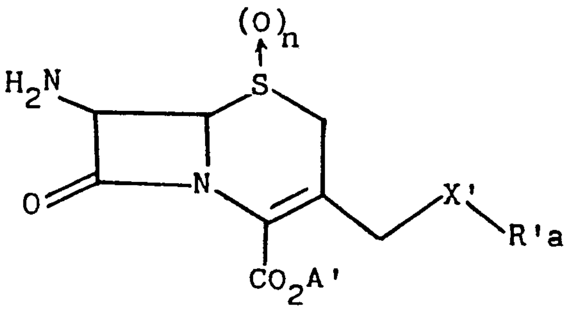

(II')

dans laquelle $R'_a$, n et X' ont a signification indiquée à la revendication 1, et A' a la signification indiquée à la revendication 2 par un produit de formule (III')

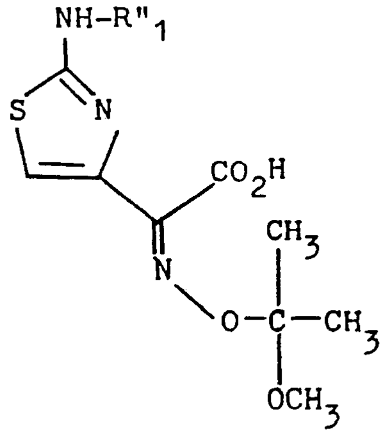

(III')

ou un dérivé fonctionnel de cet acide, formule (III') dans laquelle $R''_1$ représente un groupement protecteur du radical amino, pour ontenir un produit de formule (IV')

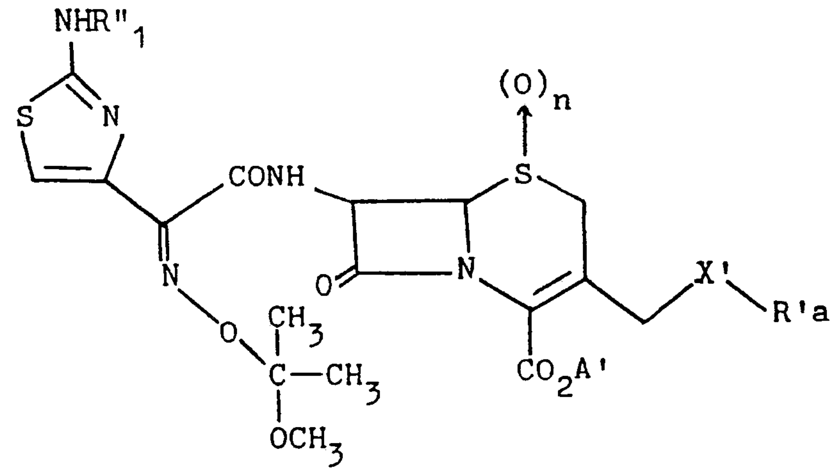

(IV')

dans laquelle A', $R''_1$, $R'_a$, X' et n ont la signification précédente, produit de formule (IV') que l'on traits si désire par un acide dan des conditions modérées, pour obtenir un produit de formule VI

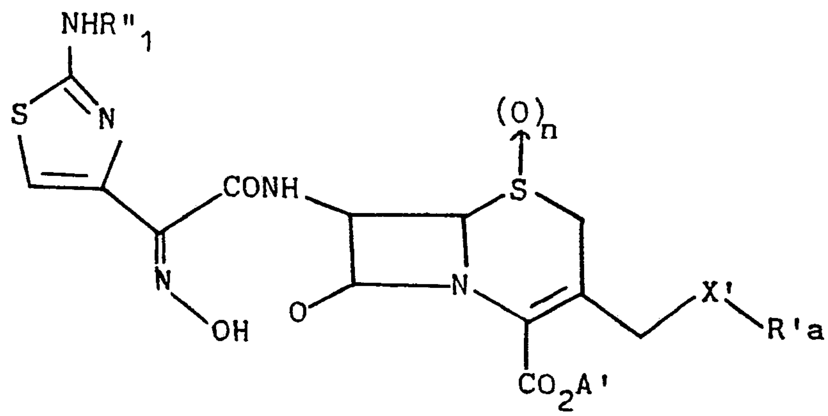

(VI)

dans laquelle $R''_1$, X', $R'_a$, A' et n ont la signification précedente.

**Revendications pour l'Etat contractant: AT**

1. Procéde de préparation des produits de formule générale (A'):

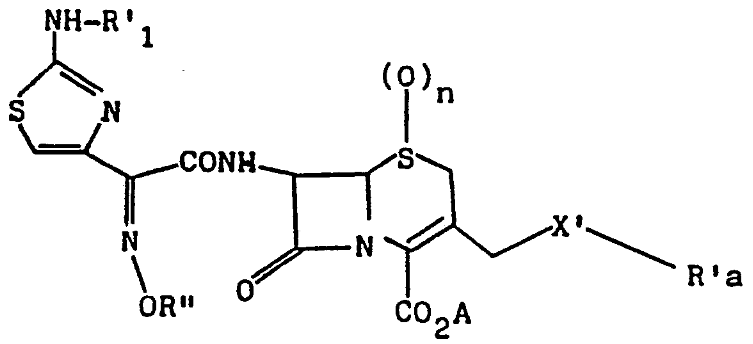

(A')

isomére *syn* dans laquelle $R'_1$, R'', n, A, X' et $R'_a$ ont les significations suivantes:

*ou bien* A] A représente un atome d'hydrogèn, un équivalent de métal alcalin, alcalino-terreux, de magnésium, d'ammonium ou d'une base organique aminée ou A représente le reste d'un groupement ester facilement clivable, $R'_a$ représente un radical alkyle éventuellement interrompu par un hétéroatome, alkényle ou alkynyle ayant au plus 6 atomes de carbone linéaire ou ramifié ou un radical benzyle ou phényléthyle éventuellement substitué par un radical carboxy, amino, ou diméthylaminoéthyle, n est égal

à 0, 1 ou 2, X' représente un atome de soufre éventuellement oxydé sous forme de sulfoxyde ou de sulfone ou un atome d'oxygène et R'' et $R'_1$ sont tels que

*soit* $R'_1$ représente un groupement protecteur du radical amino et R'' représente R', R' représente un atome d'hydrogène, un groupement protecteur du radical hydroxyle, un radical alkyle linéaire ou ramifié alkényle, alkynyle ou cycloalkyle ayant au plus 6 atomes de carbone, ou bien R' représente un radical acétyle, propionyle, butyryle, valéryle, hexanoyle, acryloyle, crotonoyle, benzoyle, carbamoyle, chacun de ces radicaux étant éventuellement substitué par un ou plusieurs radicaux choisis parmi les radicaux carboxy éventuellement salifié ou estérifié, méthoxycarbonyle, éthoxycarbonoyle, carbamoyle, deméthylcarbamoyle, amino, méthylamino, diméthylamino, halogène, méthoxy, éthoxy, propyloxy, méthylthio, éthylthiophényle, tetrazolyle, phénylthio, tetrazolylthio ou thiadiazolythio éventuellement substitué par méthyle ou bien R' représente un radical alkoxy carbonyle.

*soit* $R'_1$ représente un atome d'hydrogène et R'' représente un groupement protecteur du radical hydroxyle choisi parmi les radicaux phénoxyacétyle, benzoylformoyle, p-nitro benzoyle, propoxycarbonyle, bêta-bêta-bêta-trichloroethoxycarbonyle, benzyloxycarbonyle, tert-butoxycarbonyle, 1-cyclopropyléthoxycarbonyle, tétrahydropyrannyle, tetrahydrothiopyrannyle, méthoxytétrahydropyrannyle, 4-méthoxy benzyle, phtaloyle, isobutyryle, isovaléryle, oxalyle, succinyle, pivaloyle, mesyle, para-tert-butyl benzoyle, caprylyle, acryloyle, méthyl carbamoyle, phénylcarbamoyle ou naphtylcarbamoyle, étant entendu qui R'' ne représente pas un radical alkényle ou alkynyle ayant au plus 6 atomes de carbone ou un radical alkyle linéaire ou ramifié ayant au plus 6 atomes de carbone éventuellement substitué par un ou plusieurs radicaux choisi parmi les radicaux amino, carboxy éventuellement estérifié ou salifié, alkoxycarbonyle, alkylamino, dialkylamino ou aryle hétérocyclique lorsque $X'—R'_a$ représente un radical alkoxy, alkylthio ou alkénylthio.

*ou bien* B] n est égal à O, A représente un atome d'hydrogène, $R'_1$ représente un groupement protecteur de radical amino et

*soit* R'' représente un radical $—CH_2CO_2H$ ou $—C(CH_3)_2CO_2H$, X' représente un atome d'oxygène ou de soufre er $R'_a$ représente un radical méthyle ou éthyle

*soit* R'' représente un radical

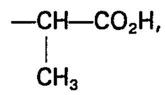

X' représente un atome d'oxygène ou de soufre et $R'_a$ représente un radical méthyle, caractérisé en ce que l'on traite un produit de formule (II')

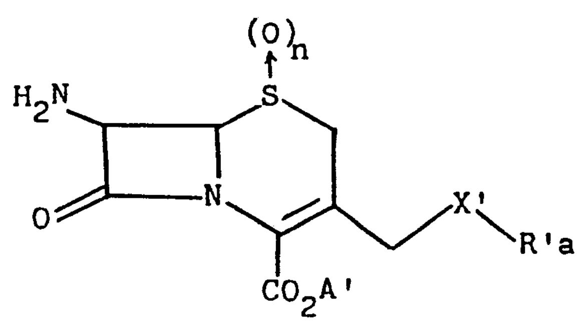

(II')

dans laquelle n, X' et $R'_a$ ont la signification indiquée ci-dessus et A' représenté un atome d'hydrogène ou le reste d'un groupement ester facilement éliminable, par un produit de formule ($III_A$:)

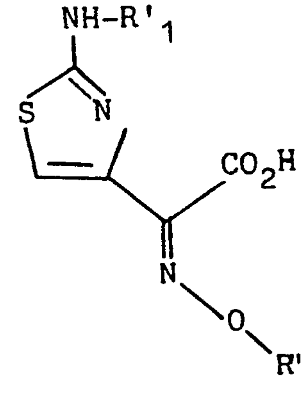

($III_A$)

ou un dérivé fonctionnel de cet acide, formule ($III_A$) dans laquelle $R'_1$ et R'' sont tels que définis ci-dessus et que l'on estérife ou salifie éventuellement le produit obtenu.

2. Procédé de préparation des produits de formule IV':

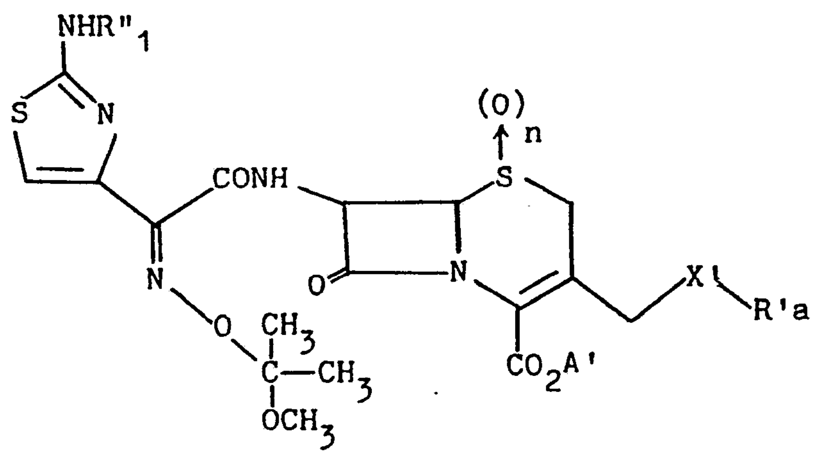

IV'

dans laquelle $R'_a$, X' et n ont la signification indiquée à la revendication 1 et A' représenté un atome d'hydrogène ou le reste d'un groupement ester facilement éliminable et $R''_1$ représenté un groupement protecteur du radical amino, et de formule VI:

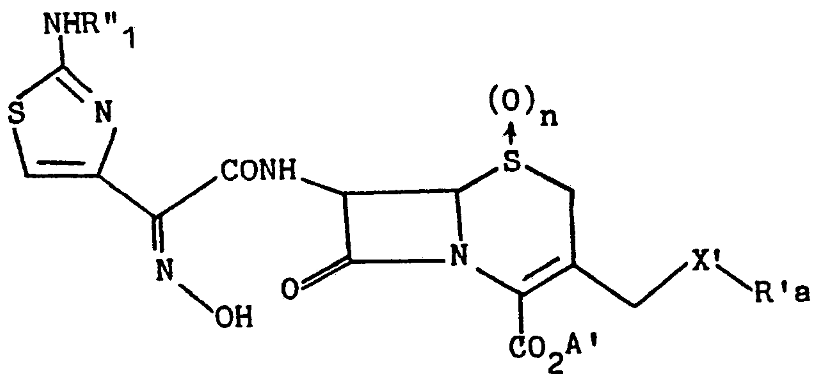

VI

dans laquelle $R'_a$, X' et n ont la signification indiquée à la revendication 1, $R''_1$ et A ont la signification indiquée ci-dessus, caractérisé en ce que l'on traite un produit de formule (II'):

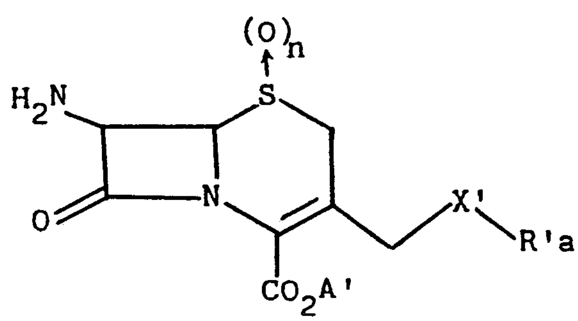

(II')

dans laquelle $R'_a$, n et X' ont la signification indiquée à la revendication 1, et A' a la signification indiquée à la revendication 2 par un produit de formule (III')

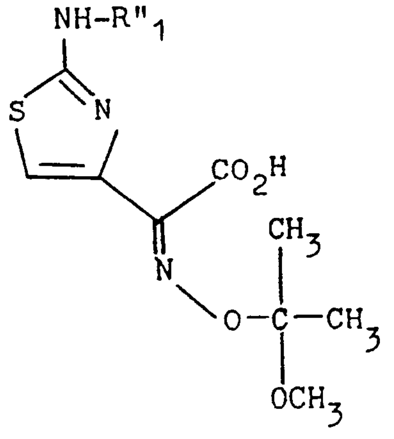

(III')

ou un dérivé fonctionnel de cet acide, formule (III') dans laquelle $R''_1$ représente un groupement protecteur du radical amino, pour obtenir un produit de formule (IV')

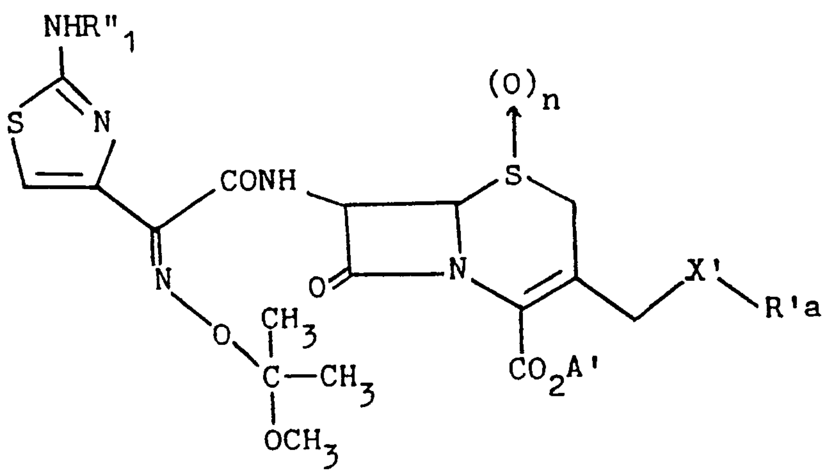

(IV')

dans laquelle A', $R''_1$, $R'_a$, X' et n ont la signification précédente, produit de formule (IV') que l'on traite si désiré par un acide dans des conditions modérées, pour obtenir un produit de formule VI

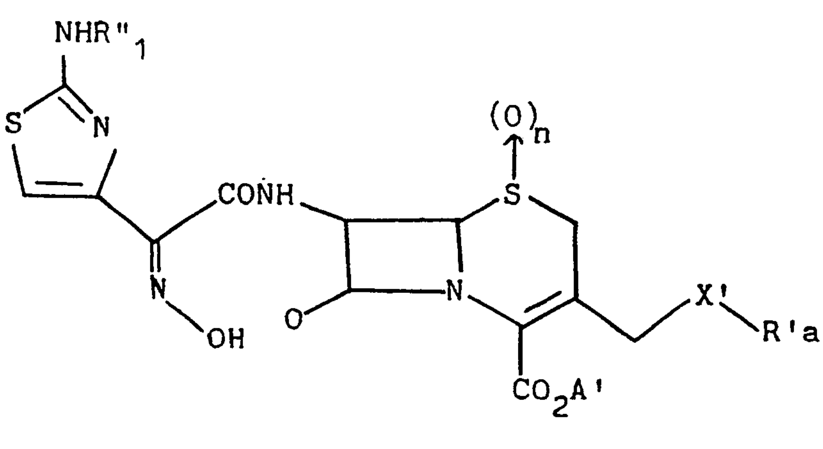

(VI)

dans laquelle $R''_1$, X', $R'_a$, A' et n ont la signification précédente.

3. Procéde selon l'une des revendications 1 à 3, caractérisé en ce que l'on prépare les produits répondant à la formule générale A:

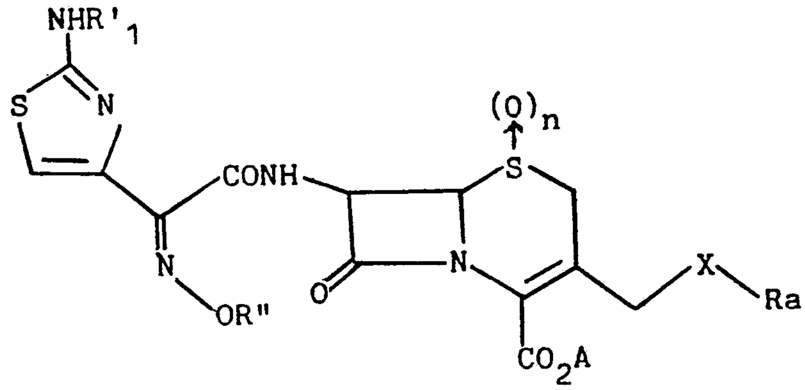

dans laquelle $R'_1$, A', R'' et no ont signification indiquée à la revendication 1, X représente un atome de soufre ou un atome d'oxygène et Ra représente un radical alkyle, alkényle ou alkynyle ayant au plus 6 atomes de carbone linéaire ou ramifie ou un radical benzyle ou phényléthyle et X représente un atome de soufre ou un atome d'oxygène.

4. Procéde selon l'une des revendications 1 à 3, caractérisé en ce que l'on prépare un produit de formule A' dans laquelle $R'_a$ représente un radical méthyle et n représente le nombre 0.

5. Procéde selon l'une des revendications 1 à 4, caractérisé en ce que l'on prépare un produit de formule A', répondant aux formules suivantes:

l'acide 7-[[2-(2-tritylamino thiazol-4-yl)2-(hydroxyimino) acétyl]amino]3-(méthylthiométhyl)ceph-3-ème 4-carboxylique isomère *syn.*

l'acide 3-méthylthiométhyl 7-[2-(2-tritylamino thiazol-4-yl)2-[(méthyl 1-méthoxy)éthoxy imino]acétamido])ceph-3-ème 4-carboxylique isomère syn.

**Patentansprüche für die Vertragsstaaten: BE CH DE GB IT LI LU NL SE**

1. Verbindungen der allgeneinen Formel (A'),

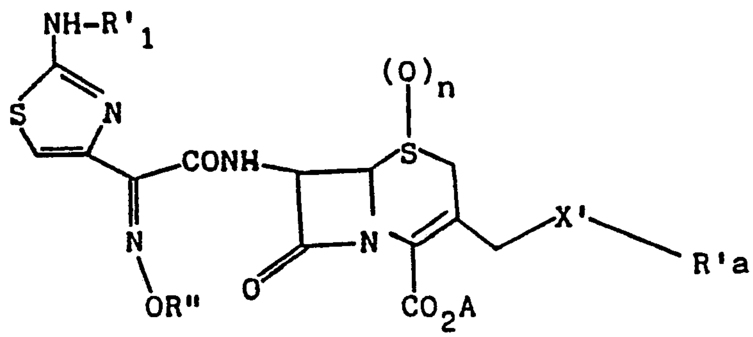

(A')

syn-Isomer in der $R'_1$, R'', n, A, X', und R'a die folgenden Bedeutungen haben:

A) A bedeutet: Wasserstoff, Alkalimetall, Erdalkalimetall, Magnesium, Ammonium oder eine organische Aminobase oder einen leicht abspaltbaren Esterrest, R'a bedeutet: geradkettiges oder verzweigtes Alkyl, gegebenenfalls durch einem Heteratom unterbrochen, Alkenyl oder Alkinyl mit höchstens 6 Kohlenstoffatomen oderBenzyl oder Phenylethyl, gegebenenfalls mit Carboxyl, Amino oder Dimethylaminoethyl substituiert, n is 0, 1 oder 2, X' bedeutet gegebenenfalls oxidierten Schwefel in Form eines Sulfoxyds oder Sulfons oder Sauerstoff, und R'' und $R'_1$ sind so, daß $R'_1$ entweder eine Aminoschutzgruppe und R'' R' bedeutet, wobei R' Wasserstoff, eine Hydroxylschutzgruppe, geradkettiges oder verzweigtes Alkyl, Alkenyl, Alkinyl oder Cycloalkyl mit höchstens 6 Kohlenstoffatomen ist,

oder R' Acetyl, Propionyl, Butyryl, Valeryl, Hexanoyl, Acryloyl, Crotonoyl, Benzoyl oder Carbamoyl ist, wobei jeder dieser Reste gegebenenfalls mit einem oder mehreren Resten substituiert ist, ausgewählt unter Carboxyl, gegebenenfalls als Salz oder Ester, Methoxycarbonyl, Ethoxycarbonyl, Carbamoyl, Dimethylcarbamoyl, Amino, Methylamino, Dimethylamino, Halogen, Methoxy, Ethoxy, Propyloxy, Methylthio, Ethylthiophenyl, Tetrazolyl, Phenylthio, Tetrazolylhio oder Thiadiazolylthio, gegebenenfalls mit Methyl substituiert, oder R' ist Alkoxycarbonyl, oder, $R'_1$ bedeutet Wasserstoff und R'' eine Hydroxylschutzgruppe, ausgewählt unter Phenoxyacetyl, Benzoylformyl, p-Nitrobenzoyl, Propoxycarbonyl, β,β,β-Trichlorethoxycarbonyl, Benzyloxycarbonyl, t-Butoxycarbonyl, 1-Cyclopropylethoxycarbonyl, Tetrahydropyranyl, Tetrahydrothiopyranyl, Methoxytetrahydropyranyl, 4-Methoxybenzyl, Phthaloyl, Isobutyryl,

Isovaleryl, Oxalyl, Succinyl, Pivaloyl, Mesyl, p-t-Butylbenzoyl, Caprylyl, Acryloyl, Methylcarbamoyl, Phenylcarbamoyl oder Naphthylcarbamoyl,

wobei R'' nicht Alkenyl oder Alkinyl mit höchstens 6 Kohlenstoffatomen oder geradkettiges oder verzweigtes Alkyl mit höchstens 6 Kohlenstoffatomen ist, das gegebenenfalls mit einem oder mehreren Resten, substituiert ist, ausgewählt unter Amino, Carboxyl, gegebenenfalls als Ester oder Salz, Alkoxycarbonyl, Alkylamino, Di-alkylamino oder heterozyklisches Aryl, wenn X'—R'a Alkoxy, Alkylthio oder Alkenylthio ist, oder B) n = 0, A ist Wasserstoff, $R'_1$ eine Aminoschutzgruppe und R'' entweder $—CH_2CO_2H$ oder $—C(CH_3)_2CO_2H$, X' Sauerstoff oder Schwefel und R'a Methyl oder Ethyl, oder R'' ist

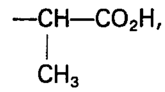

X' Sauerstoff oder Schwefel und R'a Methyl.

2. Verbindungen nach Anspruch 1 der allgemeinen Formel (IV'),

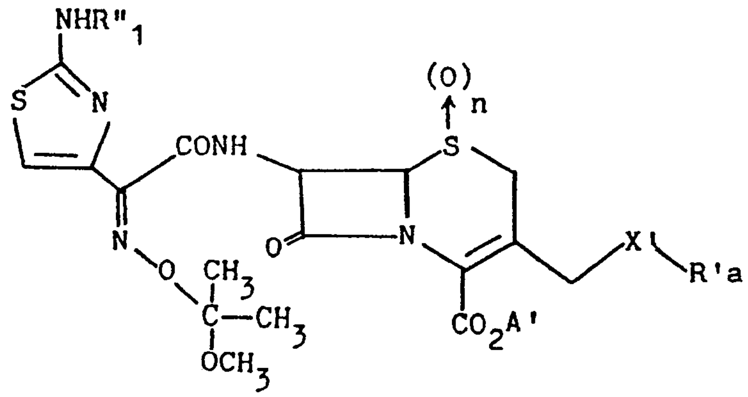

IV'

in der R'a, X' und n die in Anspruch 1 angegebene Bedeutung haben,

A' Wasserstoff oder ein leicht abspaltbarer Esterrest und

$R''_1$ eine Aminoschutzgruppe ist.

3. Verbindungen nach Anspruch 1 der Formel (VI)

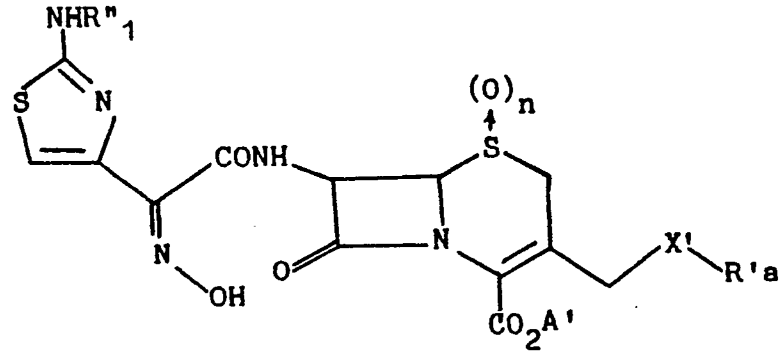

VI

in der R'a, X' und n die in Anspruch 1 angegebene Bedeutung und $R''_1$ und A' die in Anspruch 2 angegebene Bedeutung haben.

4. Verbindungen nach Anspruch 1 der allgemeinen Formel (A)

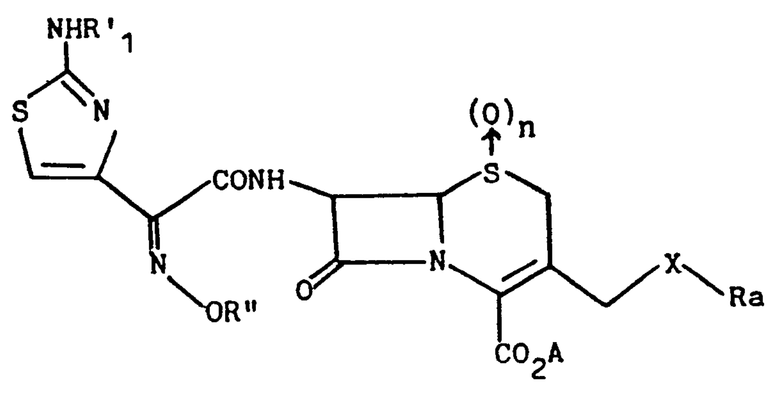

in der $R'_1$, A, R'' und n die in Anspruch 1 angegebene Bedeutung haben,

X Schwefel ider Sauerstoff bedeutet,

Ra geradkettiges oder verzweigtes Alkyl, Alkenyl oder Alkinyl mit höchstens 6 Kohlenstoffatomen oder Benzyl oder Phenethyl ist und

X Schwefel oder Sauerstoff bedeutet.

5. Verbindungen nach einem der Ansprüche 1 bis 3 der Formel (A'), in der R'a Methyl und n 0 ist.

6. Verbindungen nach Anspruch 1 der Formel (A'):

7-[[2-Tritylamino-thiazol-4-yl)-2-(hydroxyimino)-acetyl]-amino]-3-(methylthiomethyl)-ceph-3-em-4-carbonsäure-syn-isomer

3-Methylthiomethyl-7-[2-tritylamino-thiazol-4-yl)-2-[1-methyl-1-methoxy)-ethoxyimino]-acetamido]-ceph-3-em-4-carbonsäure-syn-isomer.

7. Verfahren zur Herstellung der Verbindungen der allgemeinen Formel (A') nach Anspruch 1, dadurch gekennzeichnet, daß eine Verbindung der Formel (II'),

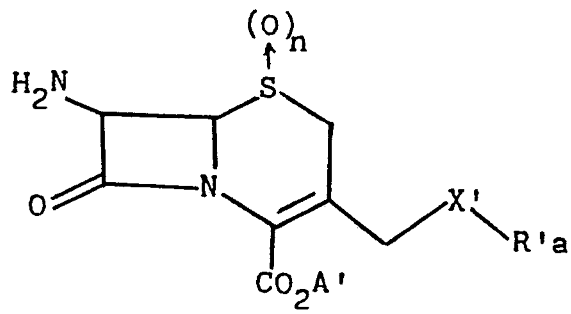

(II')

in der n, X' und R'a die in Anspruch 1 angegebebe Bedeutung haben und A' Wasserstoff oder ein leicht abspaltbarer Esterrest ist,

mit einer Verbindung der Formel ($III_A$) oder einem ihrer funktionellen Derivate umgesetzt wird

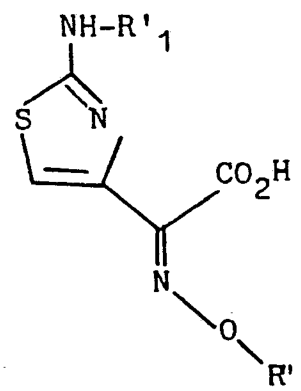

($III_A$)

in der $R'_1$ und R'' wie in Anspruch 1 definiert sind, und aus dem erhaltenen Produkt gegebenenfalls ein Ester oder Salz gebildet wird.

8. Verfahren zur Herstellung der Verbindung der Formeln (IV') und (VI) nach Anspruch 2 oder 3, dadurch gekennzeichnet, daß eine Verbindung der Formel (II'),

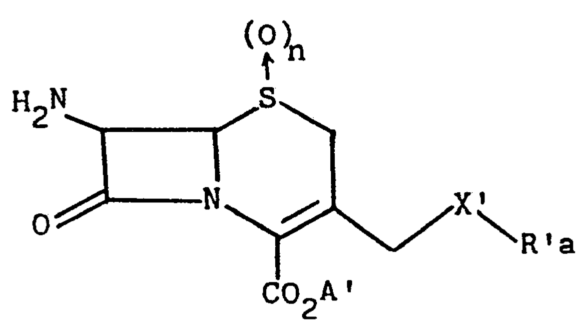

(II')

in der R'a, n und X' die in Anspruch 1 angegebene Bedeutung haben und A' die in Anspruch 2 angegebene Bedeutung hat,

mit einer Verbindung der Formel (III') oder einem ihrer funktionellen Derivate,

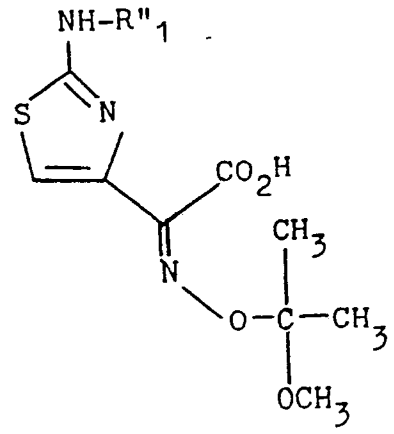

(III')

in der $R''_1$ eine Aminoschutzgruppe ist zu einer Verbindung der Formel (IV') umgesetzt wird,

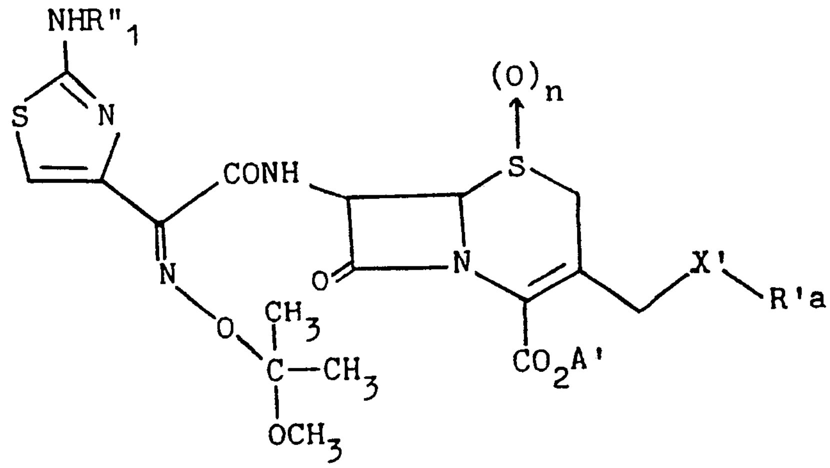

(IV')

in der A', $R''_1$, R'a, X' und n die obenangegebene Bedeutung haben, die Verbindung der Formel (IV') gegebenenfalls mit einer Säure unter milden Bedingungen zu einer Verbindung der Formel (VI) umgesetzt wird,

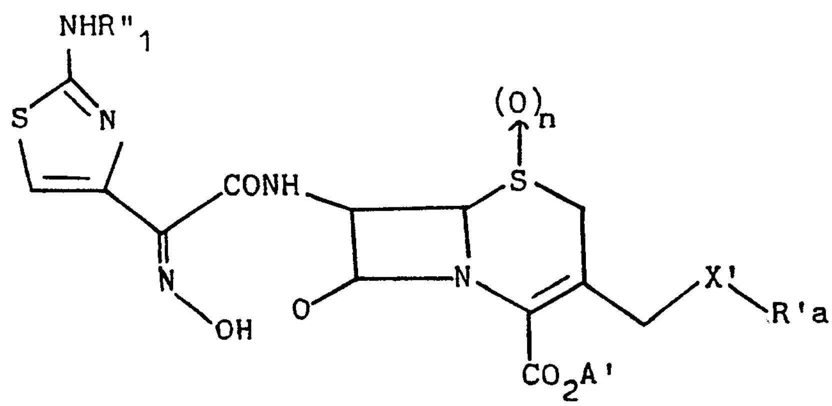

(VI)

in der $R''_1$, X', R'a, A' und n die obenangegebene Bedeutung haben.

**Patentansprüche für den Vertragsstaat: AT**

1. Verfahren zur Herstellung von Verbindungen der allgemeinen Formel (A'),

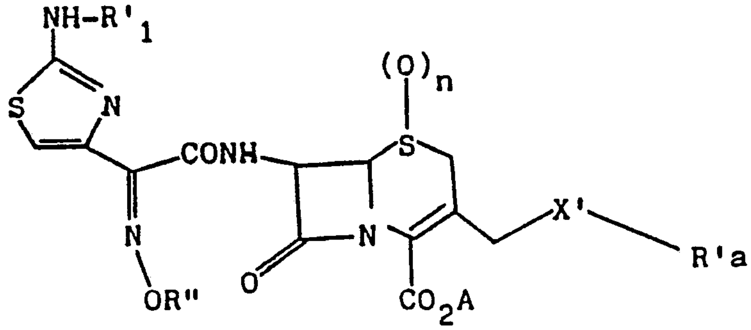

(A')

syn-Isomer in der $R'_1$, R'', n, A, X', und R'a die folgenden Bedeutungen haben;

A) A bedeutet: Wasserstoff, Alkalimetall, Erdalkalimetall, Magnesium, Ammonium oder eine organische Aminobase oder einen leicht abspaltbaren Esterrest, R'a bedeutet: geradkettiges oder verzweigtes Alkyl, gegebenenfalls durch einem Heteratomen unterbrochen, Alkenyl oder Alkinyl mit höchstens 6 Kohlenstoffatomen odr Benzyl oder Phenylethyl, gegebenenfalls mit Carboxyl, Amino oder Dimethylaminoethyl substituiert, n is 0, 1 oder 2, X' bedeutet gegebenenfalls oxidierten Schwefel in Form eines Sulfoxyds oder Sulfons oder Sauerstoff, und R'' und $R'_1$ sind so, daß $R'_1$ entweder eine Aminoschutzgruppe und R'' R' bedeutet, wobei R' Wasserstoff, eine Hydroxylschutzgruppe, geradkettiges oder verzweigtes Alkyl, Alkenyl, Alkinyl oder Cycloalkyl mit höchstens 6 Kohlenstoffatomen ist,

oder R' Acetyl, Propionyl, Butyryl, Valeryl, Hexanoyl, Acryloyl, Crotonoyl, Benzoyl oder Carbamoyl ist, wobei jeder dieser Reste gegebenenfalls mit einem oder mehreren Resten substituiert ist, ausgewählt unter Carboxyl, gegebenenfalls als Salz oder Ester, Methoxycarbonyl, Ethoxycarbonyl, Carbamoyl, Dimethylcarbamoyl, Amino, Methylamino, Dimethylamino, Halogen, Methoxy, Ethoxy, Propyloxy, Methylthio, Ethylthiophenyl, Tetrazolyl, Phenylthio, Tetrazolylthio oder Thiadiazolylthio, gegebenenfalls mit Methyl substituiert, oder R' ist Alkoxycarbonyl, oder, $R'_1$ bedeutet Wasserstoff und R'' eine Hydroxylschutzgruppe, ausgewählt unter Phenoxyacetyl, Benzoylformyl, p-Nitrobenzoyl, Propoxycarbonyl, β,β,β-Trichlorethoxycarbonyl, Benzyloxycarbonyl, t-Butoxycarbonyl, 1-Cyclopropylethoxycarbonyl, Tetrahydropyranyl, Tetrahydrothiopyranyl, Methoxytetrahydropyranyl, 4-Methoxybenzyl, Phthaloyl, Isobutyryl, Isovaleryl, Oxalyl, Succinyl, Pivaloyl, Mesyl, p-t-Butylbenzoyl, Caprylyl, Acryloyl, Methylcarbamoyl, Phenylcarbamoyl oder Naphthylcarbamoyl

wobei R'' nicht Alkenyl oder Alkinyl mit höchstens 6 Kohlenstoffatomen oder geradkettiges oder verzweigtes Alkyl mit höchstens 6 Kohlenstoffatomen ist, das gegebenenfalls mit einem oder mehreren Resten substituiert ist, ausgewählt unter Amino, Carboxyl, gegebenenfalls als Ester oder Salz, Alkoxycarbonyl, Alkylamino, Di-alkylamino oder heterozyklisches Aryl, wenn X'—R'a Alkoxy, Alkylthio oder Alkenylthio ist,

oder B) n = 0, A ist wasserstoff, $R'_1$ eine Aminoschutzgruppe und R'' entweder $—CH_2CO_2H$ oder $—C(CH_3)_2CO_2H$, X' Sauerstoff oder Schwefel und R'a Methyl oder Ethyl, oder

$$—\underset{\displaystyle CH_3}{\underset{|}{CH}}—CO_2H,$$

X' Sauerstoff oder Schwefel und R'a Methyl,
dadurch gekennzeichnet, daß eine Verbindung der Formel (II'),

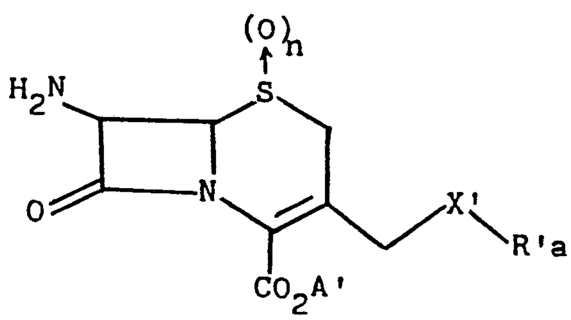

(II')

in der n, X' und R'a die oben angegebene Bedeutung haben und A' Wasserstoff oder ein leicht abspaltbarer Esterrest ist mit einer Verbindung der Formel ($III_A$) oder einem ihrer funktionellen Derivate umgesetzt wird,

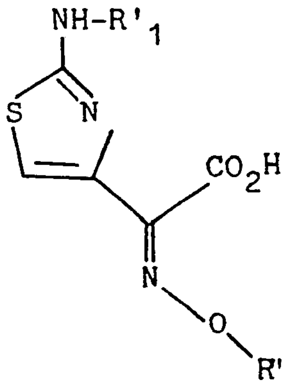

(IIIA)

in der $R'_1$ und R'' wie oben definiert sind, und aus dem erhaltenen Produkt gegenbenefalls ein Ester oder Salz gebildet wird.

2. Verfahren zur Herstellung von Verbindungen der Formel (IV'),

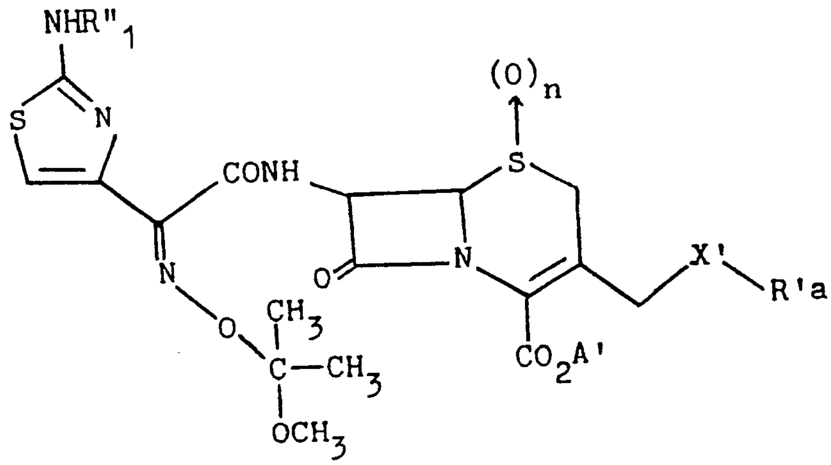

(IV')

in der R'a, X' und n die in Anspruch 1 angegebene Bedeutung haben,

A' Wasserstoff oder ein leicht abspaltbarer Esterrest und

$R''_1$ eine Aminoschutzgruppe ist,

und von Verbindungen der Formel (VI),

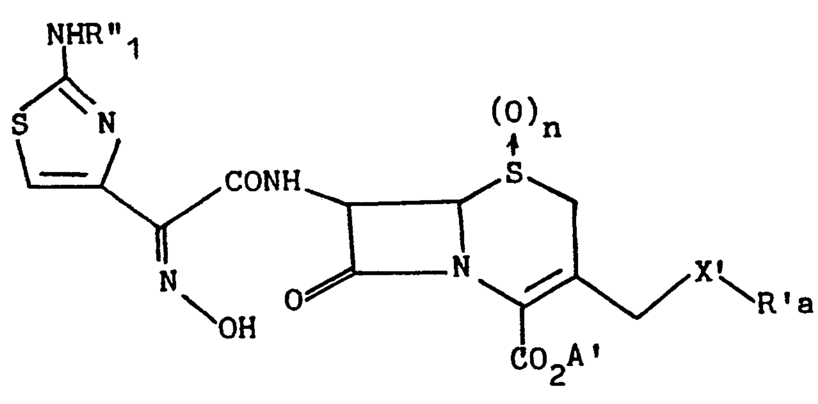

VI

in der R'a, X' und n die in Anspruch 1 angegebene Bedeutung und $R''_1$ und A' die oben angegene Bedeutung haben dadurch gekennzeichnet, daß eine Verbindung der Formel (II'),

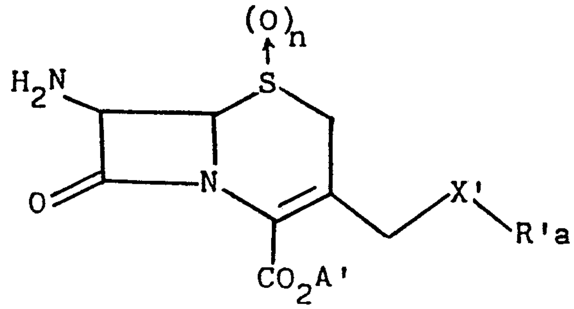

(II')

in der R'a, n und X' die in Anspruch 1 angegebene Bedeutung haben und A' die in Anspruch 2 angegebene Bedeutung hat, mit einer Verbindung der Formel (III') oder einem ihrer funktionellen Derivate,

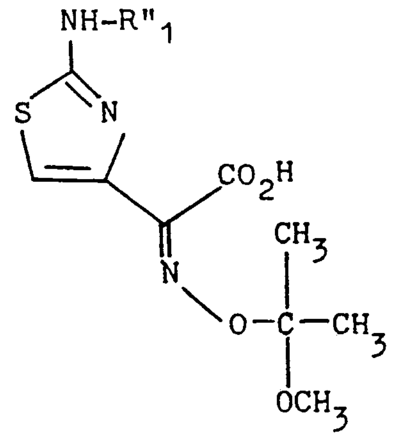

(III')

in der R''$_1$ eine Aminoschutzgruppe ist, zu einer Verbindung der Formel (IV'), umgesetzt wird,

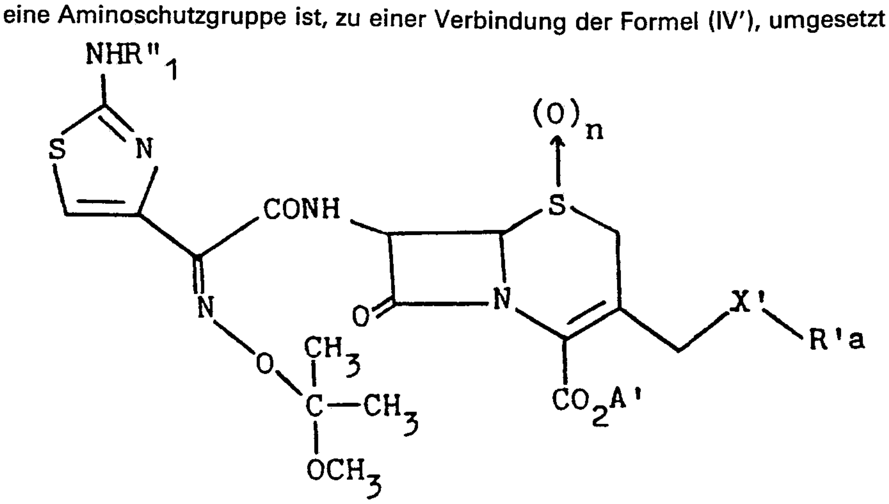

(IV')

in der A', R''$_1$, R'a, X' und n die obenangegebene Bedeutung haben, die Verbindung der Formel (IV') gegebenenfalls mit einer Säure unter milden Bedingungen zu einer Verbindung der Formel (VI) umgesetzt wird,

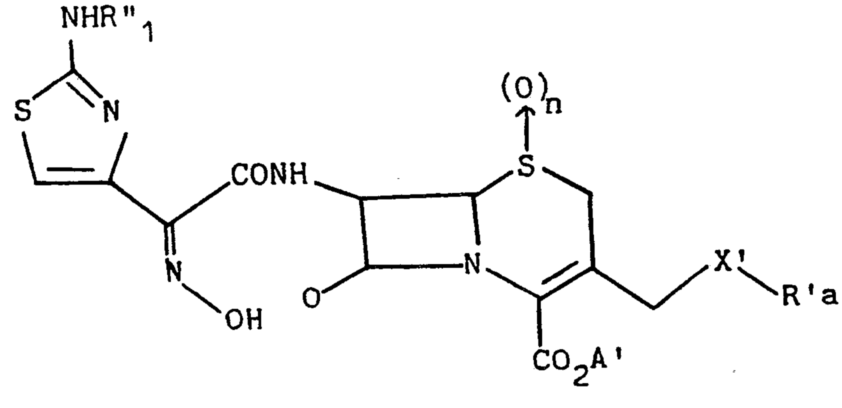

(VI)

in der $R''_1$, X', R'a, A'und n die obenangegebene Bedeutung haben.

3. Verfahren nach einem der Ansprüche 1 bis 3, dadurch gekennzeichnet, daß Verbindungen der allgemeinen Formel (A) hergestellt werden,

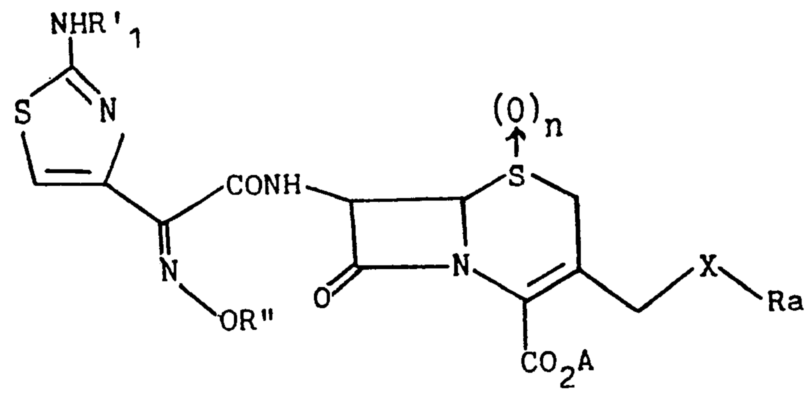

A

in der $R'_1$, A, R'' und n die in Anspruch 1 angegebene Bedeutung haben,

X Schwefel oder Sauerstoff bedeutet,

Ra geradkettiges oder verzweigtes Alkyl, Alkenyl oder Alkinyl mit höchstens 6 Kohlenstoffatomen oder Benzyl oder Phenethyl ist und

X Schwefel oder Sauerstoff bedeutet.

4. Verfahren nach einem der Ansprüche 1 bis 3, dadurch gekennzeichnet, daß eine Verbindung der Formel (A') hergestellt wird, in der R'a Methyl und n 0 ist.

5. Verfahren nach einem der Ansprüche 1 bis 4, dadurch gekennzeichnet, daß eine der folgenden Verbindungen der Formel (A') hergestellt wird:

7-[[2-(2-Tritylamino-thiazol-4-yl)-2-(hydroxyimino)-acetyl]-amino]-3-(methylthiomethyl)-ceph-3-em-4-carbonsäure-syn-isomer

3-Methylthiomethyl-7-[2-(2-tritylamino-thiazol-4-yl)-2-[(1-methyl-1-methoxy)-ethoxyimino]-acetamido]-ceph-3-em-4-carbonsäure-syn-isomer.

**Claims for the Contracting States: BE CH DE GB IT LI LU NL SE**

1. Products of general formula (A'):

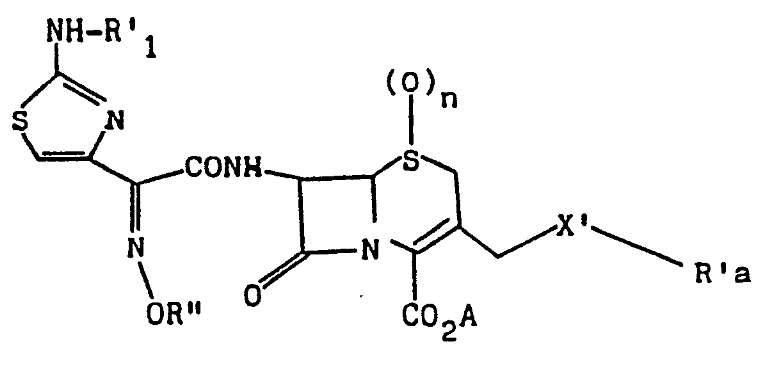

(A')

*syn* isomer in which $R'_1$, R'', n, A, X', and R'a have the following significance

either A] A represents a hydrogen atom, an equivalent of an alkali metal, of an alkaline-earth metal, of magnesium, of ammonium or of an aminated organic base or A represents the remainder of an ester group that can be easily cleaved, R'a represents an alkyl radical optionally interrupted by a heteroatom, linear or branched alkenyl or alkynyl radical having at most 6 carbon atoms or a benzyl or phenylethyl radical optionally substituted by a carboxy, amino or dimethylaminoethyl radical, n is equal to 0, 1 or 2. X' represents a sulphur atom optionally oxidised in the form of sulphoxide or sulphone or an oxygen atom and R'' and $R'_1$ are such that

either $R'_1$ represents a protector group of the amino radical and R'' represents R', R' represents a hydrogen atom, a protector group of the hydroxyl radical, a linear or branched alkyl radical, alkenyl, alkynyl or cycloalkyl radical having at most 6 carbon atoms,

or R' represents an acetyl, propionyl, butyryl, valeryl, hexanoyl, acryloyl, crotonoyl, benzoyl or carbamoyl radical, each of these radicals being optionally substituted by one or more radicals chosen from

among the following radicals, carboxy optionally salified or esterified, methoxycarbonyl, ethoxycarbonyl, carbamoyl, dimethylcarbamoyl, amino methylamino, dimethylamino, halogen, methoxy, ethoxy, propyloxy, methylthio, ethylthiophenyl, tetrazolyl, phenylthio, tetrazolylthio or thiadiazolylthio optionally substituted by methyl or R' represents an alkoxy carbonyl radical,

or $R'_1$ represents a hydrogen atom and R'' represents a protector group for the hydroxyl radical chosen from the following radicals, phenoxyacetyl, benzoylformyl, p-nitro benzoyl, propoxycarbonyl, beta-beta-beta-trichloroethoxycarbonyl, benzyloxycarbonyl, tert-butoxycarbonyl, 1-cyclopropylethoxycarbonyl, tetrahydropyrannyl, tetrahydrothiopyrannyl, methoxytetrahydropyrannyl, 4-methoxy benzyl, phthaloyl, iso-butyryl, isovaleryl, oxalyl, succinyl, pivaloyl, mesyl, para-tert-butyl benzoyl, caprylyl, acryloyl, methyl carbamoyl, phenylcarbamoyl or naphthylcarbamoyl, it being understood that the R'' does not represent an alkenyl or alkynyl radical having at most 6 carbon atoms or a linear or branched alkyl radical having at most 6 carbon atoms optionally substituted by one or more radicals chosen from the following radicals, amino, carboxy optionally esterified or salified, alkoxycarbonyl, alkylamino, dialkylamino or heterocyclic aryl when X'—R'a represents an alkoxy, alkylthio or alkenylthio radical.

either B] n is not equal to 0, A represents a hydrogen atom, $R'_1$ represents a protector group for the amino radical and either R'' represents a $—CH_2CO_2H$ radical or a $—C(CH_3)_2CO_2H$ radical, X' represents an oxygen atom or a sulphur atom and R'a represents a methyl or ethyl radical.

or R'' represents a $—\underset{CH_3}{\underset{|}{CH}}—CO_2H$,

radical, X' represents an oxygen atom or a sulphur atom and R'a represents a methyl radical.

2. The products according to claim 1, corresponding to formula IV'

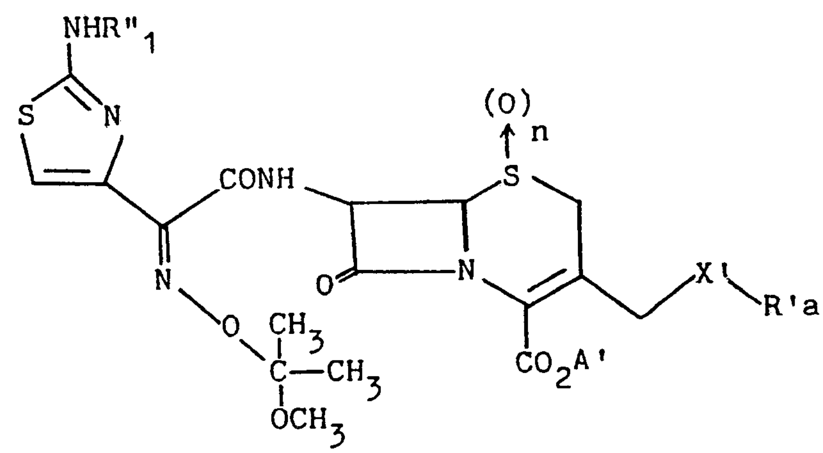

IV'

in which R'a, X and n have the significance indicated in claim 1 and A' represents a hydrogen atom or the remainder of an ester group that can be easily eliminated and $R''_1$ represents a protector group for the amino radical.

3. The products according to claim 1, corresponding to formula VI

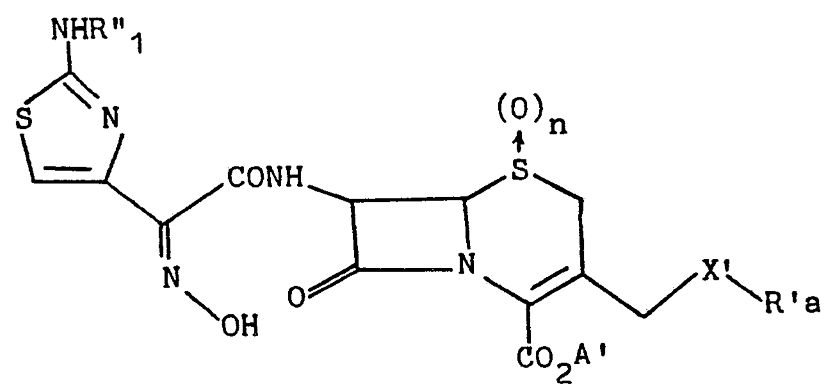

VI

in which R'a, X' and n have the significance indicated in claim 1, $R''_1$ and A' have the significance indicated in claim 2.

4. The products according to claim 1 corresponding to general formula A

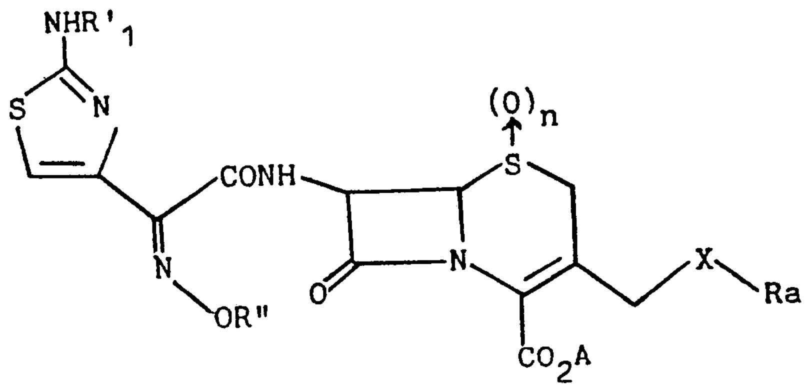

in which R'$_1$, A, R'' and n have the signifance indicated in claim 1, X represents a sulphur atom or an oxygen atom and Ra represents a linear or branched alkyl, alkenyl or alkynyl radical having at most 6 carbon atons or a benzyl or phenylethyl radical and X represents a sulphur atom or an oxygen atom.

5. The products of formula A' as defined in one of the claims 1 to 3 in which R'a represents a methyl radical and n represents the number 0.

6. The products of formula A' as defined in claim 1 corresponding to the following formulae:

7-[[2-(2-tritylamino thiazol-4-yl)-2-(hydroxyimino)-acetyl]amino]-3-(methylthiomethyl)-ceph-3-eme-4-carboxylic acid *syn* isomer.

3-methylthiomethyl-7-[2-(2-tritylamino-thiazol-4-yl-2-]-(1-methyl-1-methoxy)-ethoxy-imino[acetamido]-ceph-3-eme-4-carboxylic acid syn isomer.

7. Preparation process of products of general formula A' as defined in claim 1 characterised in that a product of formula (II')

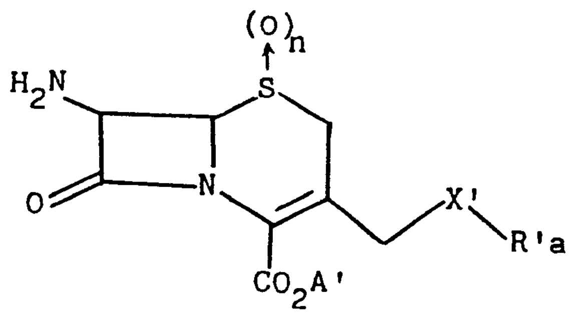

(II')

in which n, X' and R'a have the significance indicated in claim 1 and A' represents a hydrogen atom or the remainder of an ester group that can be easily eliminated, is treated with a product of formula (III$_A$)

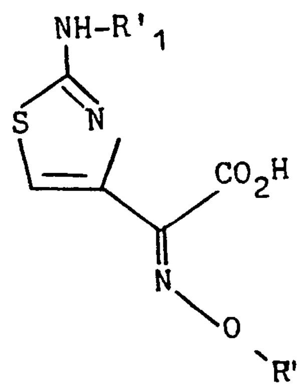

(III$_A$)

or a functional derivative of this acid, formula (III$_A$) in which R'$_1$ and R'' are as defined in claim 1 and that the product obtained is optionally esterified or salified.

8. Preparation process of products of formula IV' and VI as defined in claims 2 or 3 characterised in that a product of formula (II')

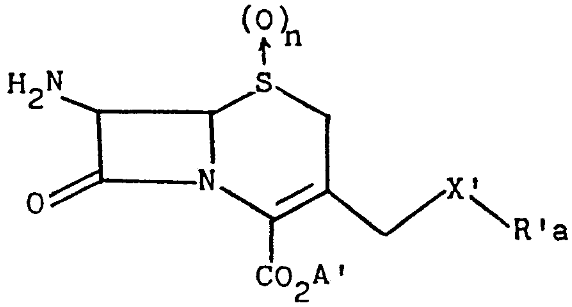

(II')

in which R'a, n and X' have the significance indicated in claim 1, and A' has the significance indicated in claim 2 is treated with a product of formula (III')

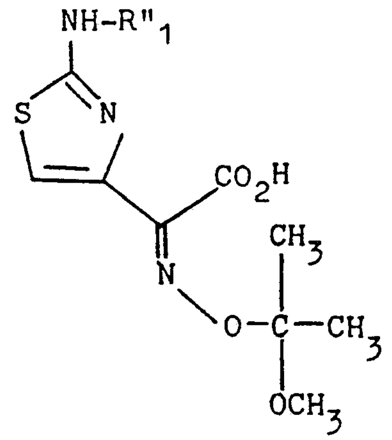

(III')

or a functional derivative of this acid, formula (III') in which $R''_1$ represents a protector group for the amino radical, to obtain a product of formula (IV')

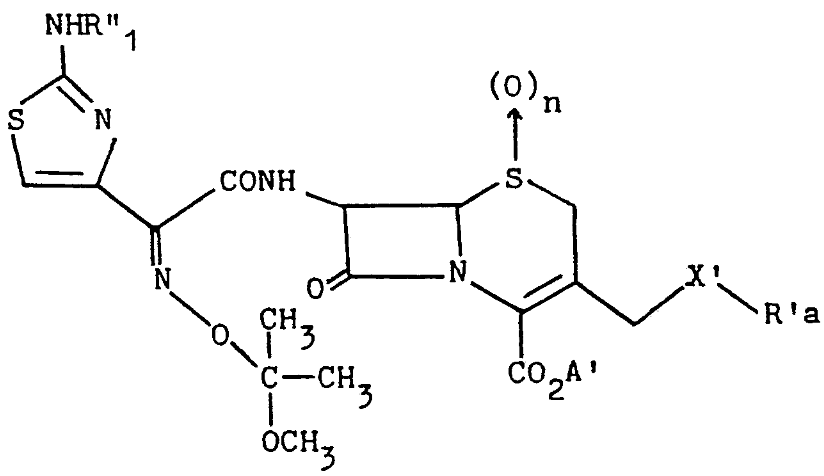

(IV')

in which A', $R''_1$, R'a, X' and n have the previous significance, which products of formula (IV') is, if desired, treated by an acid in moderate conditions, to obtain a product of formula VI

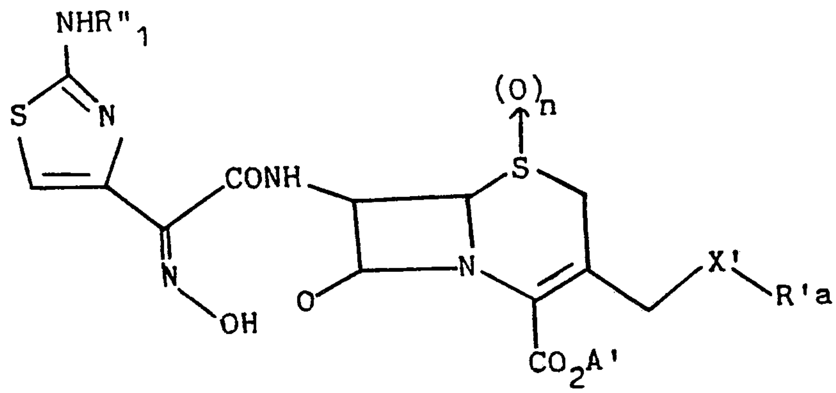

(VI)

in which R''$_1$, X', R'a, A' and n have the significance mentioned previously.

**Claims for the Contracting State: AT**

1. Preparation process of general formula (A'):

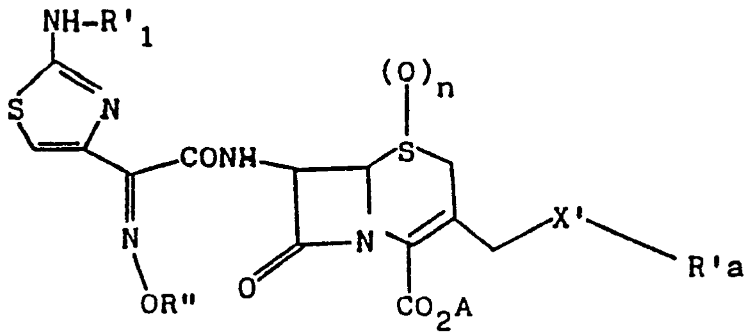

(A')

*syn* isomer in which R'$_1$, R'', n, A, X', and R'a have the following significance:

either A] represents a hydrogen atom, an equivalent of an alkali metal, of an alkaline-earth metal, of magnesium, of ammonium or of an aminated organic base or A represents the remainder of an ester group that can be easily cleaved, R'a represents an alkyl optionally interrupted by a heteroatom, linear or branched alkenyl or alkynyl radical having at most 6 carbon atoms or a benzyl or phenylethyl radical optionally substituted by a carboxy, amino or dimethylaminoethyl radical, n is equal to 0, 1 or 2, X' represents a sulphur atom optionally oxidised in the form of sulphoxide or sulphone or an oxygen atom and R'' and R'$_1$ are such that:

either R'$_1$ represents a protector group for the amino radical and R'' represents R', R' represents a hydrogen atom, a protector group for the hydroxyl radical, a linear or branched alkyl radical, alkenyl, alkynyl or cycloalkyl radical having at most 6 carbon atoms,

or R' represents an acetyl, propionic, butyryl, valeryl, hexanoyl, acryloyl, crotonoyl, benzoyl or carbamoyl radical, each of these radicals being optionally substituted by one or more radicals chosen from the following radicals, carboxy optionally salified or esterified, methoxycarbonyl, ethoxycarbonyl, carbamoyl, dimethylcarbamoyl, amino, methylamino, dimethylamino, halogen, methoxy, ethoxy, propyloxy, methylthio, ethylthiophenyl, tetrazolyl, phenylthio, tetrazolylthio or thiadiazolythio optionally substituted by methyl or R' represents an alkoxy carbonyl radical,

or R'$_1$ represents a hydrogen atom and R'' represents a protector group for the hydroxyl radical chosen from the following radicals, phenoxyacetyl, benzoylformyl, p-nitro benzoyl, propoxycarbonyl, beta-beta-beta-trichloroethoxycarbonyl, benzyloxycarbonyl, tert-butoxycarbonyl, 1-cyclopropylethoxycarbonyl, tetrahydropyranyl, tetrahydrothiopyranyl, methoxytetrahydropyranyl, 4-methoxy benzyl, phthaloyl, isobutyryl, isovaleryl, oxalyl, succinyl, pivaloyl, mesyl, para-tert-butyl benzoyl, caprylyl, acryloyl, methyl carbomoyl, phenylcarbomoyl or naphtylcarbomoyl, it being understood that the R'' does not represent an alkenyl or alkynyl radical having at most 6 carbon atoms or a linear or branched alkyl radical having at most 6 carbon atoms optionally substituted by one or more radicals chosen from the following radicals, amino, carboxy optionally esterified or salified, alkoxycarbonyl, alkylamino, dialkylamino or heterocyclic aryl when X'—R'a represents an alkoxy, alkylthio or alkenylthio radical;

or B] n is not equal to 0, A represents a hydrogen atom, R'$_1$ represents a protector group for the amino radical and either R'' represents a $—CH_2CO_2H$ radical or a $—C(CH_3)_2CO_2H$ radical, X' represents an oxygen atom or a sulphur atom and R'a represents a methyl or ethyl radical or R'' represents a

$$—\underset{\displaystyle CH_3}{\underset{|}{CH}}—CO_2H,$$

radical, X' represents an oxygen atom or a sulphur atom and R'a represents a methyl radical, characterised in that a product of formula (II')

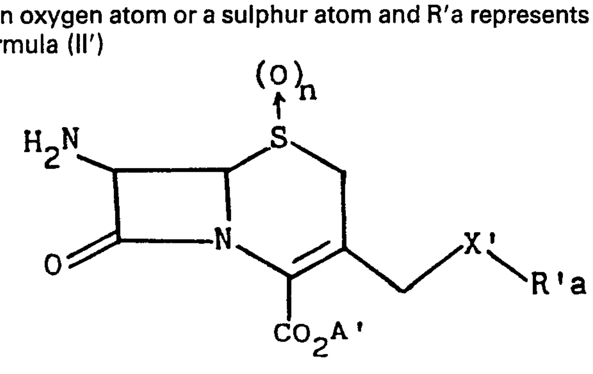

(II')

in which n, X′ and $R'_a$ have the significance indicated above and A′ represents a hydrogen atom or the remainder of an ester group that can be easily eliminated, is treated by a product of formula ($III_A$):

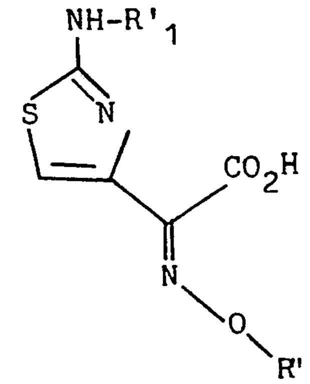

($III_A$)

or a functional derivative of this acid, formula ($III_A$) in which $R'_1$ and R″ are as defined above and that the product obtained is optionally esterified or salified.

2. Preparation process of products of formula IV′:

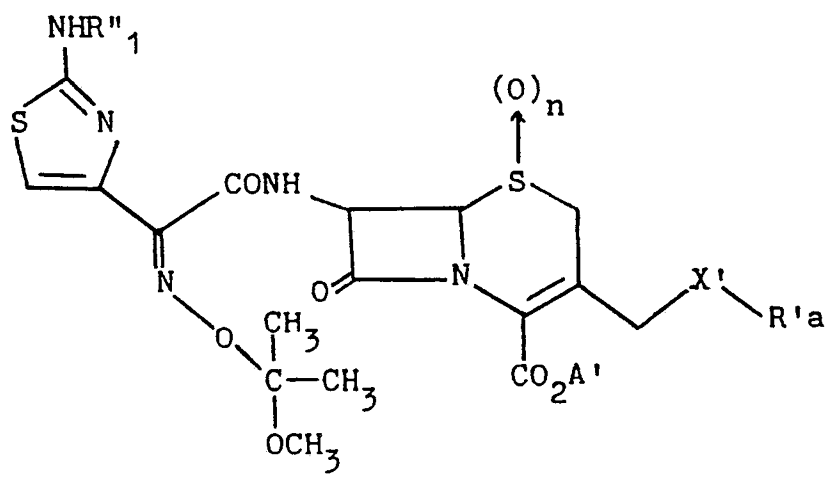

(IV′)

in which R′a, X and n have a significance indicated in claim 1 and A′ represents a hydrogen atom or the remainder of an ester group that can be easily eliminated and $R''_1$ represents a protector group of the amino radical, and of formula VI:

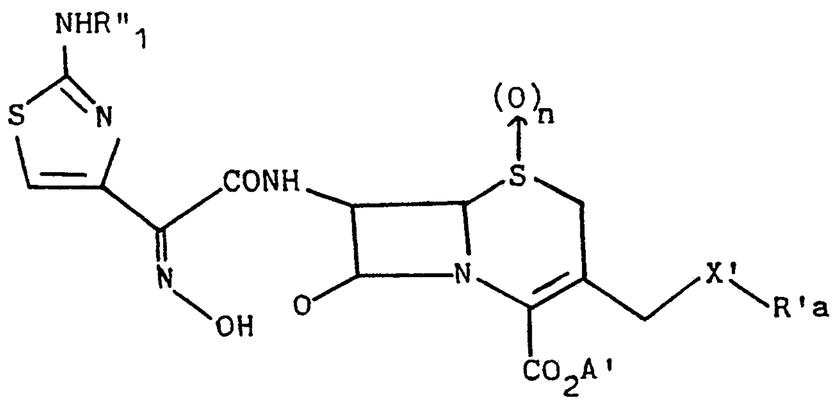

(VI)

in which R′a, X′ and n have the significance indicated in claim 1, $R''_1$ and A′ have the significance indicated above, characterised in that a product of formula II′:

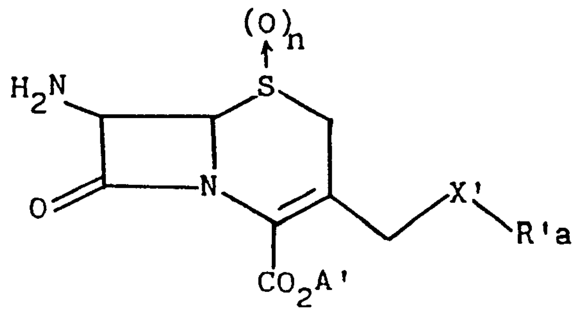

(II')

in which R'a, n and X' have the significance indicated in claim 1 and A' has the significance indicated in claim 2, is treated by a product of formula (III')

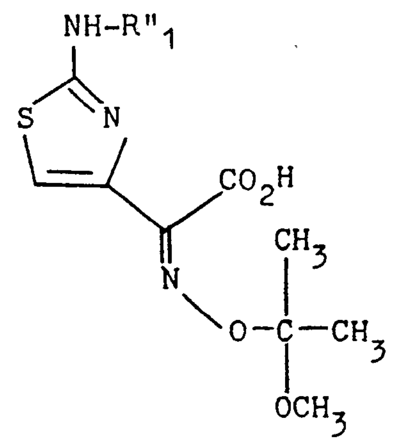

(III')

or a functional derivative of this acid, formula (III') in which $R''_1$ represents a protector group for the amino radical, to obtain a product of formula (IV')

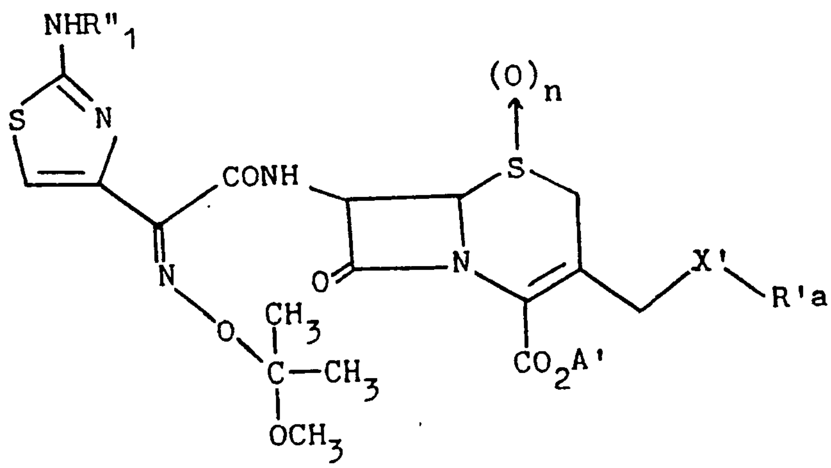

(IV')

in which A', $R''_1$, R'a, X' and n have the significance mentioned previously, which product of formula (IV') is, if desired, treated with an acid in moderate conditions, to obtain a product of formula VI

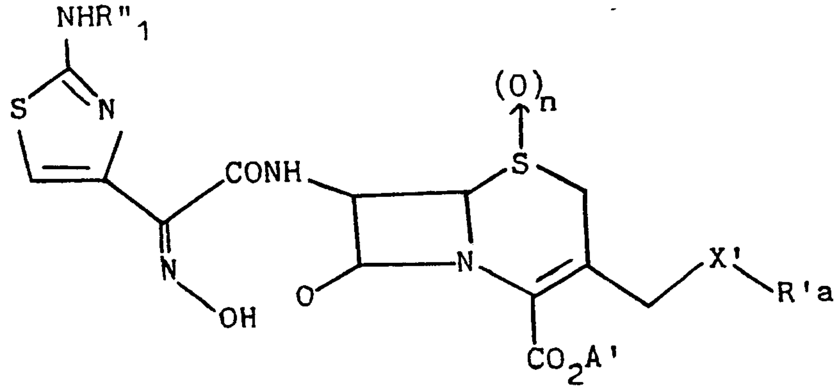

(VI)

in which R''$_1$, X' R'a, A' and n have the significance mentioned previously.

3. Process according to any one of claims 1 to 3 characterised in that the products corresponding to general formula A are prepared:

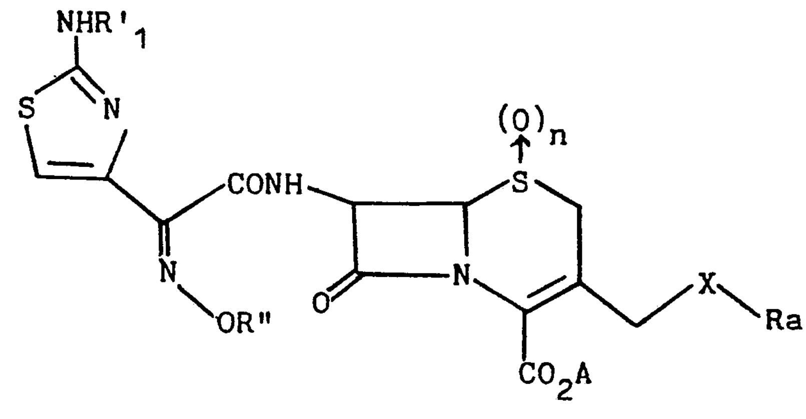

in which R'$_1$, A, R'' and n have the significance indicated in claim 1, X represents a sulphur atom or a hydrogen atom and Ra represents a linear or branched alkyl, alkenyl or alkynyl radical having at most 6 carbon atoms or a benzyl radical or a phenylethyl radical and X represents a sulphur atom or an oxygen atom.

4. Process according to one of claims 1 to 3, characterised in that a product of formula A' is prepared in which R'$_a$ represents a methyl radical and n represents the number 0.

5. Process according to one of claims 1 to 4, characterised in that a product of formula A' is prepared corresponding to the following formulae:

7-[[2-(2-tritylamino thiazol-4-yl)-2-(hydroxyimino)-acetyl]amino]-3-(methylthiomethyl)-ceph-3-eme-4-carboxylic acid *syn* isomer.

3-methylthiomethyl-7-[2-(2-tritylamino-thiazol-4-yl)-2]-(1-methyl-1-methoxy)-ethoxy-imino[acetamido]ceph-3-eme-carboxylic acid *syn* isomer.